(19) Europäisches Patentamt
European Patent Office
Office européen des brevets

(11) EP 4 198 034 A1

(12) EUROPEAN PATENT APPLICATION
published in accordance with Art. 153(4) EPC

(43) Date of publication:
**21.06.2023 Bulletin 2023/25**

(21) Application number: **21855617.3**

(22) Date of filing: **13.08.2021**

(51) International Patent Classification (IPC):
***C07D 413/06*** *(2006.01)* ***A61K 31/5377*** *(2006.01)*
***A61P 13/00*** *(2006.01)* ***A61P 25/04*** *(2006.01)*
***A61P 11/14*** *(2006.01)*

(52) Cooperative Patent Classification (CPC):
**A61K 31/5377; A61P 11/14; A61P 13/00; A61P 25/04; C07D 413/06**

(86) International application number:
**PCT/CN2021/112386**

(87) International publication number:
**WO 2022/033567 (17.02.2022 Gazette 2022/07)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.08.2020 CN 202010811267**
**01.06.2021 CN 202110609305**

(71) Applicant: **Tuojie Biotech (Shanghai) Co., Ltd.**
**Shanghai 201203 (CN)**

(72) Inventors:
- **LI, Wenming**
  **Shanghai 201203 (CN)**
- **LIU, Ning**
  **Shanghai 201203 (CN)**
- **LIU, Biao**
  **Shanghai 201203 (CN)**
- **LIU, Haomiao**
  **Shanghai 201203 (CN)**
- **YU, Jian**
  **Shanghai 201203 (CN)**
- **ZOU, Hao**
  **Shanghai 201203 (CN)**
- **ZHU, Wei**
  **Shanghai 201203 (CN)**
- **LI, Zhengtao**
  **Shanghai 201203 (CN)**
- **ZHANG, Zhen**
  **Shanghai 201203 (CN)**
- **LI, Yunfei**
  **Shanghai 201203 (CN)**

(74) Representative: **Viganò, Elena et al**
**Dragotti & Associati S.r.l.**
**Via Nino Bixio, 7**
**20129 Milano (IT)**

# (54) BENZIMIDAZOLE DERIVATIVES, PREPARATION METHOD THEREFOR AND MEDICAL USE THEREOF

(57) The present disclosure relates to benzimidazole derivatives, a preparation method therefor and a medical use thereof. Specifically, the present disclosure relates to a benzimidazole derivative represented by the general formula (I), a preparation method therefor, a pharmaceutical composition containing the derivative, and its use as a therapeutic agent, in particular its use for the treatment of diseases related to P2X3 activity.

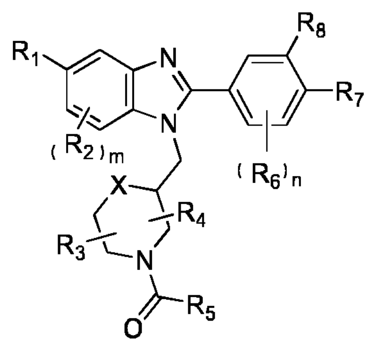

(I)



Processed by Luminess, 75001 PARIS (FR)

## Description

### TECHNICAL FIELD

The present disclosure relates to the field of pharmaceuticals, and in particular to a novel benzimidazole derivative, a preparation method therefor and use thereof in pharmaceuticals.

### BACKGROUND

P2X receptors are a family of cation-permeable ligand-gated ion channels that open in response to the binding of extracellular adenosine 5'-triphosphate (ATP). They belong to a larger family of receptors, called purinergic receptors. P2X receptors are present in a variety of organisms including human, mouse, rat, rabbit, chicken, zebrafish, bullfrog, trematode and amoebae. Seven independent genes encoding the P2X subunit have been identified and named P2X1 to P2X7, respectively. Different subunits exhibit different sensitivities to purinergic agonists and antagonists.

P2X3 receptor has 4 ATP-binding sites on a single subunit, which consist of 2 transmembrane domains, the N-terminus and C-terminus located intracellularly, and a conserved sequence located in the extracellular loop structure. High expression of the P2X3 receptor was found in both specific medium- and small-diameter neurons associated with nociceptive information. Meanwhile, the P2X3 receptor is also involved in the transmission of some non-nociceptive sensations. It has been demonstrated that the P2X3 receptor is involved in bladder sensory function and is a key receptor-mediated bladder sensory signal expressed in the mucosal tissues of the bladder, which are rich in sensory nerve fibers. P2X3 is also expressed in sensory nerve fibers of the pharyngeal mucosa, and is associated with the conduction and formation of taste sensation.

After an organism is injured or subjected to nerve damages, a large number of ATPs are released, which activate the P2X3 receptor on the presynaptic membrane to cause influx of a large number of $Ca^{2+}$ ions; and the increase in the concentration of intracellular calcium activates protein kinase A (PKA) and protein kinase C (PKC), resulting in the phosphorylation of PKA and PKC, and simultaneously promotes the release of glutamic acid, thereby activating the NMDA receptor, leading to the generation of excitatory postsynaptic current, and causing central sensitization. Many studies have shown that upregulation of P2X3 receptor expression can lead to the formation of hyperalgesia and is involved in pain signaling.

MK-7264 is a P2X3 receptor activity antagonist with $IC_{50}$ values of about 30 nM and 100-250 nM for human homologous recombinant hP2X3 and hP2X2/3, respectively, and is currently in clinical phase III for the treatment of patients with chronic cough.

### SUMMARY

The present disclosure provides a compound of formula (I) or a pharmaceutically acceptable salt or isomer thereof,

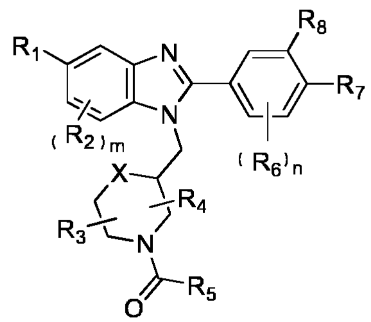

wherein $R_1$ is selected from the group consisting of hydrogen, deuterium, halogen, $C_1$-$C_6$ hydroxyalkyl, $C_1$-$C_6$ alkyl optionally substituted with halogen or deuterium, and $C_1$-$C_6$ alkoxy optionally substituted with halogen or deuterium;

$R_2$ are each independently selected from the group consisting of hydrogen, deuterium, halogen, $C_1$-$C_6$ hydroxyalkyl, $C_1$-$C_6$ alkyl optionally substituted with halogen or deuterium, and $C_1$-$C_6$ alkoxy optionally substituted with halogen or deuterium;

$R_3$ and $R_4$ are each independently selected from the group consisting of hydrogen, halogen and $C_1$-$C_4$ alkyl optionally substituted with halogen, or $R_3$ and $R_4$ form, together with the carbon atom to which they are attached, $C_3$-$C_6$

cyclohydrocarbylene optionally substituted with halogen, or $R_3$ and $R_4$ on adjacent carbon atoms together form $C_3$-$C_8$ cyclohydrocarbyl optionally substituted with halogen;
$R_5$ is selected from the group consisting of $C_1$-$C_6$ alkyl optionally substituted with halogen or cyano, $C_3$-$C_6$ cyclohydrocarbyl optionally substituted with halogen or cyano, heterocyclyl optionally substituted with halogen or cyano, $C_1$-$C_6$ alkoxy optionally substituted with halogen or cyano, and amino optionally substituted with alkyl;
$R_6$ are each independently selected from the group consisting of hydrogen, deuterium, halogen, cyano, cyclopropyl, and $C_1$-$C_6$ alkyl optionally substituted with halogen or deuterium;
$R_7$ and $R_8$ are each independently selected from the group consisting of:

a) hydrogen, deuterium, halogen, cyano, amino, hydroxy, $C_1$-$C_6$ alkyl optionally substituted with halogen, sulfone, sulfoxide, sulfonamide, sulfenamide, $C_{1-3}$ carboxyl, and $C_1$-$C_6$ alkoxy optionally substituted with halogen;

b)

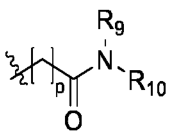 , 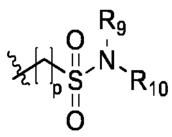 and 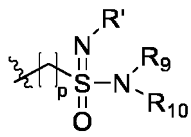 ,

wherein p is selected from the group consisting of 0, 1 and 2; $R_9$ and $R_{10}$ are each independently selected from the group consisting of hydrogen, $C_1$-$C_6$ alkyl, $C_1$-$C_6$ alkoxy, and $C_3$-$C_8$ cyclohydrocarbyl, or $R_9$ and $R_{10}$ form, together with the nitrogen atom to which they are attached, 4- to 6-membered heterocyclyl, wherein the heterocyclyl is optionally substituted with one or more substituents selected from the group consisting of halogen, hydroxy, cyano, $C_1$-$C_6$ haloalkyl, and $C_1$-$C_6$ alkyl; R' is selected from the group consisting of hydrogen, $C_1$-$C_6$ alkyl, $C_3$-$C_8$ cycloalkyl, aryl and heteroaryl; and in

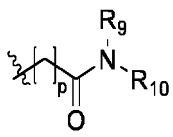 ,

when p is 0, the combination of $R_9$ and $R_{10}$ is not hydrogen and methyl;

c)

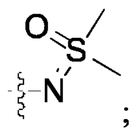 ;

d) heterocyclyl and heteroaryl, wherein the heterocyclyl and heteroaryl are each optionally substituted with one or more substituents selected from the group consisting of oxo, halogen, hydroxy, carbonyl, $C_1$-$C_6$ alkyl and cyano, wherein the $C_1$-$C_6$ alkyl is optionally substituted with one or more halogens; and

e)

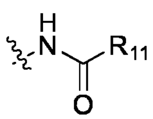 , 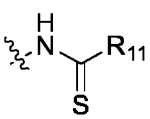 , 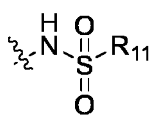 and 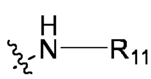 ,

wherein $R_{11}$ is selected from the group consisting of $C_1$-$C_6$ alkyl, $C_1$-$C_6$ alkoxy, aryl, heteroaryl, $C_3$-$C_8$ cyclohydrocarbyl, heterocyclyl, $C_1$-$C_6$ cyanoalkyl, $C_3$-$C_8$ cyclohydrocarbyloxy, and amino optionally substituted with $C_1$-$C_6$ alkyl; or

$R_7$ and $R_8$ form, together with the atom to which they are attached, an optionally substituted aromatic or non-aromatic heterocyclic ring;

X is selected from the group consisting of an oxygen atom, -NH- and methylene, wherein the methylene is optionally substituted with one or more substituents selected from the group consisting of halogen, $C_3$-$C_8$ cyclohydrocarbyl, $C_3$-$C_6$ cyclohydrocarbylene, and $C_1$-$C_6$ alkyl;

m is an integer of 1-3; and

n is an integer of 1-4.

In some embodiments, $R_7$ and $R_8$ are each independently selected from the group consisting of:

a) hydrogen, deuterium, halogen, cyano, amino, sulfone, sulfonamide, sulfenamide, and $C_1$-$C_3$ alkyl substituted with one or more halogen;

b)

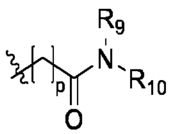 , 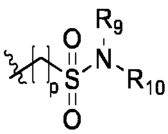 and 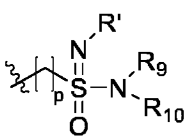 ,

wherein p is selected from the group consisting of 0, 1 and 2; $R_9$ and $R_{10}$ are each independently selected from the group consisting of hydrogen, $C_1$-$C_3$ alkyl, $C_1$-$C_3$ alkoxy, and $C_3$-$C_6$ cyclohydrocarbyl, or $R_9$ and $R_{10}$ form, together with the nitrogen atom to which they are attached, 4- to 6-membered heterocyclyl, wherein the heterocyclyl is optionally substituted with one or more substituents selected from the group consisting of halogen, hydroxy and $C_1$-$C_3$ alkyl; R' is selected from the group consisting of hydrogen, $C_1$-$C_3$ alkyl, $C_3$-$C_6$ cycloalkyl, aryl and heteroaryl; and in

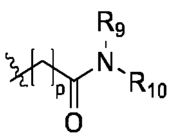 ,

when p is 0, the combination of $R_9$ and $R_{10}$ is not hydrogen and methyl;

c)

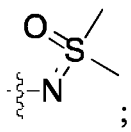 ;

d) 4- to 6-membered heterocyclyl or heteroaryl, wherein the heterocyclyl or heteroaryl is optionally substituted with one or more substituents selected from the group consisting of oxo, halogen, hydroxy, carbonyl, $C_1$-$C_3$ alkyl and cyano, wherein the $C_1$-$C_3$ alkyl can be optionally substituted with one or more halogens; and

e)

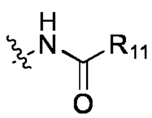 , 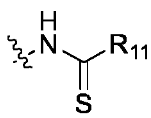 , 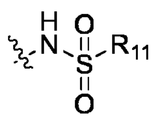 and 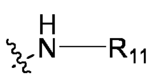 ,

wherein $R_{11}$ is selected from the group consisting of $C_1$-$C_3$ alkyl, $C_1$-$C_3$ alkoxy, 5- to 6-membered aryl or heteroaryl, 3- to 8-membered cyclohydrocarbyl, 3- to 8-membered heterocyclyl, $C_1$-$C_3$ cyanoalkyl, $C_3$-$C_6$ cyclohydrocarbyloxy,

and amino optionally substituted with $C_1$-$C_3$ alkyl.

In some embodiments, $R_7$ is 4- to 6-membered heterocyclyl or heteroaryl, wherein the heterocyclyl or heteroaryl is optionally substituted with one or more substituents selected from the group consisting of oxo, halogen, hydroxy, carbonyl, $C_1$-$C_3$ alkyl and cyano, wherein the $C_1$-$C_3$ alkyl is optionally substituted with one or more halogens; and $R_8$ is selected from the group consisting of hydrogen, deuterium, halogen, cyano, and $C_1$-$C_3$ alkyl substituted with one or more halogens.

In some embodiments, $R_7$ is 4- to 6-membered heterocyclyl, wherein the heterocyclyl comprises -NH-C(=O)- or -NH-S(=O)$_2$-, and the heterocyclyl is optionally substituted with one or more substituents selected from the group consisting of oxo, halogen, hydroxy, carbonyl, $C_1$-$C_3$ alkyl and cyano, wherein the $C_1$-$C_3$ alkyl is optionally substituted with one or more halogens; and

$R_8$ is selected from the group consisting of hydrogen, deuterium, halogen, cyano, and $C_1$-$C_3$ alkyl substituted with one or more halogens.

In some embodiments, $R_7$ is 5-membered heterocyclyl, wherein the heterocyclyl comprises -NH-C(=O)-, and the heterocyclyl is optionally substituted with one or more substituents selected from the group consisting of oxo, halogen, hydroxy, carbonyl, $C_1$-$C_3$ alkyl and cyano, wherein the $C_1$-$C_3$ alkyl is optionally substituted with one or more halogens; and $R_8$ is selected from the group consisting of hydrogen, deuterium, halogen, cyano, and $C_1$-$C_3$ alkyl substituted with one or more halogens.

In some embodiments, in the compound of formula (I),

$R_1$ is selected from the group consisting of hydrogen, deuterium, halogen, $C_1$-$C_6$ hydroxyalkyl, $C_1$-$C_6$ alkyl optionally substituted with halogen or deuterium, and $C_1$-$C_6$ alkoxy optionally substituted with halogen or deuterium;

$R_2$ are each independently selected from the group consisting of hydrogen, deuterium, halogen, $C_1$-$C_6$ hydroxyalkyl, $C_1$-$C_6$ alkyl optionally substituted with halogen or deuterium, and $C_1$-$C_6$ alkoxy optionally substituted with halogen or deuterium;

$R_3$ and $R_4$ are each independently selected from the group consisting of hydrogen, halogen and $C_1$-$C_4$ alkyl optionally substituted with halogen, or $R_3$ and $R_4$ form, together with the carbon atom to which they are attached, $C_3$-$C_6$ cyclohydrocarbylene optionally substituted with halogen, or $R_3$ and $R_4$ on adjacent carbon atoms together form $C_3$-$C_8$ cyclohydrocarbyl optionally substituted with halogen;

$R_5$ is selected from the group consisting of $C_1$-$C_6$ alkyl optionally substituted with halogen or cyano, $C_3$-$C_6$ cyclohydrocarbyl optionally substituted with halogen or cyano, heterocyclyl optionally substituted with halogen or cyano, $C_1$-$C_6$ alkoxy optionally substituted with halogen or cyano, and amino optionally substituted with alkyl;

$R_6$ are each independently selected from the group consisting of hydrogen, deuterium, halogen, cyano, cyclopropyl, and $C_1$-$C_6$ alkyl optionally substituted with halogen or deuterium;

$R_7$ and $R_8$ form, together with the atom to which they are attached, a 3- to 12-membered aromatic or non-aromatic heterocyclic ring, wherein the heterocyclic ring is monocyclic or bicyclic, and the heterocyclic ring is optionally substituted with one or more substituents selected from the group consisting of $C_1$-$C_6$ alkylamide, halogen, oxo, $C_1$-$C_6$ alkyl optionally substituted with halogen, and $C_1$-$C_6$ alkoxy;

X is selected from the group consisting of an oxygen atom, -NH- and methylene, wherein the methylene is optionally substituted with one or more substituents selected from the group consisting of halogen, $C_3$-$C_8$ cyclohydrocarbyl, $C_3$-$C_6$ cyclohydrocarbylene, and $C_1$-$C_6$ alkyl;

m is an integer of 1-3; and

n is an integer of 1-4.

In some embodiments, $R_7$ and $R_8$ form, together with the atom to which they are attached, a 3- to 12-membered non-aromatic heterocyclic ring, wherein the heterocyclic ring is monocyclic or bicyclic, the heterocyclic ring comprises -NH-C(=O)- or -NH-S(=O)$_2$-, and the heterocyclic ring is optionally substituted with one or more substituents selected from the group consisting of $C_1$-$C_6$ alkylamide, halogen, oxo, $C_1$-$C_6$ alkyl optionally substituted with halogen, and $C_1$-$C_6$ alkoxy.

In some embodiments, $R_7$ and $R_8$ form, together with the atom to which they are attached, a 4- to 8-membered non-aromatic heterocyclic ring, wherein the heterocyclic ring is monocyclic or bicyclic, the heterocyclic ring comprises -NH-C(=O)- or -NH-S(=O)$_2$-, and the heterocyclic ring is optionally substituted with one or more substituents selected from the group consisting of $C_1$-$C_3$ alkylamide, halogen, oxo, $C_1$-$C_3$ alkyl optionally substituted with halogen, and $C_1$-$C_3$ alkoxy.

In some embodiments, in the compound of formula (I),

$R_1$ is selected from the group consisting of hydrogen, deuterium, halogen, $C_1$-$C_6$ hydroxyalkyl, $C_1$-$C_6$ alkyl optionally substituted with halogen or deuterium, and $C_1$-$C_6$ alkoxy optionally substituted with halogen or deuterium;

$R_2$ are each independently selected from the group consisting of hydrogen, deuterium, halogen, $C_1$-$C_6$ hydroxyalkyl, $C_1$-$C_6$ alkyl optionally substituted with halogen or deuterium, and $C_1$-$C_6$ alkoxy optionally substituted with halogen or deuterium;

$R_3$ and $R_4$ are each independently selected from the group consisting of hydrogen, halogen and $C_1$-$C_4$ alkyl optionally substituted with halogen, or $R_3$ and $R_4$ form, together with the carbon atom to which they are attached, $C_3$-$C_6$ cyclohydrocarbylene optionally substituted with halogen, or $R_3$ and $R_4$ on adjacent carbon atoms together form $C_3$-$C_8$ cyclohydrocarbyl optionally substituted with halogen;

$R_5$ is selected from the group consisting of $C_1$-$C_6$ alkyl optionally substituted with halogen or cyano, $C_3$-$C_6$ cyclohydrocarbyl optionally substituted with halogen or cyano, heterocyclyl optionally substituted with halogen or cyano, $C_1$-$C_6$ alkoxy optionally substituted with halogen or cyano, and amino optionally substituted with alkyl;

$R_6$ are each independently selected from the group consisting of hydrogen, deuterium, halogen, cyano, cyclopropyl, and $C_1$-$C_6$ alkyl optionally substituted with halogen or deuterium;

$R_7$ is

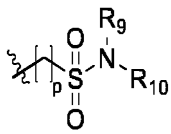

,

wherein p is selected from the group consisting of 0, 1 and 2; $R_9$ and $R_{10}$ are each independently selected from the group consisting of hydrogen, $C_1$-$C_3$ alkyl, $C_1$-$C_3$ alkoxy and $C_3$-$C_6$ cyclohydrocarbyl, or $R_9$ and $R_{10}$ form, together with the nitrogen atom to which they are attached, 4- to 6-membered heterocyclyl, wherein the heterocyclyl is optionally substituted with one or more substituents selected from the group consisting of halogen, hydroxy and $C_1$-$C_3$ alkyl;

$R_8$ is selected from the group consisting of hydrogen, deuterium, halogen, cyano, and $C_1$-$C_3$ alkyl substituted with one or more halogens;

X is selected from the group consisting of an oxygen atom, -NH- and methylene, wherein the methylene is optionally substituted with one or more substituents selected from the group consisting of halogen, $C_3$-$C_8$ cyclohydrocarbyl, $C_3$-$C_6$ cyclohydrocarbylene, and $C_1$-$C_6$ alkyl;

m is an integer of 1-3; and

n is an integer of 1-4.

In some embodiments, in the compound of formula (I),

$R_7$ is

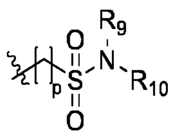

,

wherein p is 0 or 1; $R_9$ is selected from the group consisting of hydrogen, $C_1$-$C_3$ alkyl, $C_1$-$C_3$ alkoxy and $C_3$-$C_6$ cyclohydrocarbyl, $R_{10}$ is hydrogen, or

$R_9$ and $R_{10}$ form, together with the nitrogen atom to which they are attached, 4- to 6-membered heterocyclyl, wherein the heterocyclyl is optionally substituted with one or more halogens; and

$R_8$ is selected from the group consisting of hydrogen, deuterium and halogen.

In some embodiments, in the compound of formula (I),

$R_1$ is selected from the group consisting of hydrogen, deuterium, halogen, $C_1$-$C_6$ hydroxyalkyl, $C_1$-$C_6$ alkyl optionally substituted with halogen or deuterium, and $C_1$-$C_6$ alkoxy optionally substituted with halogen or deuterium;

$R_2$ are each independently selected from the group consisting of hydrogen, deuterium, halogen, $C_1$-$C_6$ hydroxyalkyl, $C_1$-$C_6$ alkyl optionally substituted with halogen or deuterium, and $C_1$-$C_6$ alkoxy optionally substituted with halogen or deuterium;

$R_3$ and $R_4$ are each independently selected from the group consisting of hydrogen, halogen and $C_1$-$C_4$ alkyl optionally substituted with halogen, or $R_3$ and $R_4$ form, together with the carbon atom to which they are attached, $C_3$-$C_6$

cyclohydrocarbylene optionally substituted with halogen, or $R_3$ and $R_4$ on adjacent carbon atoms together form $C_3$-$C_8$ cyclohydrocarbyl optionally substituted with halogen;

$R_5$ is selected from the group consisting of $C_1$-$C_6$ alkyl optionally substituted with halogen or cyano, $C_3$-$C_6$ cyclohydrocarbyl optionally substituted with halogen or cyano, heterocyclyl optionally substituted with halogen or cyano, $C_1$-$C_6$ alkoxy optionally substituted with halogen or cyano, and amino optionally substituted with alkyl;

$R_6$ are each independently selected from the group consisting of hydrogen, deuterium, halogen, cyano, cyclopropyl, and $C_1$-$C_6$ alkyl optionally substituted with halogen or deuterium;

$R_7$ is

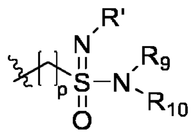

,

wherein p is selected from the group consisting of 0, 1 and 2; $R_9$ and $R_{10}$ are each independently selected from the group consisting of hydrogen, $C_1$-$C_3$ alkyl, $C_1$-$C_3$ alkoxy and $C_3$-$C_6$ cyclohydrocarbyl, or $R_9$ and $R_{10}$ form, together with the nitrogen atom to which they are attached, 4- to 6-membered heterocyclyl, wherein the heterocyclyl is optionally substituted with one or more substituents selected from the group consisting of halogen, hydroxy and $C_1$-$C_3$ alkyl; R' is selected from the group consisting of hydrogen, $C_1$-$C_3$ alkyl, $C_3$-$C_6$ cycloalkyl, aryl and heteroaryl;

$R_8$ is selected from the group consisting of hydrogen, deuterium, halogen, cyano, and $C_1$-$C_3$ alkyl substituted with one or more halogens;

X is selected from the group consisting of an oxygen atom, -NH- and methylene, wherein the methylene is optionally substituted with one or more substituents selected from the group consisting of halogen, $C_3$-$C_8$ cyclohydrocarbyl, $C_3$-$C_6$ cyclohydrocarbylene, and $C_1$-$C_6$ alkyl;

m is an integer of 1-3; and

n is an integer of 1-4.

In some embodiments, in the compound of formula (I),

$R_7$ is

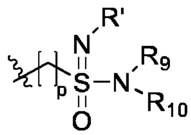

,

wherein p is 0 or 1; $R_9$ is selected from the group consisting of hydrogen, $C_1$-$C_3$ alkyl, $C_1$-$C_3$ alkoxy and $C_3$-$C_6$ cyclohydrocarbyl, $R_{10}$ is hydrogen, or $R_9$ and $R_{10}$ form, together with the nitrogen atom to which they are attached, 4- to 6-membered heterocyclyl, wherein the heterocyclyl is optionally substituted with one or more halogens; R' is selected from the group consisting of hydrogen, $C_1$-$C_3$ alkyl, $C_3$-$C_6$ cycloalkyl, aryl and heteroaryl; and

$R_8$ is selected from the group consisting of hydrogen, deuterium and halogen.

In some embodiments, in the compound of formula (I),

$R_1$ is selected from the group consisting of hydrogen, deuterium, $C_1$-$C_6$ alkyl optionally substituted with halogen or deuterium, and halogen;

$R_2$ are each independently selected from the group consisting of hydrogen, deuterium, $C_1$-$C_6$ alkyl optionally substituted with halogen or deuterium, and halogen;

$R_3$ and $R_4$ are each independently hydrogen or halogen, or $R_3$ and $R_4$ form, together with the carbon atom to which they are attached, $C_3$-$C_6$ cyclohydrocarbylene optionally substituted with halogen, or $R_3$ and $R_4$ on adjacent carbon atoms together form $C_3$-$C_8$ cyclohydrocarbyl;

$R_5$ is selected from the group consisting of $C_1$-$C_6$ alkyl optionally substituted with halogen or cyano, $C_3$-$C_6$ cyclohydrocarbyl optionally substituted with halogen or cyano, heterocyclyl optionally substituted with halogen or cyano, $C_1$-$C_6$ alkoxy optionally substituted with halogen or cyano, and amino optionally substituted with alkyl;

$R_6$ are each independently selected from the group consisting of hydrogen, deuterium, halogen, cyano, cyclopropyl, and $C_1$-$C_6$ alkyl optionally substituted with halogen or deuterium;

$R_7$ and $R_8$ are each independently selected from the group consisting of:

a) hydrogen, deuterium, halogen, cyano, amino, hydroxy, $C_1$-$C_6$ alkyl optionally substituted with halogen, sulfone, sulfoxide, sulfonamide, sulfenamide, $C_{1-3}$ carboxyl, and $C_1$-$C_6$ alkoxy optionally substituted with halogen;

b)

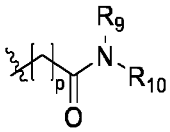, 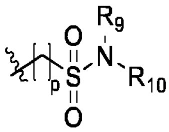 and 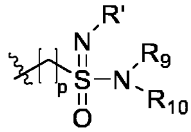,

wherein p is selected from the group consisting of 0, 1 and 2; $R_9$ and $R_{10}$ are each independently selected from the group consisting of hydrogen, $C_1$-$C_3$ alkyl, $C_1$-$C_3$ alkoxy, and $C_3$-$C_6$ cyclohydrocarbyl, or $R_9$ and $R_{10}$ form, together with the nitrogen atom to which they are attached, 4- to 6-membered heterocyclyl, wherein the heterocyclyl is optionally substituted with one or more substituents selected from the group consisting of halogen, hydroxy and $C_1$-$C_3$ alkyl; R' is selected from the group consisting of hydrogen, $C_1$-$C_3$ alkyl, $C_3$-$C_6$ cycloalkyl, aryl and heteroaryl; and in

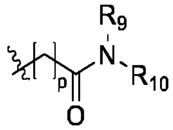,

when p is 0, the combination of $R_9$ and $R_{10}$ is not hydrogen and methyl;

c)

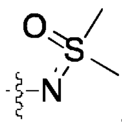;

d) heterocyclyl and heteroaryl, wherein the heterocyclyl and heteroaryl are each optionally substituted with one or more substituents selected from the group consisting of oxo, halogen, hydroxy, carbonyl, $C_1$-$C_6$ alkyl and cyano, wherein the $C_1$-$C_6$ alkyl is optionally substituted with one or more halogens; and

e)

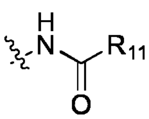, 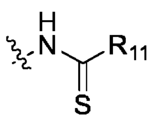, 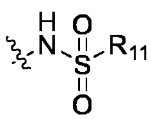 and 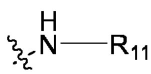,

wherein $R_{11}$ is selected from the group consisting of $C_1$-$C_6$ alkyl, $C_1$-$C_6$ alkoxy, aryl, heteroaryl, $C_3$-$C_8$ cyclohydrocarbyl, heterocyclyl, $C_1$-$C_6$ cyanoalkyl, $C_3$-$C_8$ cyclohydrocarbyloxy, and amino optionally substituted with $C_1$-$C_6$ alkyl; or

$R_7$ and $R_8$ form, together with the atom to which they are attached, an optionally substituted aromatic or non-aromatic heterocyclic ring;
X is selected from the group consisting of an oxygen atom, -NH- and methylene, wherein the methylene is optionally substituted with one or more substituents selected from the group consisting of halogen, $C_3$-$C_8$ cyclohydrocarbyl, $C_3$-$C_6$ cyclohydrocarbylene, and $C_1$-$C_6$ alkyl;
m is an integer of 1-3; and
n is an integer of 1-4.

In some embodiments, $R_5$ is $C_1$-$C_6$ alkyl optionally substituted with halogen or cyano or $C_1$-$C_6$ alkoxy optionally

substituted with halogen or cyano;

$R_6$ are each independently selected from the group consisting of hydrogen, deuterium, halogen and cyano; and n is an integer of 1-4.

In some embodiments, $R_1$ is selected from the group consisting of hydrogen, deuterium, $C_1$-$C_3$ alkyl optionally substituted with halogen or deuterium, and halogen;

$R_2$ are each independently selected from the group consisting of hydrogen, deuterium, $C_1$-$C_3$ alkyl optionally substituted with halogen or deuterium, and halogen;
$R_3$ and $R_4$ are each independently hydrogen or halogen, or $R_3$ and $R_4$ form, together with the carbon atom to which they are attached, $C_3$-$C_6$ cyclohydrocarbylene optionally substituted with halogen;
$R_5$ is $C_1$-$C_6$ alkyl or $C_1$-$C_6$ alkoxy;
$R_6$ are each independently selected from the group consisting of hydrogen, deuterium, halogen and cyano;
$R_7$ and $R_8$ are each independently selected from the group consisting of:

a) hydrogen, deuterium, halogen, cyano, amino, hydroxy, $C_1$-$C_6$ alkyl optionally substituted with halogen, sulfone, sulfoxide, sulfonamide, sulfenamide, $C_{1-3}$ carboxyl, and $C_1$-$C_6$ alkoxy optionally substituted with halogen;
b)

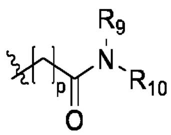 , 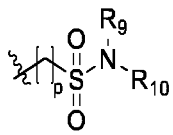 and 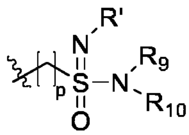 ,

wherein p is selected from the group consisting of 0, 1 and 2; $R_9$ and $R_{10}$ are each independently selected from the group consisting of hydrogen, $C_1$-$C_6$ alkyl, $C_1$-$C_6$ alkoxy, and $C_3$-$C_8$ cyclohydrocarbyl, or $R_9$ and $R_{10}$ form, together with the nitrogen atom to which they are attached, 4- to 6-membered heterocyclyl, wherein the heterocyclyl is optionally substituted with one or more substituents selected from the group consisting of halogen, hydroxy, cyano, $C_1$-$C_6$ haloalkyl, and $C_1$-$C_6$ alkyl; and in

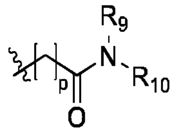 ,

when p is 0, the combination of $R_9$ and $R_{10}$ is not hydrogen and methyl;
c)

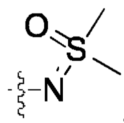 ;

d) heterocyclyl and heteroaryl, wherein the heterocyclyl and heteroaryl are each optionally substituted with one or more substituents selected from the group consisting of oxo, halogen, hydroxy, carbonyl, $C_1$-$C_6$ alkyl and cyano, wherein the $C_1$-$C_6$ alkyl is optionally substituted with one or more halogens; and
e)

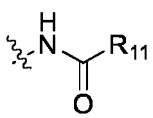 , 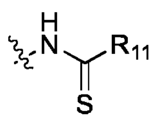 , 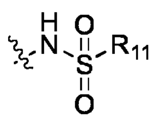 and 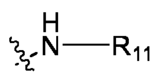 ,

wherein $R_{11}$ is selected from the group consisting of $C_1$-$C_6$ alkyl, $C_1$-$C_6$ alkoxy, aryl, heteroaryl, $C_3$-$C_8$ cyclohy-

drocarbyl, heterocyclyl, $C_1$-$C_6$ cyanoalkyl, $C_3$-$C_8$ cyclohydrocarbyloxy, and amino optionally substituted with $C_1$-$C_6$ alkyl; or

$R_7$ and $R_8$ form, together with the atom to which they are attached, an optionally substituted aromatic or non-aromatic heterocyclic ring;

m is an integer of 1-3; and

n is an integer of 1-4.

In some embodiments, in the compound of formula (I),

$R_1$ is selected from the group consisting of hydrogen, deuterium, halogen, $C_1$-$C_6$ hydroxyalkyl, $C_1$-$C_6$ alkyl optionally substituted with halogen or deuterium, and $C_1$-$C_6$ alkoxy optionally substituted with halogen or deuterium;
$R_2$ are each independently selected from the group consisting of hydrogen, deuterium, halogen, $C_1$-$C_6$ hydroxyalkyl, $C_1$-$C_6$ alkyl optionally substituted with halogen or deuterium, and $C_1$-$C_6$ alkoxy optionally substituted with halogen or deuterium;
$R_3$ and $R_4$ are each independently selected from the group consisting of hydrogen, halogen and $C_1$-$C_4$ alkyl optionally substituted with halogen, or $R_3$ and $R_4$ form, together with the carbon atom to which they are attached, $C_3$-$C_6$ cyclohydrocarbylene optionally substituted with halogen, or $R_3$ and $R_4$ on adjacent carbon atoms together form $C_3$-$C_8$ cyclohydrocarbyl optionally substituted with halogen;
$R_5$ is selected from the group consisting of $C_1$-$C_6$ alkyl optionally substituted with halogen or cyano, $C_3$-$C_6$ cyclohydrocarbyl optionally substituted with halogen or cyano, heterocyclyl optionally substituted with halogen or cyano, $C_1$-$C_6$ alkoxy optionally substituted with halogen or cyano, and amino optionally substituted with alkyl;
$R_6$ are each independently selected from the group consisting of hydrogen, deuterium, halogen, cyano, cyclopropyl, and $C_1$-$C_6$ alkyl optionally substituted with halogen or deuterium;
$R_7$ is the following group optionally substituted with one or more substituents selected from the group consisting of methyl, a fluorine atom, a chlorine atom, halomethyl and cyano:

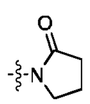 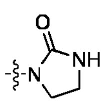 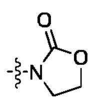 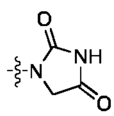 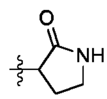 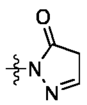 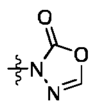 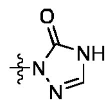 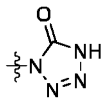 ,

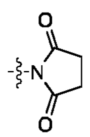 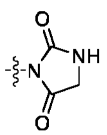 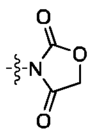 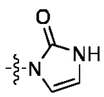 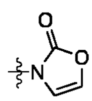 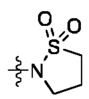 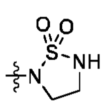 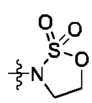 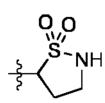 ,

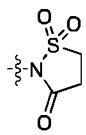 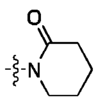 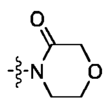 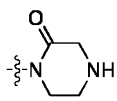 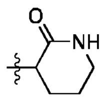 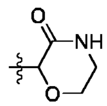 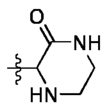 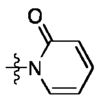 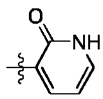

and

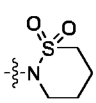 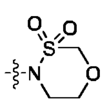 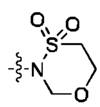 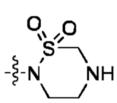 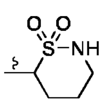 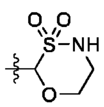 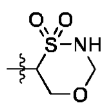 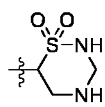 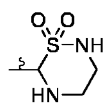 ;

Rg is selected from the group consisting of hydrogen, deuterium, halogen, cyano, and $C_1$-$C_3$ alkyl substituted with one or more halogens;
X is selected from the group consisting of an oxygen atom, -NH- and methylene, wherein the methylene is optionally substituted with one or more substituents selected from the group consisting of halogen, $C_3$-$C_8$ cyclohydrocarbyl, $C_3$-$C_6$ cyclohydrocarbylene, and $C_1$-$C_6$ alkyl;

m is an integer of 1-3; and
n is an integer of 1-4.

In some embodiments, in the compound of formula (I),

$R_1$ is selected from the group consisting of hydrogen, deuterium, halogen, $C_1$-$C_6$ hydroxyalkyl, $C_1$-$C_6$ alkyl optionally substituted with halogen or deuterium, and $C_1$-$C_6$ alkoxy optionally substituted with halogen or deuterium;
$R_2$ are each independently selected from the group consisting of hydrogen, deuterium, halogen, $C_1$-$C_6$ hydroxyalkyl, $C_1$-$C_6$ alkyl optionally substituted with halogen or deuterium, and $C_1$-$C_6$ alkoxy optionally substituted with halogen or deuterium;
$R_3$ and $R_4$ are each independently selected from the group consisting of hydrogen, halogen and $C_1$-$C_4$ alkyl optionally substituted with halogen, or $R_3$ and $R_4$ form, together with the carbon atom to which they are attached, $C_3$-$C_6$ cyclohydrocarbylene optionally substituted with halogen, or $R_3$ and $R_4$ on adjacent carbon atoms together form $C_3$-$C_8$ cyclohydrocarbyl optionally substituted with halogen;
$R_5$ is selected from the group consisting of $C_1$-$C_6$ alkyl optionally substituted with halogen or cyano, $C_3$-$C_6$ cyclohydrocarbyl optionally substituted with halogen or cyano, heterocyclyl optionally substituted with halogen or cyano, $C_1$-$C_6$ alkoxy optionally substituted with halogen or cyano, and amino optionally substituted with alkyl;
$R_7$ and $R_8$ form, together with the atom to which they are attached, a heterocyclic ring A

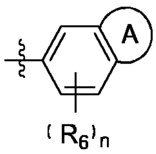
,

wherein the heterocyclic ring A is selected from the group consisting of the following structures:

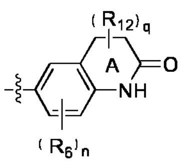 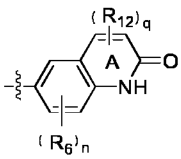 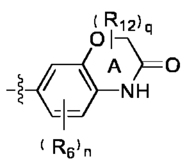 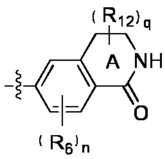 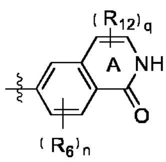 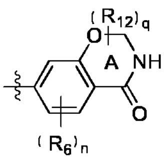

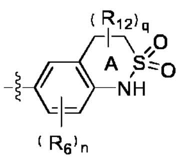 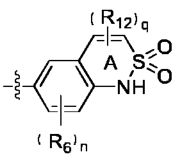 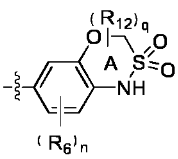 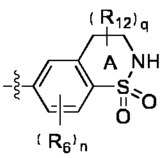 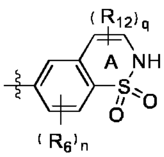 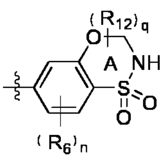

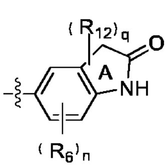 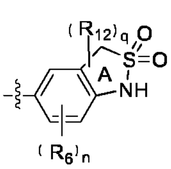 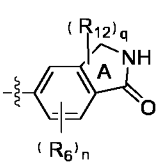 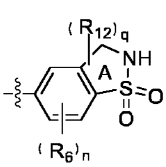 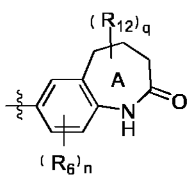 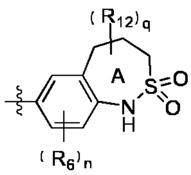

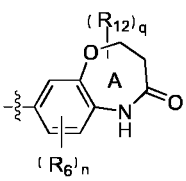 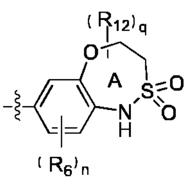 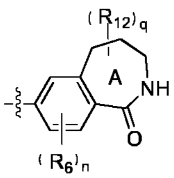 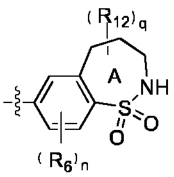 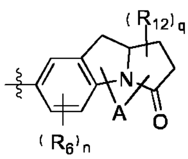 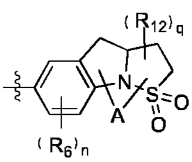

;

$R_6$ are each independently selected from the group consisting of hydrogen, deuterium, halogen and cyano;
$R_{12}$ is independently selected from the group consisting of halogen, $C_1$-$C_3$ alkyl, and $C_3$-$C_6$ cyclohydrocarbylene, or adjacent $R_{12}$ together form a ring, wherein the ring is optionally substituted with one or more halogens or $C_1$-$C_3$ alkyl;
m is an integer of 1-3;
n is an integer of 1-3; and
q is an integer of 0-6.

In some embodiments, in the compound of formula (I),

$R_1$ is selected from the group consisting of hydrogen, deuterium, $C_1$-$C_3$ alkyl optionally substituted with halogen or deuterium, and halogen;
$R_2$ are each independently selected from the group consisting of hydrogen, deuterium, $C_1$-$C_3$ alkyl optionally substituted with halogen or deuterium, and halogen;
$R_3$ and $R_4$ are each independently hydrogen or halogen, or $R_3$ and $R_4$ form, together with the carbon atom to which they are attached, $C_3$-$C_6$ cyclohydrocarbylene optionally substituted with halogen;
$R_5$ is $C_1$-$C_6$ alkyl or $C_1$-$C_6$ alkoxy;
$R_7$ and $R_8$ form, together with the atom to which they are attached, a heterocyclic ring A

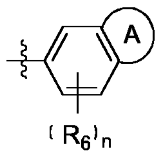
,

wherein the heterocyclic ring A is selected from the group consisting of the following structures:

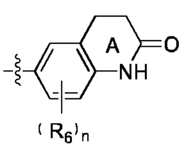 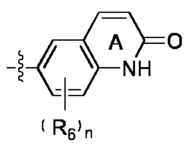 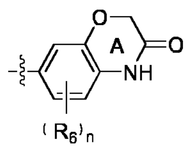 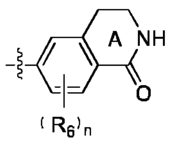 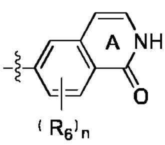 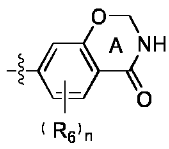

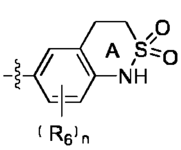 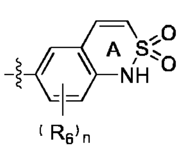 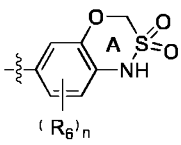 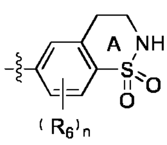 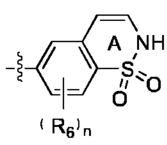 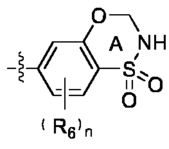

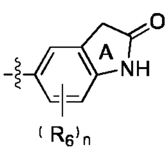 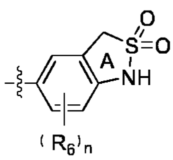 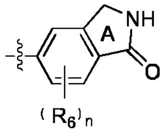 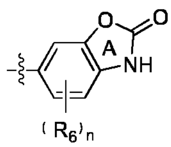 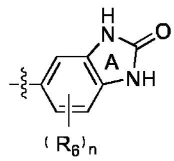 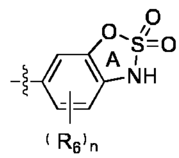

;

$R_6$ are each independently selected from the group consisting of hydrogen, deuterium, halogen and cyano;
m is an integer of 1-3; and
n is an integer of 1-3.

In some embodiments, in the compound of formula (I),

$R_1$ is selected from the group consisting of hydrogen, deuterium, halogen, $C_1$-$C_6$ hydroxyalkyl, $C_1$-$C_6$ alkyl optionally substituted with halogen or deuterium, and $C_1$-$C_6$ alkoxy optionally substituted with halogen or deuterium;
$R_2$ are each independently selected from the group consisting of hydrogen, deuterium, halogen, $C_1$-$C_6$ hydroxyalkyl, $C_1$-$C_6$ alkyl optionally substituted with halogen or deuterium, and $C_1$-$C_6$ alkoxy optionally substituted with halogen or deuterium;

$R_3$ and $R_4$ are each independently selected from the group consisting of hydrogen, halogen and $C_1$-$C_4$ alkyl optionally substituted with halogen, or $R_3$ and $R_4$ form, together with the carbon atom to which they are attached, $C_3$-$C_6$ cyclohydrocarbylene optionally substituted with halogen, or $R_3$ and $R_4$ on adjacent carbon atoms together form $C_3$-$C_8$ cyclohydrocarbyl optionally substituted with halogen;
$R_5$ is selected from the group consisting of $C_1$-$C_6$ alkyl optionally substituted with halogen or cyano, $C_3$-$C_6$ cyclohydrocarbyl optionally substituted with halogen or cyano, heterocyclyl optionally substituted with halogen or cyano, $C_1$-$C_6$ alkoxy optionally substituted with halogen or cyano, and amino optionally substituted with alkyl;
$R_6$ are each independently selected from the group consisting of hydrogen, deuterium, halogen, cyano, cyclopropyl, and $C_1$-$C_6$ alkyl optionally substituted with halogen or deuterium;
$R_7$ is selected from the group consisting of the following groups:

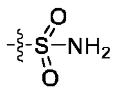 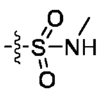 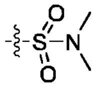 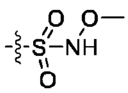 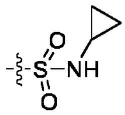 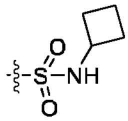 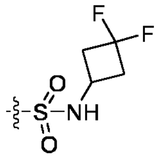 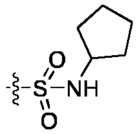

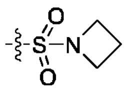 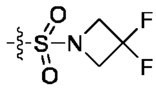 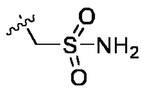 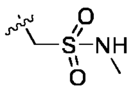 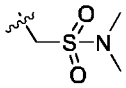 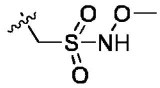

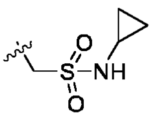 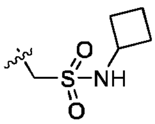 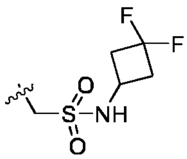 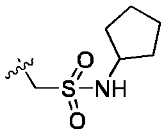 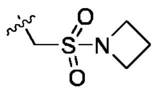 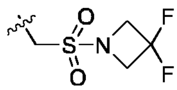 ;

$R_8$ is selected from the group consisting of hydrogen, deuterium, halogen, cyano, and $C_1$-$C_3$ alkyl substituted with one or more halogens;
X is selected from the group consisting of an oxygen atom, -NH- and methylene, wherein the methylene is optionally substituted with one or more substituents selected from the group consisting of halogen, $C_3$-$C_8$ cyclohydrocarbyl, $C_3$-$C_6$ cyclohydrocarbylene, and $C_1$-$C_6$ alkyl;
m is an integer of 1-3; and
n is an integer of 1-4.

In some embodiments, in the compound of formula (I),

$R_1$ is selected from the group consisting of hydrogen, deuterium, halogen, $C_1$-$C_6$ hydroxyalkyl, $C_1$-$C_6$ alkyl optionally substituted with halogen or deuterium, and $C_1$-$C_6$ alkoxy optionally substituted with halogen or deuterium;
$R_2$ are each independently selected from the group consisting of hydrogen, deuterium, halogen, $C_1$-$C_6$ hydroxyalkyl, $C_1$-$C_6$ alkyl optionally substituted with halogen or deuterium, and $C_1$-$C_6$ alkoxy optionally substituted with halogen or deuterium;
$R_3$ and $R_4$ are each independently selected from the group consisting of hydrogen, halogen and $C_1$-$C_4$ alkyl optionally substituted with halogen, or $R_3$ and $R_4$ form, together with the carbon atom to which they are attached, $C_3$-$C_6$ cyclohydrocarbylene optionally substituted with halogen, or $R_3$ and $R_4$ on adjacent carbon atoms together form $C_3$-$C_8$ cyclohydrocarbyl optionally substituted with halogen;
$R_5$ is selected from the group consisting of $C_1$-$C_6$ alkyl optionally substituted with halogen or cyano, $C_3$-$C_6$ cyclohydrocarbyl optionally substituted with halogen or cyano, heterocyclyl optionally substituted with halogen or cyano, $C_1$-$C_6$ alkoxy optionally substituted with halogen or cyano, and amino optionally substituted with alkyl;
$R_6$ are each independently selected from the group consisting of hydrogen, deuterium, halogen, cyano, cyclopropyl, and $C_1$-$C_6$ alkyl optionally substituted with halogen or deuterium;
$R_7$ is selected from the group consisting of the following groups:

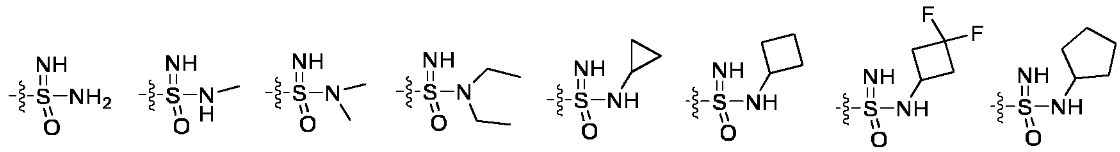

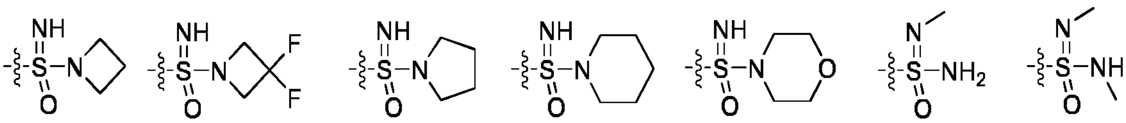

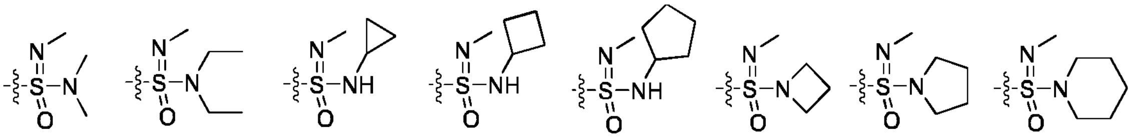

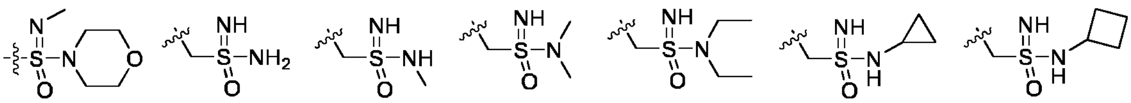

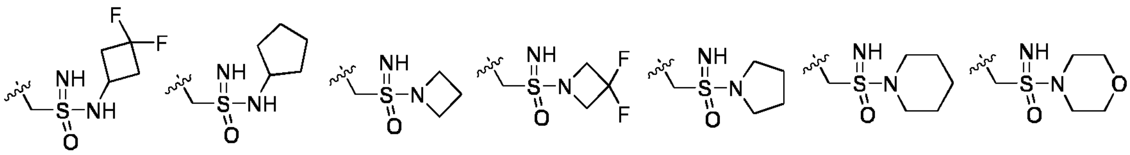

;

$R_8$ is selected from the group consisting of hydrogen, deuterium, halogen, cyano, and $C_1$-$C_3$ alkyl substituted with one or more halogens;

X is selected from the group consisting of an oxygen atom, -NH- and methylene, wherein the methylene is optionally substituted with one or more substituents selected from the group consisting of halogen, $C_3$-$C_8$ cyclohydrocarbyl, $C_3$-$C_6$ cyclohydrocarbylene, and $C_1$-$C_6$ alkyl;

m is an integer of 1-3; and

n is an integer of 1-4.

The present disclosure also provides a compound of formula (I-1) or a pharmaceutically acceptable salt or isomer thereof,

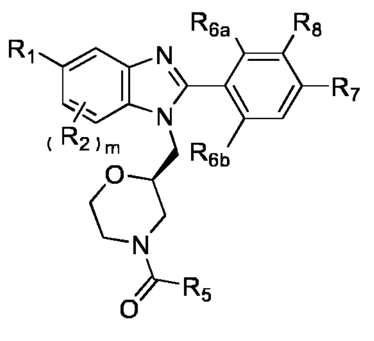

(I-1)

wherein $R_1$ is selected from the group consisting of hydrogen, deuterium, $C_1$-$C_3$ alkyl optionally substituted with halogen or deuterium, and halogen;

$R_2$ are each independently selected from the group consisting of hydrogen, deuterium, $C_1$-$C_3$ alkyl optionally substituted with halogen or deuterium, and halogen;

$R_5$ is $C_1$-$C_3$ alkyl or $C_1$-$C_3$ alkoxy;

$R_{6a}$ and $R_{6b}$ are each independently selected from the group consisting of hydrogen, deuterium, a chlorine atom, a fluorine atom and cyano;

$R_7$ is a 4- to 6-membered heterocyclyl, wherein the heterocyclyl comprises -NH-C(=O)- or -NH-S(=O)$_2$-, and the heterocyclyl is optionally substituted with one or more substituents selected from the group consisting of oxo, halogen, hydroxy, carbonyl, $C_1$-$C_3$ alkyl and cyano, wherein the $C_1$-$C_3$ alkyl is optionally substituted with one or more halogens;

$R_8$ is selected from the group consisting of hydrogen, deuterium, halogen, cyano and $C_1$-$C_3$ alkyl substituted with one or more halogens; and

m is an integer of 1-3.

In some embodiments, in the compound of formula (I-1), $R_7$ is the following group optionally substituted with one or more substituents selected from the group consisting of methyl, a fluorine atom, a chlorine atom, halomethyl and cyano:

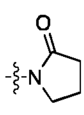 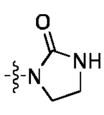 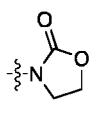 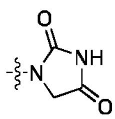 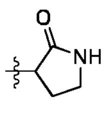 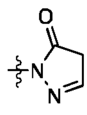 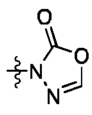 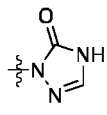 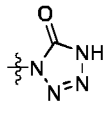

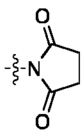 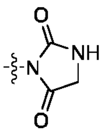 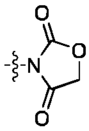 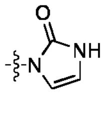 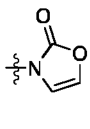 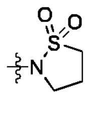 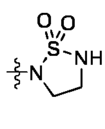 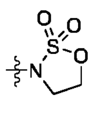 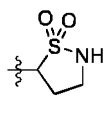

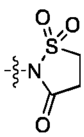 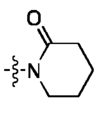 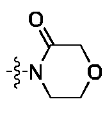 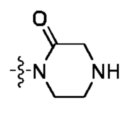 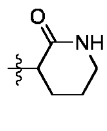 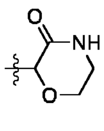 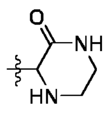 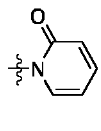 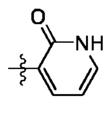

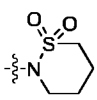 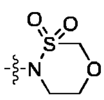 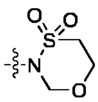 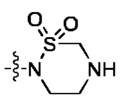 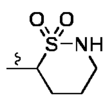 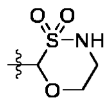 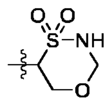 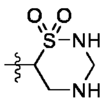 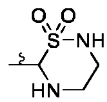

; and

$R_8$ is selected from the group consisting of hydrogen, deuterium, halogen and cyano. The present disclosure also provides a compound of formula (I-1) or a pharmaceutically acceptable salt or isomer thereof,

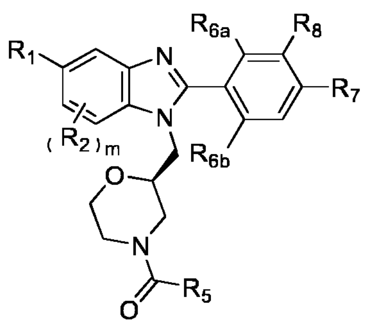

(I-1)

wherein $R_1$ is selected from the group consisting of hydrogen, deuterium, $C_1$-$C_3$ alkyl optionally substituted with halogen or deuterium, and halogen;

$R_2$ are each independently selected from the group consisting of hydrogen, deuterium, $C_1$-$C_3$ alkyl optionally substituted with halogen or deuterium, and halogen;

$R_5$ is $C_1$-$C_3$ alkyl or $C_1$-$C_3$ alkoxy;

$R_{6a}$ and $R_{6b}$ are each independently selected from the group consisting of hydrogen, deuterium, a chlorine atom, a fluorine atom and cyano;

$R_7$ and $R_8$ form, together with the atom to which they are attached, a 4- to 8-membered non-aromatic heterocyclic ring, wherein the heterocyclic ring is monocyclic or bicyclic, the heterocyclic ring comprises -NH-C(=O)- or -NH-S(=O)$_2$-, and the heterocyclic ring is optionally substituted with one or more substituents selected from the group consisting of $C_1$-$C_3$ alkylamide, halogen, oxo, $C_1$-$C_3$ alkyl optionally substituted with halogen, and $C_1$-$C_3$ alkoxy; and

m is an integer of 1-3.

In some embodiments, in the compound of formula (I-1), $R_7$ and $R_8$ form, together with the atom to which they are attached, heterocyclic ring A

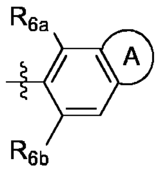

,

wherein the heterocyclic ring A is selected from the group consisting of the following structures:

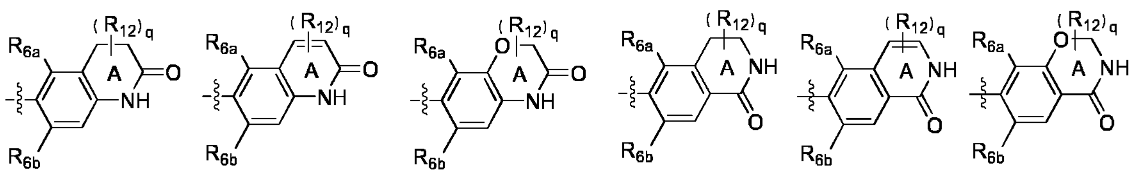

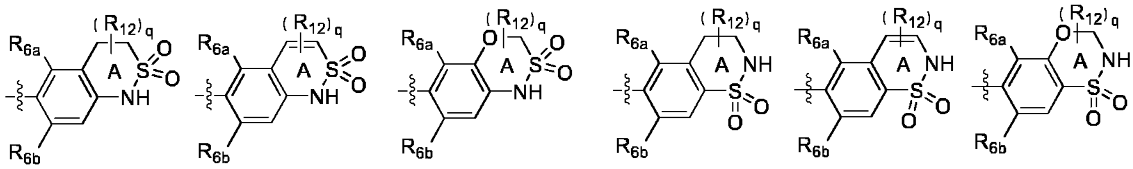

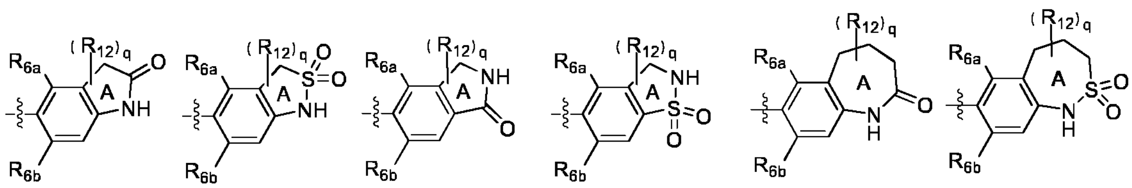

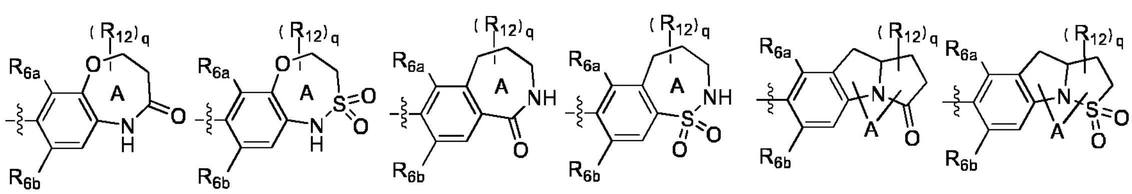

$R_{6a}$ and $R_{6b}$ are each independently selected from the group consisting of hydrogen, deuterium, a chlorine atom and a fluorine atom;

$R_{12}$ are each independently selected from the group consisting of halogen, $C_1$-$C_3$ alkyl, and $C_3$-$C_6$ cyclohydrocarbylene, or adjacent $R_{12}$ together form a ring, wherein the ring is optionally substituted with one or more halogens or $C_1$-$C_3$ alkyl; and

q is an integer of 0-6.

In some embodiments, in the compound of formula (I-1), $R_7$ and $R_8$ form, together with the atom to which they are attached, heterocyclic ring A

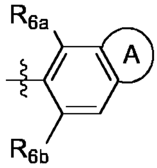

,

wherein the heterocyclic ring A is selected from the group consisting of the following structures:

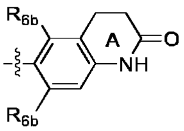 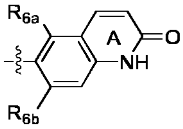 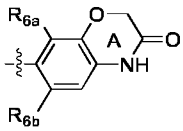 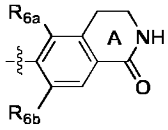 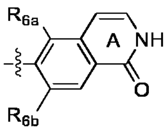 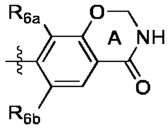

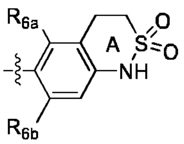 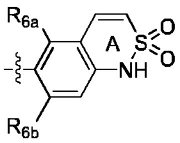 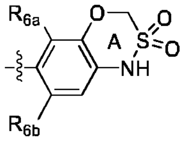 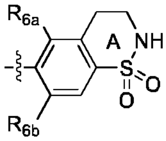 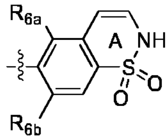 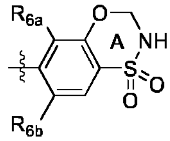

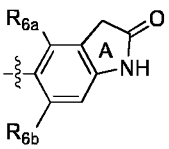 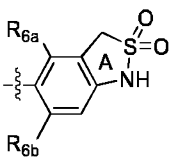 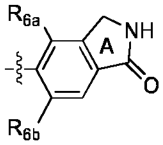 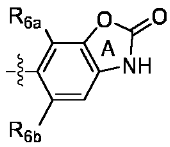 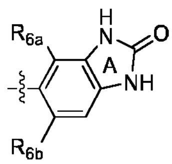 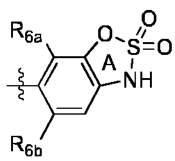

; and

$R_{6a}$ and $R_{6b}$ are each independently selected from the group consisting of hydrogen, deuterium, a chlorine atom and a fluorine atom.

The present disclosure also provides a compound of formula (I-1) or a pharmaceutically acceptable salt or isomer thereof,

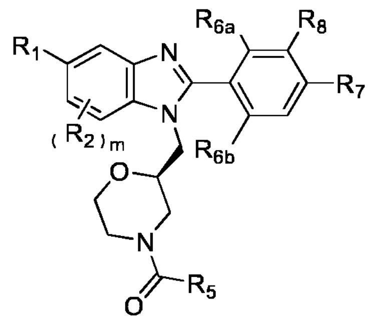

(I-1)

wherein $R_1$ is selected from the group consisting of hydrogen, deuterium, $C_1$-$C_3$ alkyl optionally substituted with halogen or deuterium, and halogen;

$R_2$ are each independently selected from the group consisting of hydrogen, deuterium, $C_1$-$C_3$ alkyl optionally substituted with halogen or deuterium, and halogen;
$R_5$ is $C_1$-$C_3$ alkyl or $C_1$-$C_3$ alkoxy;
$R_{6a}$ and $R_{6b}$ are each independently selected from the group consisting of hydrogen, deuterium, a chlorine atom, a fluorine atom and cyano;

$R_7$ is

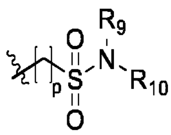

,

wherein p is selected from the group consisting of 0, 1 and 2; $R_9$ and $R_{10}$ are each independently selected from the group consisting of hydrogen, $C_1$-$C_3$ alkyl, $C_1$-$C_3$ alkoxy and $C_3$-$C_6$ cyclohydrocarbyl, or $R_9$ and $R_{10}$ form, together with the nitrogen atom to which they are attached, 4- to 6-membered heterocyclyl, wherein the heterocyclyl is optionally substituted with one or more substituents selected from the group consisting of halogen, hydroxy and $C_1$-$C_3$ alkyl;

$R_8$ is selected from the group consisting of hydrogen, deuterium, halogen, cyano and $C_1$-$C_3$ alkyl substituted with one or more halogens; and

m is an integer of 1-3.

In some embodiments, in the compound of formula (I-1), $R_7$ is selected from the group consisting of the following groups:

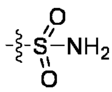 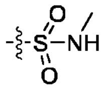 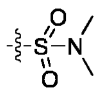 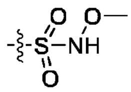 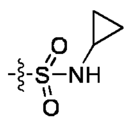 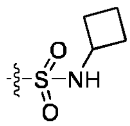 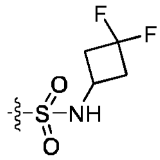 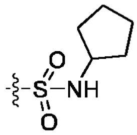

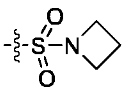 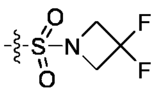 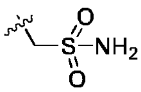 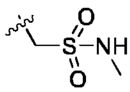 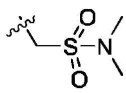 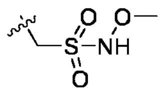

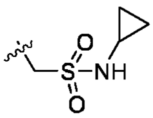 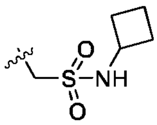 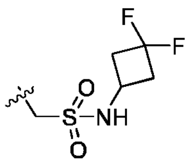 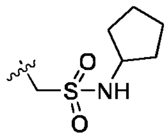 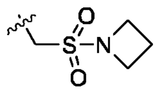 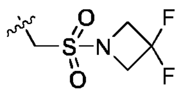

;

and

$R_8$ is selected from the group consisting of hydrogen, deuterium, halogen, cyano, and $C_1$-$C_3$ alkyl substituted with one or more halogens.

The present disclosure also provides a compound of formula (I-1) or a pharmaceutically acceptable salt or isomer thereof,

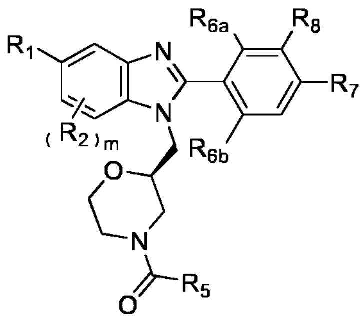

(I-1)

wherein $R_1$ is selected from the group consisting of hydrogen, deuterium, $C_1$-$C_3$ alkyl optionally substituted with halogen or deuterium, and halogen;

$R_2$ are each independently selected from the group consisting of hydrogen, deuterium, $C_1$-$C_3$ alkyl optionally substituted with halogen or deuterium, and halogen;
$R_5$ is $C_1$-$C_3$ alkyl or $C_1$-$C_3$ alkoxy;
$R_{6a}$ and $R_{6b}$ are each independently selected from the group consisting of hydrogen, deuterium, a chlorine atom, a fluorine atom and cyano;
$R_7$ is

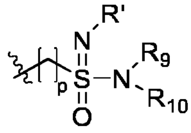,

wherein p is selected from the group consisting of 0, 1 and 2; $R_9$ and $R_{10}$ are each independently selected from the group consisting of hydrogen, $C_1$-$C_3$ alkyl, $C_1$-$C_3$ alkoxy and $C_3$-$C_6$ cyclohydrocarbyl, or $R_9$ and $R_{10}$ form, together with the nitrogen atom to which they are attached, 4- to 6-membered heterocyclyl, wherein the heterocyclyl is optionally substituted with one or more substituents selected from the group consisting of halogen, hydroxy and $C_1$-$C_3$ alkyl; R' is selected from the group consisting of hydrogen, $C_1$-$C_3$ alkyl, $C_3$-$C_6$ cycloalkyl, aryl and heteroaryl;
$R_8$ is selected from the group consisting of hydrogen, deuterium, halogen, cyano and $C_1$-$C_3$ alkyl substituted with one or more halogens; and
m is an integer of 1-3.

In some embodiments, in the compound of formula (I-1), $R_7$ is selected from the group consisting of the following groups:

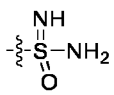 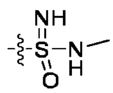 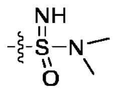 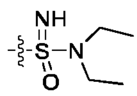 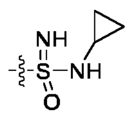 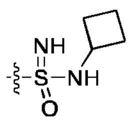 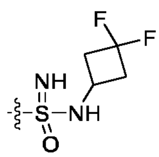 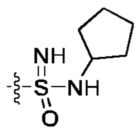

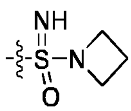 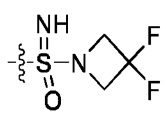 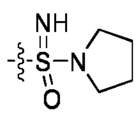 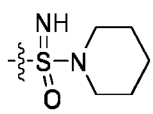 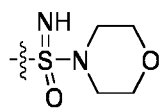 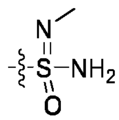 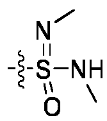

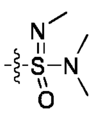 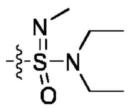 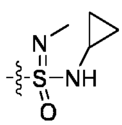 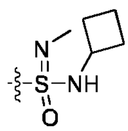 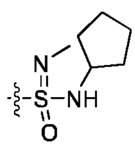 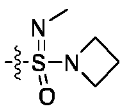 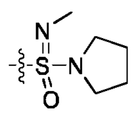 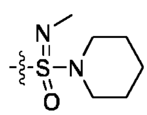

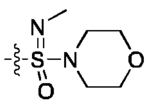 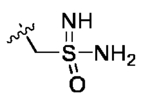 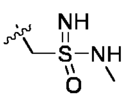 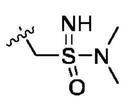 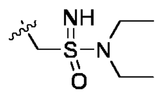 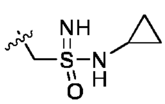 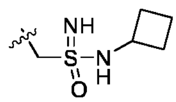

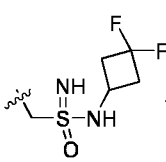 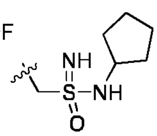 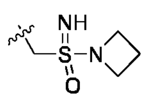 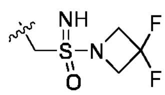 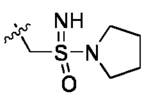 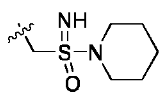 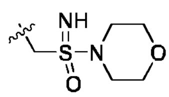

; and
$R_8$ is selected from the group consisting of hydrogen, deuterium, halogen, cyano, and $C_1$-$C_3$ alkyl substituted with one

or more halogens.

The present disclosure also provides a compound as shown below or a pharmaceutically acceptable salt or isomer thereof,

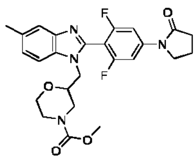, 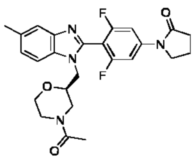, 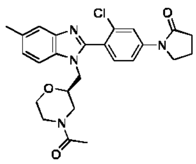, 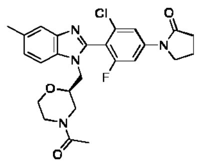, 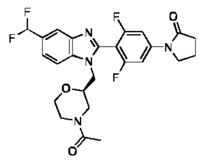,

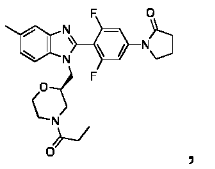, 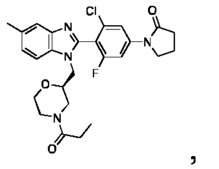, 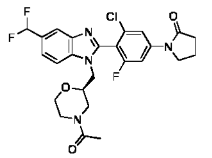, 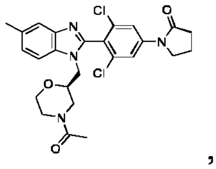, 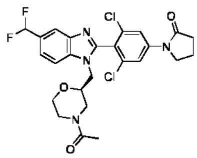,

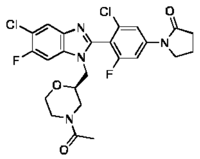, 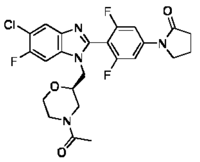, 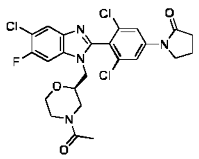, 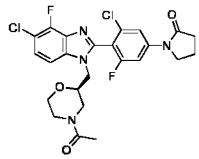, 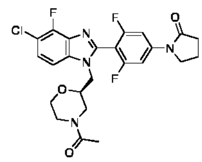,

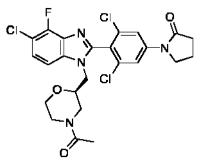, 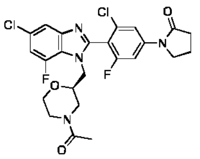, 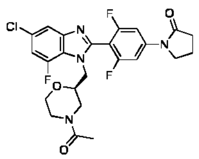, 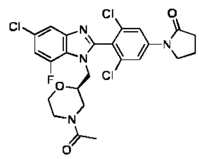, 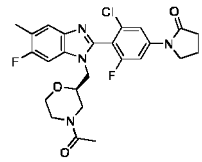,

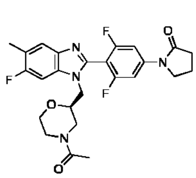, 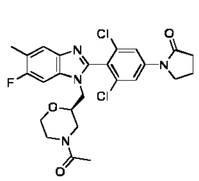, 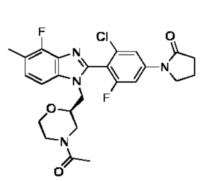, 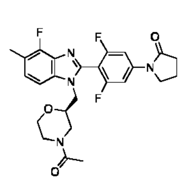, 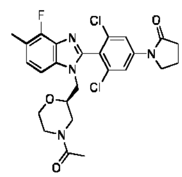,

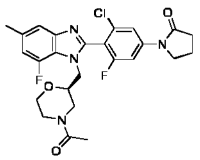, 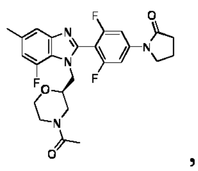, 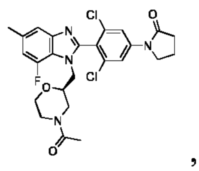, 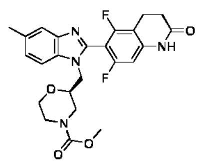, 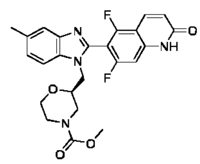,

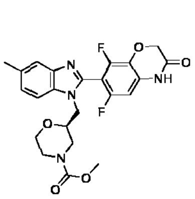, 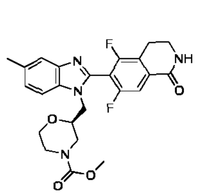, 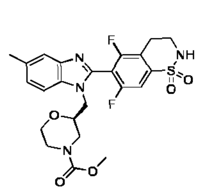, 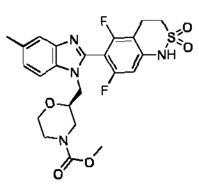, 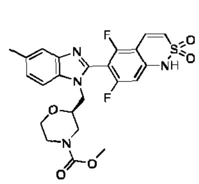,

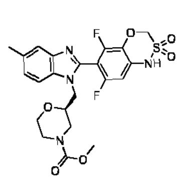, 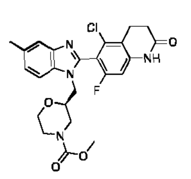, 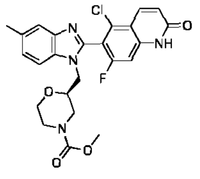, 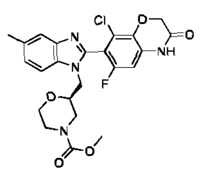, 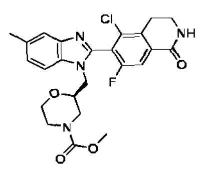,

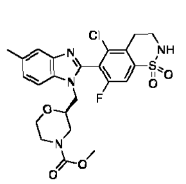, 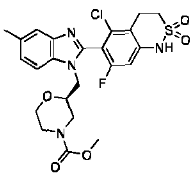, 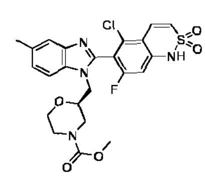, 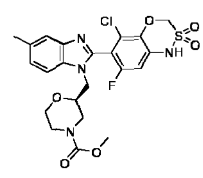, 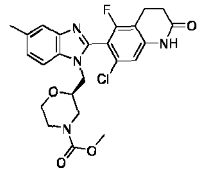,

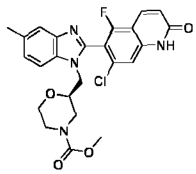, 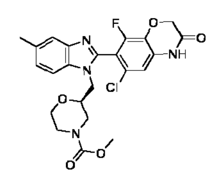, 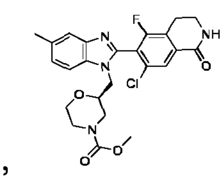, 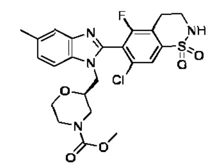, 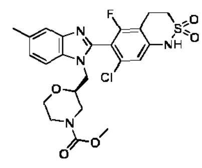,

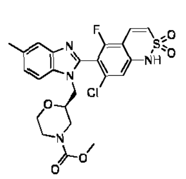, 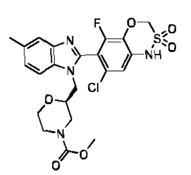, 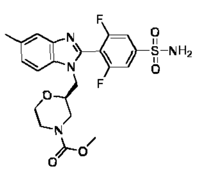, 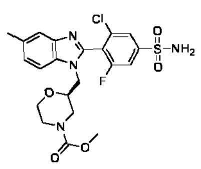, 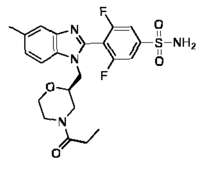,

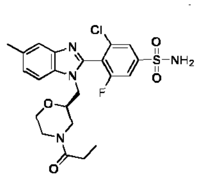, 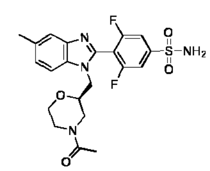, 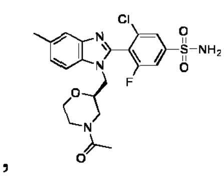, 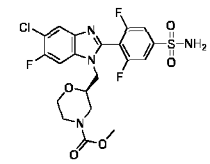, 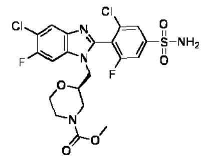,

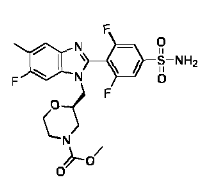, 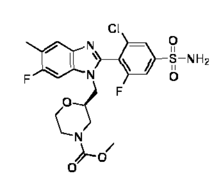, 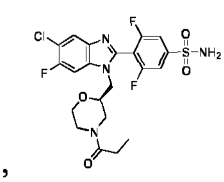, 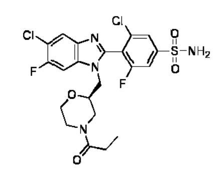, 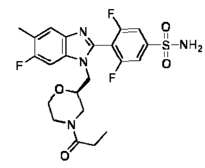,

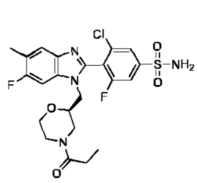, 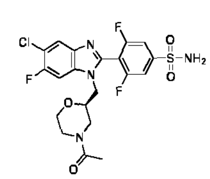, 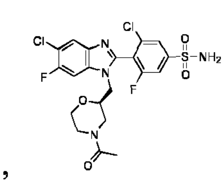, 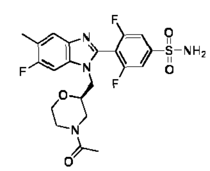, 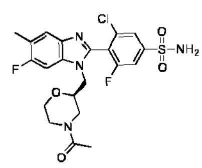,

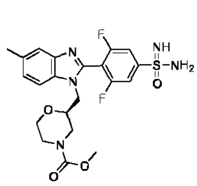, 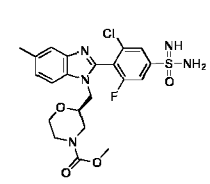, 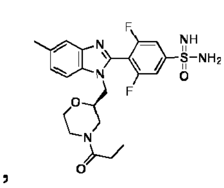, 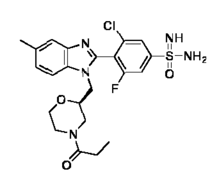, 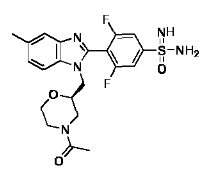,

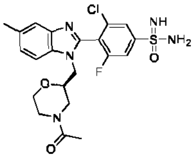,

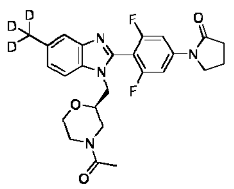,

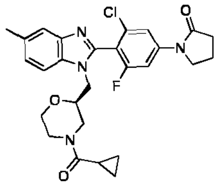,

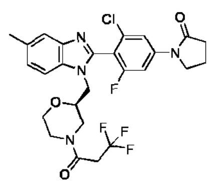,

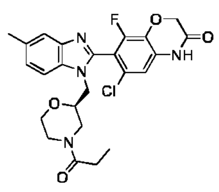

and

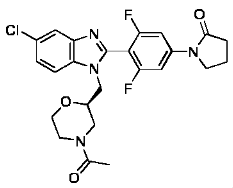.

The present disclosure provides a method for preparing a compound of formula (I) or a pharmaceutically acceptable salt or isomer thereof, which comprises the following steps:

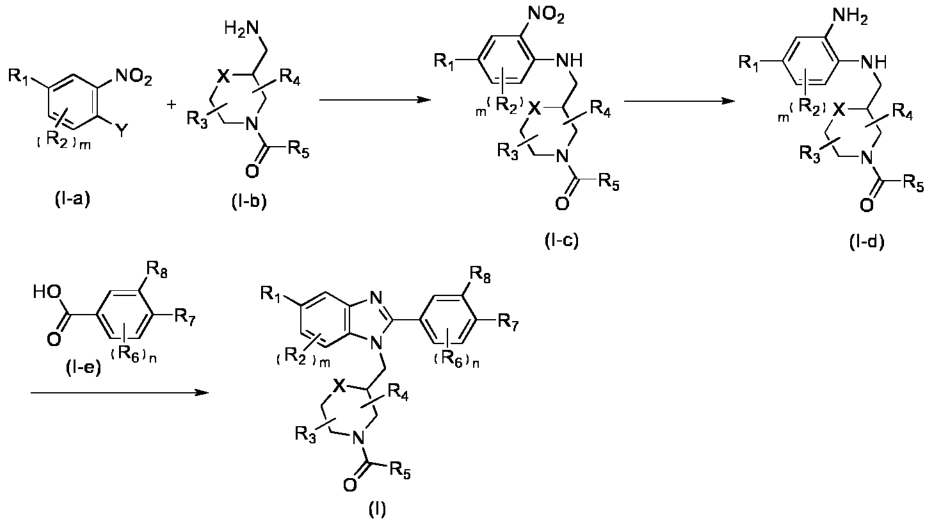

(I-a) (I-b) (I-c) (I-d) (I-e) (I)

subjecting a compound of formula (I-a) to a reaction with a compound of formula (I-b) under an alkaline condition to give a compound of formula (I-c); subjecting the compound of formula (I-c) to a reduction reaction to give a compound of formula (I-d); subjecting the compound of formula (I-d) to a ring closure reaction with a compound of formula (I-e) under an acidic condition to give the compound of formula (I);

wherein $R_1$, $R_2$, $R_3$, $R_4$, $R_5$, $R_6$, $R_7$, $R_8$, X, m and n are as defined in the compound of formula (I); and Y is selected from the group consisting of halogen, sulfonyl and sulfinyl.

The present disclosure also provides another method for preparing a compound of formula (I) or a pharmaceutically acceptable salt or isomer thereof, which comprises the following steps:

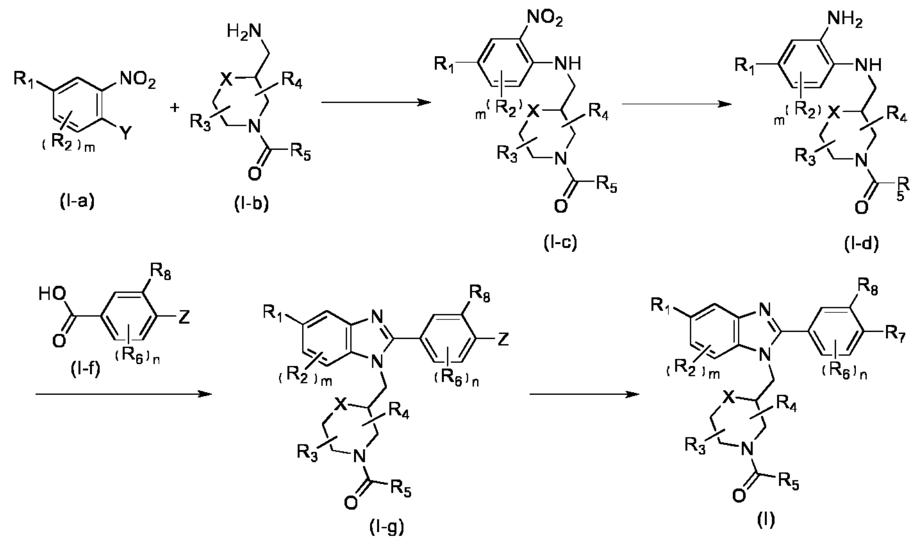

subjecting a compound of formula (I-a) to a reaction with a compound of formula (I-b) under an alkaline condition to give a compound of formula (I-c); subjecting the compound of formula (I-c) to a reduction reaction to give a compound of formula (I-d); subjecting the compound of formula (I-d) to a ring closure reaction with a compound of formula (I-e) under an acidic condition to give a compound of formula (I-g); and subjecting the compound of formula (I-g) under the action of a catalyst to give the compound of formula (I);

wherein the catalyst is selected from the group consisting of palladium/carbon, Raney Ni, tetrakis(triphenylphosphine)palladium(0), palladium dichloride, palladium acetate, [1,1'-bis(diphenylphosphino)ferrocene]palladium(II) dichloride, 1,1'-bis(dibenzylphosphino)ferrocene-palladium(II)dichloride, tris(dibenzylideneacetone)dipalladium(0), 2-dicyclohexylphosphino-2',6'-dimethoxybiphenyl, [1,1'-bis(di-*tert*-butylphosphino)ferrocene]palladium(II) dichlorine, cuprous iodide, cuprous bromide, cuprous chloride and copper(II) trifluoromethanesulphonate;
$R_1$, $R_2$, $R_3$, $R_4$, $R_5$, $R_6$, $R_7$, $R_8$, X, m and n are as defined in the compound of formula I; and
Y and Z are each independently selected from the group consisting of halogen, sulfonyl and sulfinyl.

The present disclosure also provides a method for preparing the compound or the pharmaceutically acceptable salt or isomer thereof. In particular, the preparation is performed by the methods of the Examples.

The present disclosure also relates to a pharmaceutical composition comprising the compound or the pharmaceutically acceptable salt or isomer thereof described herein. Generally, the composition also comprises at least one pharmaceutically acceptable carrier, diluent or excipient.

In some embodiments, in the pharmaceutical composition, the compound or the pharmaceutically acceptable salt or isomer thereof is in a unit dose of 0.001mg-1000 mg.

In certain embodiments, the pharmaceutical composition comprises 0.01%-99.99% of the compound or the pharmaceutically acceptable salt thereof described above based on the total weight of the composition. In certain embodiments, the pharmaceutical composition comprises 0.1%-99.9% of the compound or the pharmaceutically acceptable salt thereof described above. In certain embodiments, the pharmaceutical composition comprises 0.5%-99.5% of the compound or the pharmaceutically acceptable salt thereof described above. In certain embodiments, the pharmaceutical composition comprises 1%-99% of the compound or the pharmaceutically acceptable salt thereof described above. In certain embodiments, the pharmaceutical composition comprises 2%-98% of the compound or the pharmaceutically acceptable salt thereof described above.

In certain embodiments, the pharmaceutical composition comprises 0.01%-99.99% of a pharmaceutically acceptable excipient based on the total weight of the composition. In certain embodiments, the pharmaceutical composition comprises 0.1%-99.9% of a pharmaceutically acceptable excipient. In certain embodiments, the pharmaceutical composition comprises 0.5%-99.5% of a pharmaceutically acceptable excipient. In certain embodiments, the pharmaceutical composition comprises 1%-99% of a pharmaceutically acceptable excipient. In certain embodiments, the pharmaceutical composition comprises 2%-98% of a pharmaceutically acceptable excipient.

The present disclosure also relates to use of the compound or the pharmaceutically acceptable salt or isomer thereof, or the pharmaceutical composition comprising the same in the preparation of a medicament for treating a disease related to P2X3 activity. The present disclosure also relates to the compound or the pharmaceutically acceptable salt or isomer thereof, or the pharmaceutical composition comprising the same, for use as a medicament.

The present disclosure also relates to the compound or the pharmaceutically acceptable salt or isomer thereof, or the pharmaceutical composition comprising the same, for use in treating a disease related to P2X3 activity.

The present disclosure also relates to a method for treating a disease related to P2X3 activity, which comprises administering to a patient in need a therapeutically effective amount of the compound or the pharmaceutically acceptable salt or isomer thereof, or the pharmaceutical composition comprising the same.

In some embodiments, the disease related to P2X3 activity refers to a disease related to P2X3 overactivity. The compound of the present disclosure is highly selective for P2X3 and can avoid loss of taste sensation. In some embodiments, the compound of the present disclosure has an antagonistic effect on a P2X3 homologous receptor more than 20-fold stronger than on a P2X2/3 heteromeric receptor. In some embodiments, the compound of the present disclosure has an antagonistic effect on a P2X3 homologous receptor more than 30-fold stronger than on a P2X2/3 heteromeric receptor. In some embodiments, the compound of the present disclosure has an antagonistic effect on a P2X3 homologous receptor more than 50-fold stronger than on a P2X2/3 heteromeric receptor. In some embodiments, the compound of the present disclosure has an antagonistic effect on a P2X3 homologous receptor more than 100-fold stronger than on a P2X2/3 heteromeric receptor.

The present disclosure also relates to use of the compound or the pharmaceutically acceptable salt or isomer thereof, or the pharmaceutical composition comprising the same in the preparation of a medicament for treating a disease such as pain, urinary tract diseases and cough.

The present disclosure also relates to the compound or the pharmaceutically acceptable salt or isomer thereof, or the pharmaceutical composition comprising the same, for use in treating diseases such as pain, urinary tract diseases and cough.

The present disclosure also relates to a method for treating pain, urinary tract diseases, cough, and the like, which comprises administering to a patient in need a therapeutically effective amount of the compound or the pharmaceutically acceptable salt or isomer thereof, or the pharmaceutical composition comprising the same.

In some embodiments, the pain may be, for example, chronic pain, neuropathic pain, acute pain, back pain, cancer pain, pain caused by rheumatoid arthritis, migraine, and visceral pain. The urinary tract disorders are, for example, overactive bladder (also known as urinary incontinence), pelvic hypersensitivity, and urethritis.

In some embodiments, the compound of the present disclosure or the pharmaceutically acceptable salt or isomer thereof, or the pharmaceutical composition comprising the same, can be used for treating gastrointestinal disorders, including, for example, constipation and functional gastrointestinal disorders (e.g., irritable bowel syndrome or functional dyspepsia); can be used for treating cancer; can be used for treating cardiovascular disorders or for cardioprotection after myocardial infarction; can be used as an immunomodulator, particularly in the treatment of autoimmune diseases (e.g. arthritis), in skin transplantation, organ transplantation or similar surgical needs, in collagen diseases or in allergies, or used as an anti-tumor or anti-viral agent; can be used for treating multiple sclerosis, Parkinson’s disease, and Huntington’s chorea; can be used for treating depression, anxiety, stress-related disorders (e.g., post-traumatic stress disorder, panic disorder, social phobia, or obsessive compulsive disorder), premature ejaculation, psychosis, traumatic brain injury, stroke, Alzheimer’s disease, spinal injury, drug addiction (e.g., abuse in alcohol, nicotine, opioid, or other drugs), or sympathetic nervous system disorders (e.g., hypertension); can be used for treating diarrhea; and can be used for treating pulmonary disorders such as asthma, cough or pulmonary edema. The compound or the pharmaceutically acceptable salt or isomer thereof of the present disclosure can be formulated in a dosage form suitable for oral, buccal, vaginal, rectal, inhalation, insufflation, intranasal, sublingual, topical, or parenteral (e.g., intramuscular, subcutaneous, intraperitoneal, intrathoracic, intravenous, epidural, intrathecal, intracerebroventricular, or by injection into the joints) administration.

The pharmaceutically acceptable salt of the compound described herein may be selected from the group consisting of inorganic or organic salts.

The term "treatment" refers to the administration of a pharmaceutical composition for prophylactic and/or therapeutic purposes. By "preventing a disease" is meant prophylactically treating a subject who has not yet developed a disease but is susceptible to, or is at risk of developing, a specific disease. By "treating a disease" is meant treating a patient who is suffering from a disease to improve or stabilize the patient’s condition.

Any isotopically-labeled (or radiolabeled) derivative of the compound or the pharmaceutically acceptable salt or isomer thereof described herein is encompassed by the present disclosure. Such derivatives are those in which one or more atoms are replaced with an atom whose atomic mass or mass number is different from that usually found in nature. Examples of radionuclides that may be incorporated include $^{2}H$ (also written as "D", i.e., deuterium), $^{3}H$ (also written as "T", i.e., tritium), $^{11}C$, $^{13}C$, $^{14}C$, $^{13}N$, $^{15}N$, $^{15}O$, $^{17}O$, $^{18}O$ $^{18}F$, $^{36}Cl$, $^{82}Br$, $^{75}Br$, $^{76}Br$, $^{77}Br$, $^{123}I$, $^{124}I$, $^{125}I$, $^{31}P$, $^{32}P$, $^{35}S$, and $^{131}I$. The radionuclide used will depend on the particular application of the radiolabeled derivative. For example, for *in vitro* receptor labeling and competition assays, $^{3}H$ or $^{14}C$ is often useful. For radiographic application, $^{11}C$ or $^{18}F$ is often useful. In some embodiments, the radionuclide is $^{3}H$. In some embodiments, the radionuclide is $^{14}C$. In some embodiments, the radionuclide is $^{11}C$. Moreover, in some embodiments, the radionuclide is $^{18}F$.

Unless otherwise stated, when a position is specifically designated as deuterium (D), that position shall be

understood to be deuterium having an abundance that is at least 3000 times greater than the natural abundance of deuterium (which is 0.015%) (i.e., incorporating at least 45% deuterium).

Unless otherwise stated, the following terms used in the specification and claims have the following meanings.

The term "pharmaceutical composition" refers to a mixture containing one or more of the compounds or the physiologically/pharmaceutically acceptable salts or pro-drugs thereof described herein, and other chemical components, for example, physiologically/pharmaceutically acceptable carriers and excipients. The pharmaceutical composition is intended to promote the administration to an organism, so as to facilitate the absorption of the active ingredient, thereby exerting biological activities.

The term "pharmaceutically acceptable excipient" includes, but is not limited to, any adjuvant, carrier, excipient, glidant, sweetener, diluent, preservative, dye/colorant, flavoring agent, surfactant, wetting agent, dispersant, suspending agent, stabilizer, isotonic agent, solvent, or emulsifier that has been approved by the U.S. food and drug administration as acceptable for use in humans or livestock animals.

"Effective amount" or "therapeutically effective amount" described herein includes an amount sufficient to ameliorate or prevent a symptom or condition of a medical condition. An effective amount also refers to an amount sufficient to allow or facilitate diagnosis. The effective amount for a particular patient or veterinary subject may vary with factors such as the condition to be treated, the general health of the patient, the method and route and dosage of administration, and the severity of side effects. An effective amount may be the maximum dose or administration regimen to avoid significant side effects or toxic effects.

In the chemical structure of the compound of the present disclosure, a bond "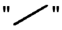" represents an unspecified configuration, that is, if chiral isomers exist in the chemical structure, the bond "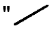" may be "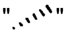" or "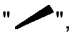", or contains both the configurations of "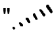" and "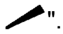". In the chemical structure of the compound described herein, a bond "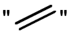" is not specified with a configuration, that is, the bond "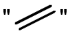" may be in an E configuration or a Z configuration, or contains both configurations of E and Z.

The compounds and intermediates of the present disclosure may also exist in different tautomeric forms, and all such forms are included within the scope of the present disclosure. The term "tautomer" or "tautomeric form" refers to structural isomers of different energies that can interconvert via a low energy barrier. For example, proton tautomers (also known as proton transfer tautomers) include interconversion via proton migration, such as keto-enol and imine-enamine, lactam-lactim isomerization. An example of a lactam-lactim equilibrium is present between A and B as shown below.

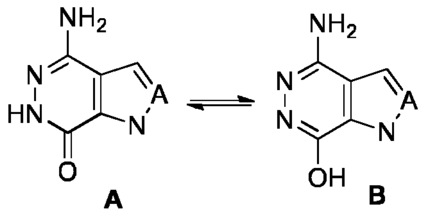

A B

"Halogen" refers to fluorine, chlorine, bromine and iodine.

All compounds in the present disclosure can be drawn as form A or form B. All tautomeric forms are within the scope of the present disclosure. The nomenclature of the compounds does not exclude any tautomers.

"Alkyl" refers to a linear or branched alkyl group, including linear and branched groups of 1 to 20 carbon atoms, preferably containing 1 to 6 carbon atoms, and more preferably 1 to 3 carbon atoms, e.g., methyl, ethyl, n-propyl, isopropyl and the like. The alkyl may be substituted or unsubstituted, and when it is substituted, the substitution with a substituent may be performed at any accessible connection site, wherein the substituent is preferably one or more groups independently selected from the group consisting of halogen, hydroxy, oxo, amino, $C_{1-6}$ alkyl, $C_{1-6}$ alkoxy, 3- to 6-membered cycloalkyl, 3-to 6-membered heterocycloalkyl, 6- to 10-membered aryl or 5- to 10-membered heteroaryl, wherein the alkyl, alkoxy, cycloalkyl, heterocycloalkyl, aryl or heteroaryl is optionally substituted with one or more halogens, hydroxy, amino, $C_{1-6}$ alkyl or $C_{1-6}$ alkoxy. "Alkoxy" refers to an alkyloxy group, wherein the alkyl is as defined above, e.g., methoxy, ethoxy and the like. The alkoxy may be optionally substituted or unsubstituted, and when it is substituted, the substituent is preferably one or more groups independently selected from the group consisting of halogen, hydroxy, oxo, nitro, cyano, amino, $C_{1-6}$ alkyl, $C_{1-6}$ alkoxy, 3- to 6-membered cycloalkyl, and 3- to 6-membered heterocycloalkyl, wherein the alkyl, alkoxy, cycloalkyl or heterocycloalkyl is substituted with one or more groups selected from the group consisting of halogen, hydroxy, amino, $C_{1-6}$ alkyl and $C_{1-6}$ alkoxy.

"Heterocyclyl" refers to a non-aromatic cyclic group containing 1 to 6 heteroatoms or 3 to 18 ring atoms, wherein the heteroatoms are selected from the group consisting of oxygen, nitrogen and sulfur. The heterocyclyl preferably

contains 1 to 4 heteroatoms, more preferably 1 to 3 heteroatoms, and even more preferably 1 or 2 heteroatoms; the heterocyclyl is preferably 3- to 12-membered, more preferably 3- to 8-membered or 4-to 8-membered, even more preferably 4- to 6-membered, and further more preferably 5-membered or 6-membered. Unless otherwise specifically indicated in the specification, heterocyclyl may be a monocyclic, bicyclic, tricyclic or tetracyclic system, and may include spiro or bridged ring systems; the nitrogen, carbon or sulfur atoms in heterocyclyl may optionally be oxidized; the nitrogen atoms may optionally be quaternized; and heterocyclyl may be partially or fully saturated. Apart from the moieties "-NH-C(=O)-" and "-NH-S(=O)$_2$-", other moieties on the cyclic structure of "heterocyclyl containing -NH-C(=O)- or -NH-S(=O)$_2$-" optionally contains a heteroatom.

The term "aryl" refers to a 6- to 14-membered, preferably 6- to 10-membered carbon monocyclic or fused polycyclic (in which the rings share a pair of adjacent carbon atoms) group having a conjugated $\pi$-electron system, such as phenyl and naphthyl. "Heteroaryl" refers to an aromatic cyclic group containing 1 to 4 heteroatoms or 5 to 14 ring atoms, wherein the heteroatoms are selected from the group consisting of oxygen, sulfur and nitrogen. The heteroaryl is preferably 4- to 6-membered or 6- to 12-membered, and more preferably 5-membered or 6-membered. For example. Non-limiting examples of heteroaryl include: imidazolyl, furyl, thienyl, thiazolyl, pyrazolyl, oxazolyl, pyrrolyl, tetrazolyl, pyridyl, pyrimidinyl, thiadiazole, pyrazine,

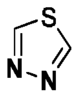 , 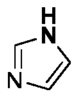 , 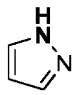

and the like.

The heteroaryl may be optionally substituted or unsubstituted, and when it is substituted, the substituent is preferably one or more of groups independently selected from the group consisting of alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, carboxyl and a carboxylate group.

The aryl or heteroaryl may be optionally substituted or unsubstituted, and when it is substituted, the substituent is preferably one or more groups independently selected from the group consisting of halogen, hydroxy, oxo, nitro, cyano, amino, $C_{1-6}$ alkyl, $C_{1-6}$ alkoxy, 3- to 6-membered cycloalkyl, and 3- to 6-membered heterocycloalkyl, wherein the alkyl, alkoxy, cycloalkyl or heterocycloalkyl is substituted with one or more groups selected from the group consisting of halogen, hydroxy, amino, $C_{1-6}$ alkyl and $C_{1-6}$ alkoxy.

"Cyclohydrocarbyl" or "cycloalkyl" refers to a stable non-aromatic monocyclic or polycyclic hydrocarbyl group consisting of carbon and hydrogen atoms only, which may comprise a spiro or bridged ring system, and contains 3 to 15 carbon atoms, 3 to 10 carbon atoms, 3 to 8 carbon atoms, 3 to 6 atoms or 5 to 7 carbon atoms; it is saturated or unsaturated, and is linked to the rest of the molecule by a single bond. The monocyclic cyclohydrocarbyl includes non-bridged cycloalkyl, e.g., cyclopropyl, cyclobutyl, cyclopentyl, cyclohexyl, cycloheptyl and cyclooctyl. The cycloalkyl may be substituted or unsubstituted, and when it is substituted, the substituent is preferably one or more groups independently selected from the group consisting of halogen, hydroxy, oxo, nitro, cyano, amino, $C_{1-6}$ alkyl, $C_{1-6}$ alkoxy, 3- to 6-membered cycloalkyl, and 3- to 6-membered heterocycloalkyl, wherein the alkyl, alkoxy, cycloalkyl or heterocycloalkyl is substituted with one or more groups selected from the group consisting of halogen, hydroxy, amino, $C_{1-6}$ alkyl and $C_{1-6}$ alkoxy. "Cyclohydrocarbylene" or "cycloalkylene" refers to a divalent cyclohydrocarbyl group derived from cyclohydrocarbyl, e.g.,

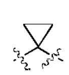 , 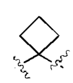 , 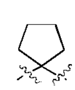 , 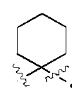,

and the like. The cycloalkylene may be substituted or unsubstituted, and when it is substituted, the substituent is preferably one or more groups independently selected from the group consisting of halogen, hydroxy, oxo, nitro, cyano, amino, $C_{1-6}$ alkyl, $C_{1-6}$ alkoxy, 3- to 6-membered cycloalkyl, and 3- to 6-membered heterocycloalkyl, wherein the alkyl, alkoxy, cycloalkyl or heterocycloalkyl is substituted with one or more groups selected from the group consisting of halogen, hydroxy, amino, $C_{1-6}$ alkyl and $C_{1-6}$ alkoxy.

"Optionally" or "optional" means that the event or circumstance subsequently described may, but not necessarily, occur, and that the description includes instances where the event or circumstance occurs or does not occur. For example, "$C_1$-$C_6$ alkyl optionally substituted with halogen or cyano" means that halogen or cyano may, but not necessarily, be present, and the description includes the instance where alkyl is substituted with halogen or cyano and the instance where alkyl is not substituted with halogen and cyano.

The term "hydroxy" refers to -OH group.

The term "halogen" refers to fluorine, chlorine, bromine or iodine.

The term "cyano" refers to -CN.
The term "amino" refers to $-NH_2$.
The term "nitro" refers to $-NO_2$.
The term "oxo" refers to =O substituent.
The term "carboxyl" refers to -C(O)OH.
"Substituted" means that one or more, preferably 1-5, more preferably 1-3 hydrogen atoms in the group are independently substituted with a corresponding number of substituents.
"Pharmaceutically acceptable salt" refers to salts of the compounds of the present disclosure, which are safe and effective for use in the body of a mammal and possess the requisite biological activities. The salts may be prepared separately during the final separation and purification of the compound, or by reacting an appropriate group with an appropriate base or acid. Bases commonly used to form pharmaceutically acceptable salts include inorganic bases, e.g., sodium hydroxide and potassium hydroxide, and organic bases, e.g., ammonia. Acids commonly used to form pharmaceutically acceptable salts include inorganic acids and organic acids.

## DETAILED DESCRIPTION

The structure of the compound is determined by nuclear magnetic resonance (NMR) spectroscopy and/or mass spectrometry (MS). NMR shift ($\delta$) is given in a unit of $10^{-6}$ (ppm). NMR spectra are determined using a Bruker AVANCE-400 nuclear magnetic resonance instrument, with deuterated dimethyl sulfoxide (DMSO-$d_6$), deuterated chloroform ($CDCl_3$) and deuterated methanol ($CD_3OD$) as determination solvents and tetramethylsilane (TMS) as an internal standard.
HPLC analysis is performed using a Waters ACQUITY ultra high performance LC, Shimadzu LC-20A systems, Shimadzu LC-2010HT series, or Agilent 1200 LC high performance liquid chromatograph (ACQUITY UPLC BEH C18 1.7 μm 2.1×50 mm column, Ultimate XB-C18 3.0×150 mm column, or Xtimate C18 2.1×30 mm column). MS analysis is performed using Waters SQD2 mass spectrometer in positive/negative ion mode with a mass scan range of 100-1200.
Chiral HPLC analysis is performed using Chiralpak IC-3 100×4.6 mm I.D., 3 μm, Chiralpak AD-3 150×4.6 mm I.D., 3 μm, Chiralpak AD-3 50×4.6 mm I.D., 3 μm, Chiralpak AS-3 150×4.6 mm I.D., 3 μm, Chiralpak AS-3 100×4.6 mm I.D., 3 μm, ChiralCel OD-3 150×4.6 mm I.D., 3 μm, ChiralCel OD-3 100×4.6 mm I.D., 3 μm, ChiralCel OJ-H 150×4.6 mm I.D., 5 μm, ChiralCel OJ-3 150×4.6 mm I.D., 3 μm chromatographic columns.
Huanghai HSGF254 or Qingdao GF254 silica gel plates of specifications 0.15 mm to 0.2 mm are adopted for thin layer chromatography (TLC) analysis and 0.4 mm to 0.5 mm for TLC separation and purification.
Yantai Huanghai silica gel of 100-200 mesh, 200-300 mesh or 300-400 mesh is generally used as a carrier in column chromatography.
Chiral HPLC preparation is performed using a DAICEL CHIRALPAK IC (250×30 mm, 10 μm) or Phenomenex-Amylose-1 (250×30 mm, 5 μm) column.
Known starting materials described herein may be synthesized using or according to methods known in the art, or may be purchased from ABCR GmbH & Co. KG, Acros Organics, Aldrich Chemical Company, Accela ChemBio Inc., Chembee Chemicals, and other companies.
In the examples, all reactions can be performed under argon atmosphere or nitrogen atmosphere unless otherwise specified.
The argon atmosphere or nitrogen atmosphere means that the reaction flask is connected to a balloon containing about 1 L of argon or nitrogen.
The hydrogen atmosphere means that the reaction flask is connected to a balloon containing about 1 L of hydrogen.
Parr 3916EKX hydrogenator, Qinglan QL-500 hydrogenator or HC2-SS hydrogenator is used in the pressurized hydrogenation reactions.
The hydrogenation reactions usually involve 3 cycles of vacuumization and hydrogen purge.
In the examples, a solution refers to an aqueous solution unless otherwise specified.
In the examples, the reaction temperature is room temperature, i.e., 20 °C to 30 °C, unless otherwise specified.
The monitoring of the reaction progress in the examples is conducted by thin layer chromatography (TLC). The developing solvent for reactions, the eluent system for column chromatography for purification of compounds, the developing solvent system for thin layer chromatography system and the volume ratio of the solvents are adjusted according to the polarity of the compound, or by adding a small amount of basic or acidic reagents such as triethylamine and acetic acid.
The positive compound **MK-7264** is prepared by referring to the experimental procedures in the patent WO2005095359.

Example **1**

1-[4-(1-{[(2*R*)-4-acetylmorpholin-2-yl]methyl} -5-methyl-1*H*-1,3-benzooxadiazol-2-yl)-3,5-difluorophenyl]pyrrolidin-2-one (1)

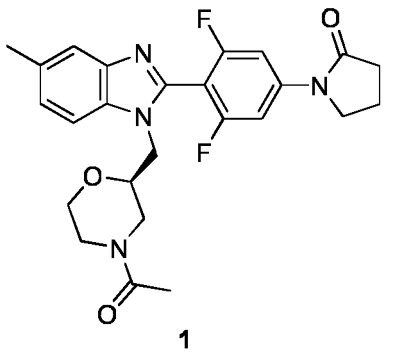

1

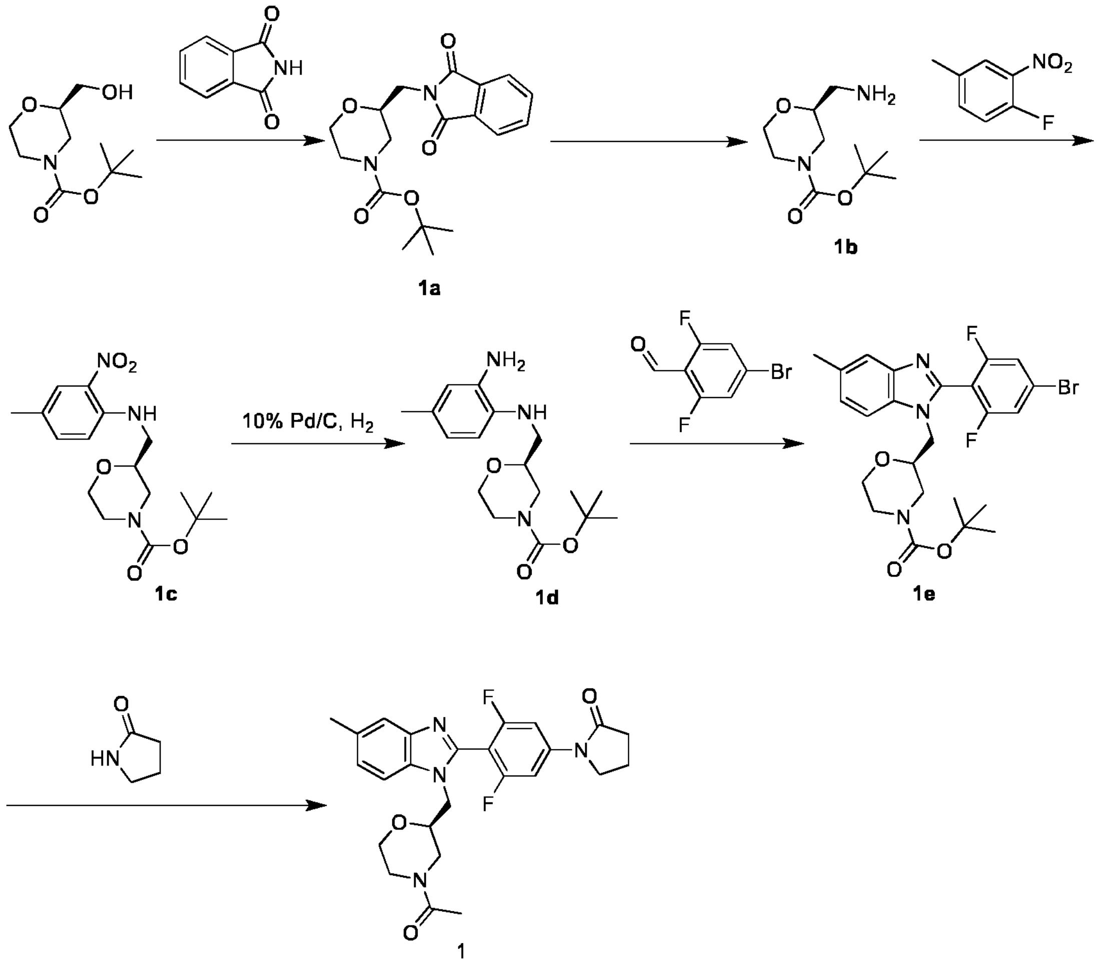


1a

1b

1c

1d

1e

1

Step 1: *tert*-butyl (2*S*-2-[(1,3-dicarbonyl-2,3-dihydro-1*H*-isoindol-2-yl)methyl]morpholine-4-carboxylate (**1a**)

*Tert*-butyl (2*R*)-2-(hydroxymethyl)morpholine-4-carboxylate (3.00 g, 13.81 mmol), 2,3-dihydro-1H isoindole-1,3-dione (2.23 g, 15.19 mmol) and triphenylphosphine (5.43 g, 20.71 mmol) were dissolved in THF (50 mL), diisopropyl azodicarboxylate (4.1 mL, 20.71 mmol) was added dropwise at 0 °C under nitrogen atmosphere, and the mixture was stirred at room temperature for 12 h. After LCMS showed that the starting material was reacted completely, the reaction

mixture was concentrated under reduced pressure. The residue was purified by flash silica gel chromatography (ethyl acetate/petroleum ether = 0-30%, 60 mL/min) to give the title compound **1a** (6.00 g, yield: 87.8%).

MS (ESI) m/z = 291.1 $[M+H]^+$.

$^1$H NMR (400 MHz, $CDCl_3$) $\delta$ 7.83 - 7.76 (m, 2H), 7.69 - 7.63 (m, 2H), 4.91 (spt, *J* = 6.2 Hz, 3H), 3.96 - 3.84 (m, 1H), 3.83 - 3.77 (m, 2H), 3.75 - 3.64 (m, 2H), 3.60 (dd, *J* = 4.6, 13.6 Hz, 1H), 3.37 (dt, *J* = 2.9, 11.4 Hz, 1H), 2.92 (br s, 1H), 2.69 (br s, 1H), 1.39 (s, 9H), 1.21 (s, 11H).

Step 2: *tert*-butyl (2*S*)-2-(aminomethyl)morpholine-4-carboxylate (**1b**)

To a solution of compound **1a** (6.00 g, 12.12 mmol) in ethanol (150 mL) was added hydrazine hydrate (1.07 g, 18.19 mmol) at room temperature, and the mixture was stirred at 80 °C for 1 h. After LCMS showed that the reaction was completed, the reaction mixture was cooled to room temperature and filtered, and the filtrate was concentrated under reduced pressure. The residue was dissolved in ethyl acetate (80 mL) and filtered, and the filtrate was concentrated under reduced pressure to give the title compound 1b (3.00 g, yield: 91.5%).

$^1$H NMR (400 MHz, $CDCl_3$) $\delta$ 6.27 (br s, 2H), 3.82 (br d, *J* = 10.9 Hz, 3H), 3.51 - 3.41 (m, 1H), 3.34 - 3.23 (m, 1H), 3.29 (br dd, *J* = 3.7, 6.7 Hz, 1H), 2.85 (br s, 1H), 2.73 - 2.63 (m, 2H), 2.57 (br s, 1H), 1.40 (s, 9H).

Step 3: tert-butyl (2*S*)-2-{[(4-methyl-2-nitrophenyl)amino]methyl}morpholine-4-carboxylate (**1c**)

To a solution of compound **1b** (3.00 g, 11.10 mmol) in 1,4-dioxane (60 mL) were added 1-fluoro-4-methyl-2-nitrobenzene (1.72 g, 11.10 mmol) and ethyl diisopropylamine (3.7 mL, 22.19 mmol) at room temperature, and the mixture was stirred at 100 °C for 12 h. After LCMS showed that the starting material was consumed completely, water (50 mL) was added, and the mixture was extracted with ethyl acetate (80 mL $\times$ 3). The combined organic phase was dried over anhydrous sodium sulfate, filtered and concentrated under reduced pressure to give a crude product, which was purified by flash silica gel chromatography (ethyl acetate/petroleum ether = 0-17%, 60 mL/min) to give the title compound **1c** (2.35 g, yield: 60%).

MS (ESI) m/z = 374.2 $[M+Na]^+$.

$^1$H NMR (400 MHz, $CDCl_3$) $\delta$ 8.11 (br s, 1H), 7.99 (d, *J* = 0.8 Hz, 1H), 7.30 - 7.27 (m, 1H), 6.77 (d, *J* = 8.8 Hz, 1H), 4.06 - 3.81 (m, 3H), 3.75 - 3.65 (m, 1H), 3.64 - 3.53 (m, 1H), 3.47 - 3.29 (m, 2H), 3.09 - 2.91 (m, 1H), 2.80 (br s, 1H), 2.28 (s, 3H), 1.47 (s, 9H).

Step 4: *tert*-butyl (2*S*)-2-{[(2-amino-4-methylphenyl)amino]methyl}morpholine-4-carboxylate (**1d**)

To a solution of compound **1c** (2.00 g, 5.69 mmol) in methanol (20 mL) was added 10% Pd/C (100 mg) at room temperature, and the resulting mixture was purged 3 times with hydrogen and stirred at room temperature for 12 h. After LCMS showed that the starting material was consumed completely, the reaction mixture was filtered, and the filtrate was concentrated in vacuum to give the title compound **1d** (1.73 g, yield: 94.4%).

MS (ESI) m/z = 322.2 $[M+H]^+$.

$^1$H NMR (400 MHz, $CDCl_3$): $\delta$ 6.65 - 6.50 (m, 3H), 3.92 (br d, *J* = 10.0 Hz, 3H), 3.70 - 3.62 (m, 1H), 3.60 - 3.51 (m, 1H), 3.44 (br s, 2H), 3.32 - 3.00 (m, 3H), 2.97 (br s, 1H), 2.79 (br s, 1H), 2.23 (s, 3H), 1.48 (s, 9H).

Step 5: tert-butyl (2*S*)-2-{[2-(4-bromo-2,6-difluorophenyl)-5-methyl-1*H*-1,3-benzooxadiazol-1-yl]methyl }morpholine-4-carboxylate (**1e**)

To a solution of compound **1d** (810.0 mg, 2.52 mmol) in *n*-butanol (14 mL) were added 4-bromo-2,6-difluorobenzaldehyde (612.6 mg, 2.77 mmol) and acetic acid (144 μL, 2.52 mmol) at room temperature, and the resulting mixture was purged 3 times with nitrogen and stirred at 90 °C for 14 h. After LCMS showed that the starting material was reacted completely, the reaction solution was concentrated in vacuum to give a crude product, which was purified by flash silica gel chromatography (ethyl acetate/petroleum ether = 0-17%) to give the title compound 1e (189.8 mg, yield: 14.4%).

MS (ESI) m/z = 524.2 $[M+H]^+$.

$^1$H NMR (400 MHz, $CDCl_3$) $\delta$ 7.63 (br s, 1H), 7.37 (br d, *J* = 7.6 Hz, 1H), 7.26 (br d, *J* = 6.0 Hz, 2H), 7.19 (br d, *J* = 7.2 Hz, 1H), 4.10 (br s, 2H), 3.88 - 3.53 (m, 4H), 3.30 (br s, 1H), 2.77 (br s, 1H), 2.51 (br s, 3H), 2.48 - 2.38 (m, 1H), 1.42 (br s, 9H).

Step 6: 1-[4-(1-{[(2*R*)-4-acetylmorpholin-2-yl]methyl} -5-methyl-1*H*-1,3-benzooxadiazol-2-yl)-3,5-difluorophenyl]pyrrolidin-2-one (**1**)

To a solution of compound **1e** (90.0 mg, 0.172 mmol) and pyrrolidin-2-one (29.3 mg, 0.345 mmol) in 1,4-dioxane (3 mL) were added $Cs_2CO_3$ (112.3 mg, 0.345 mmol), $Pd(OAc)_2$ (5.8 mg, 0.026 mmol) and Xantphos (20.0 mg, 0.034 mmol) at room temperature, and the resulting mixture was purged with nitrogen for 5 min and stirred under microwave irradiation at 100 °C for 35 min. After LCMS showed that the reaction was completed, water (5 mL) was added to quench the reaction, and the mixture was extracted with ethyl acetate (10 mL × 3). The organic phases were combined, dried over anhydrous sodium sulfate and filtered. The filtrate was concentrated under reduced pressure to give a crude product, which was dissolved in dichloromethane (2 mL). Trifluoroacetic acid (400 μL) was added in an ice-water bath, and the mixture was stirred at room temperature for 1 h. After LCMS showed that the reaction was completed, the reaction mixture was concentrated under reduced pressure, and the resulting residue was dissolved in methanol (2 mL). Triethylamine (238 μL, 1.71 mmol) was added in an ice-water bath to adjust pH to 8, followed by the addition of acetic anhydride (80 μL, 0.854 mmol), and the mixture was reacted at room temperature for 1 h. After LCMS showed that the reaction was completed, the reaction mixture was concentrated. The residue was purified by C18 reverse phase chromatography (acetonitrile/water (containing 0.05% ammonia water) = 10%-75%) and lyophilized to give the title compound 1 (52.0 mg, yield: 64%).

MS (ESI) m/z = 469.2 $[M+H]^+$.

$^1H$ NMR (400 MHz, DMSO-$d_6$) $\delta$ 8.43 (br d, *J* = 7.0 Hz, 1H), 7.63 (dd, *J* = 3.3, 10.3 Hz, 2H), 7.37 (s, 1H), 6.93 - 6.73 (m, 1H), 4.09 (br dd, *J* = 9.2, 18.9 Hz, 2H), 3.99 - 3.85 (m, 3H), 3.77 - 3.52 (m, 2H), 3.51 - 3.41 (m, 2H), 3.09 - 2.99 (m, 2H), 2.90 - 2.82 (m, 1H), 2.73 - 2.65 (m, 1H), 2.63 - 2.58 (m, 2H), 2.41 (s, 3H), 2.18 - 2.03 (m, 2H).

Example **2**

Methyl 2-((2-(2,6-difluoro-4-(2-carbonylpyrrolidin-1-yl)phenyl)-5-methyl-1*H*-benzo[*d*]imidazo 1-1-yl)methyl)morpholine-4-carboxylate (**2**)

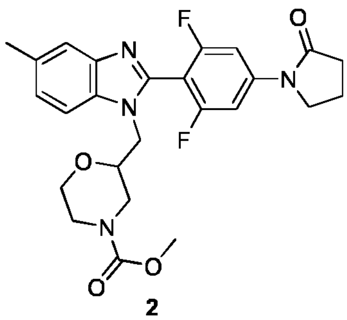

**2**

Example **2** was synthesized by referring to the synthetic procedures for Example 1, with tert-butyl (2*R*)-2-(hydroxymethyl)morpholine-4-carboxylate replaced by tert-butyl 2-(hydroxymethyl)morpholine-4-carboxylate in step 1, and acetic anhydride replaced by methyl chloroformate in step 6.

MS (ESI) m/z = 486.4 $[M+H]^+$.

Example **3**

(*S*)-1-(4-(1-((4-acetylmorpholin-2-yl)methyl)-5-methyl-1*H*-benzo[d]imidazol-2-yl)-3-ch lorophenyl)pyrrolidin-2-one (**3**)

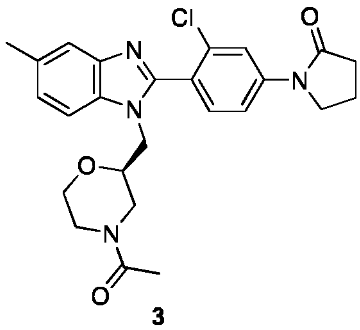

3

Example **3** was synthesized by referring to the synthetic procedures for Example **1,** with 4-bromo-2,6-difluorobenzaldehyde replaced by 4-bromo-2-chlorobenzaldehyde in step 5.

MS (ESI) m/z = 467.2 $[M+H]^+$.

Example **4**

(*S*)-1-(4-(1-((4-acetylmorpholin-2-yl)methyl)-5-methyl-1*H*-benzo[d]imidazol-2-yl)-3-ch loro-5-fluorophenyl)pyrrolidin-2-one (**4**)

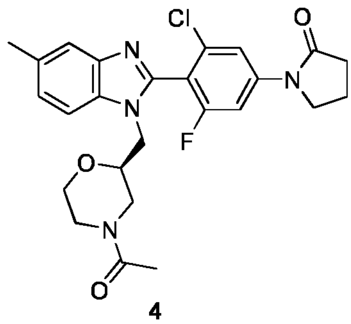

4

Example **4** was synthesized by referring to the synthetic procedures for Example **1,** with 4-bromo-2,6-difluorobenzaldehyde replaced by 4-bromo-2-chloro-6-fluorobenzaldehyde in step 5.

MS (ESI) m/z = 485.5 $[M+H]^+$.

$^1H$ NMR (400 MHz, DMSO-$d_6$) $\delta$ 7.88 (br s, 1H), 7.79 (br d, *J* = 11.6 Hz, 1H), 7.70 - 7.60 (m, 1H), 7.49 (br s, 1H), 7.20 - 7.12 (m, 1H), 4.33 - 4.16 (m, 2H), 4.03 (br d, *J* = 15.0 Hz, 1H), 3.97 - 3.87 (m, 3H), 3.78 (br d, *J* = 17.6 Hz, 1H), 3.68 - 3.55 (m, 1H), 3.12 (br d, *J* = 11.0 Hz, 1H), 2.99 (br d, *J* = 11.6 Hz, 1H), 2.81 - 2.70 (m, 2H), 2.58 (br d, *J* = 7.9 Hz, 2H), 2.44 (br s, 3H), 2.14 - 2.06 (m, 2H), 1.93 (s, 2H).

Example **5**

(*S*)-1-(4-(1-((4-acetylmorpholin-2-yl)methyl)-5-(difluoromethyl)-1*H*-benzo[*d*]imidazol-2-yl)-3,5-difluorophenyl)pyrrolidin-2-one (5)

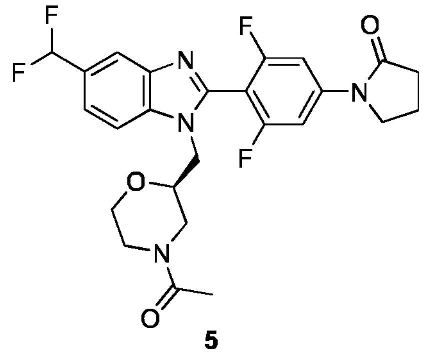

**5**

Example **5** was synthesized by referring to the synthetic procedures for Example **1,** with 1-fluoro-4-methyl-2-nitrobenzene replaced by 4-(difluoromethyl)-1-fluoro-2-nitrobenzene in step 3.

MS (ESI) m/z = 505.5 $[M+H]^+$.

Example **6**

Methyl (*S*)-2-((2-(2,6-difluoro-4-(methylcarbamoyl)phenyl)-6-methyl-3*H*-imidazo[4,5-*b*]pyridi n-3-yl)methyl)morpholine-4-carboxylate (**6**)

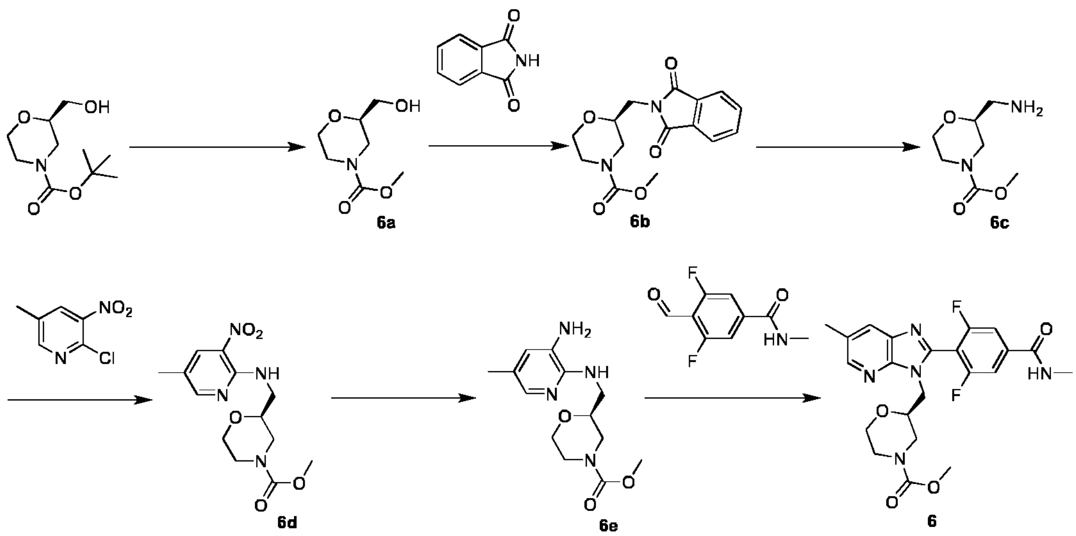

**6a** **6b** **6c** **6d** **6e** **6**

Step 1: methyl (*R*)-2-(hydroxymethyl)morpholine-4-carboxylate (**6a**)

To a solution of *tert*-butyl (2*R*)-2-(hydroxymethyl)morpholine-4-carboxylate (10.00 g, 46.03 mmol) in methanol (50 mL) was added dropwise a solution of 4 N HCl in methanol (57.5 mL, 230.13 mmol) at room temperature, and the mixture was stirred at room temperature for 4 h. After TLC (petroleum ether/ethyl acetate = 3/1, sample was treated with a saturated $NaHCO_3$ solution) showed that the starting material was reacted completely, the reaction mixture was concentrated under reduced pressure. The resulting residue was dissolved in dichloromethane (50 mL), and ethyl diisopropylamine (38.0 mL, 230.13 mmol) and methyl chloroformate (4.3 mL, 55.23 mmol) were added dropwise slowly and successively in an ice-water bath. The mixture was stirred at room temperature for 2 h. After TLC (petroleum ether/ethyl acetate = 1/2) showed that the starting material was reacted completely, water (100 mL) was added to quench the reaction, and the mixture was extracted with ethyl acetate (50 mL × 5). The combined organic phase was dried over anhydrous sodium sulfate, filtered and concentrated under reduced pressure to give the title compound **6a** (9.45 g, yield: 94%).

$^1H$ NMR (400 MHz, $CDCl_3$) $\delta$ 4.02 - 3.85 (m, 3H), 3.73 (s, 3H), 3.69 - 3.66 (m, 1H), 3.63 - 3.53 (m, 3H), 3.05 - 2.92 (m, 1H), 2.90 - 2.72 (m, 1H).

Step 2: methyl (*S*)-2-((1,3-dicarbonylisoindolin-2-yl)methyl)morpholine-4-carboxylate (**6b**)

Compound **6b** was synthesized by referring to step 1 in Example **1,** with *tert*-butyl (2R)-2-(hydroxymethyl)morpholine-4-carboxylater replaced by compound **6a.**

MS (ESI) m/z = 305.0 $[M+H]^+$.

$^1H$ NMR (400 MHz, $CDCl_3$) $\delta$ 7.91-7.84 (m, 2H), 7.77-7.71 (m, 2H), 3.99 (br s, 1H), 3.93-3.87 (m, 2H), 3.82-3.80 (m, 1H), 3.77 (br s, 1H), 3.71 (s, 3H), 3.70-3.64 (m, 1H), 3.47 (dt, J = 2.4, 11.2 Hz, 1H), 3.04 (br s, 1H), 2.83 (br t, J = 11.6 Hz, 1H).

Step 3: methyl (S)-2-(aminomethyl)morpholine-4-carboxylate (**6c**)

Compound **6c** was synthesized by referring to step 2 in Example **1,** with compound **1a** replaced by compound **6b.**

$^1H$ NMR (400 MHz, $CDCl_3$) $\delta$ 3.91 (br d, *J* = 9.2 Hz, 3H), 3.72 (s, 3H), 3.61-3.48 (m, 1H), 3.37 (br d, *J* = 2.4 Hz, 1H), 2.99 (br s, 1H), 2.76 (br d, *J* = 6.4 Hz, 1H), 2.74-2.63 (m, 1H).

Step 4: methyl (*S*)-2-(((5-methyl-3-nitropyridin-2-yl)amino)methyl)morpholine-4-carboxylate (**6d**)

To a solution of 2-chloro-5-methyl-3-nitropyridine (200.0 mg, 1.16 mmol) and compound **6c** (356.2 mg, 1.39 mmol) in dimethyl sulfoxide (2 mL) was added ethyl diisopropylamine (0.57 mL, 3.48 mmol) at room temperature, and the mixture was stirred at 60 °C for 12 h. After LCMS showed that the starting material was consumed completely, the reaction mixture was diluted with ethyl acetate (30 mL) and filtered, and the filtrate was concentrated under reduced pressure to give a crude product, which was purified by flash silica gel chromatography (ethyl acetate/petroleum ether = 0-50%) to give the title compound **6d** (237.2 mg, yield: 66%).

MS (ESI) m/z = 310.9 $[M+H]^+$.

$^1H$ NMR (400 MHz, $CDCl_3$) $\delta$ 8.42-8.19 (m, 3H), 4.09-3.83 (m, 3H), 3.73 (s, 3H), 3.70-3.51 (m, 3H), 3.11-2.93 (m, 1H), 2.89-2.69 (m, 1H), 2.28 (s, 3H).

Step 5: methyl (*S*)-2-(((3-amino-5-methylpyridin-2-yl)amino)methyl)morpholine-4-carboxylate (**6e**)

Compound **6e** was synthesized by referring to step 4 in Example **1,** with compound **1c** replaced by compound **6d.**

$^1H$ NMR (400 MHz, $CDCl_3$) $\delta$ 7.55 (s, 1H), 6.71 (s, 1H), 4.31 (br s, 1H), 4.07-3.82 (m, *J* = 12.8 Hz, 3H), 3.72 (s, 3H), 3.71-3.63 (m, 2H), 3.61-3.51 (m, 1H), 3.44-3.31 (m, 1H), 3.21 (br s, 2H), 3.06-2.96 (m, 1H), 2.86-2.75 (m, 1H), 2.16 (s, 3H).

Step 6: methyl (*S*)-2-((2-(2,6-difluoro-4-(methylcarbamoyl)phenyl)-6-methyl-3*H*-imidazo[4,5-*b*]pyridi n-3-yl)methyl)morpholine-4-carboxylate (**6**)

Compound **6** synthesized by referring to step 5 in Example **1,** with compound **1d** replaced by compound **6e,** and 4-bromo-2,6-difluorobenzaldehyde replaced by 3,5-difluoro-4-formyl-*N*-methyl benzamide.

MS (ESI) m/z = 460.1 $[M+H]^+$.

$^1H$ NMR (400 MHz, $CDCl_3$) $\delta$ 8.32 (s, 1H), 7.92 (s, 1H), 7.53 (br s, 1H), 7.45 (br d, *J* = 8.8 Hz, 2H), 4.36 (dd, *J* = 3.2, 14.4 Hz, 1H), 4.16 (br dd, *J* = 7.6, 14.4 Hz, 1H), 3.98 (br s, 1H), 3.83-3.69 (m, 1H), 3.66 (s, 3H), 3.65-3.61 (m, 1H), 3.57 (br d, *J* = 10.8 Hz, 1H), 3.23 (br t, *J* = 10.8 Hz, 1H), 3.03 (d, *J* = 4.4 Hz, 3H), 2.76 (br s, 1H), 2.53 (s, 3H), 2.47 (br s, 1H).

Example 7

Methyl (*S*)-2-((2-(2,6-difluoro-4-(methylcarbamoyl)phenyl)-5-methyl-1*H*-imidazo[4,5-*b*]pyridi n-1-yl)methyl)morpholine-4-carboxylate (**7**)

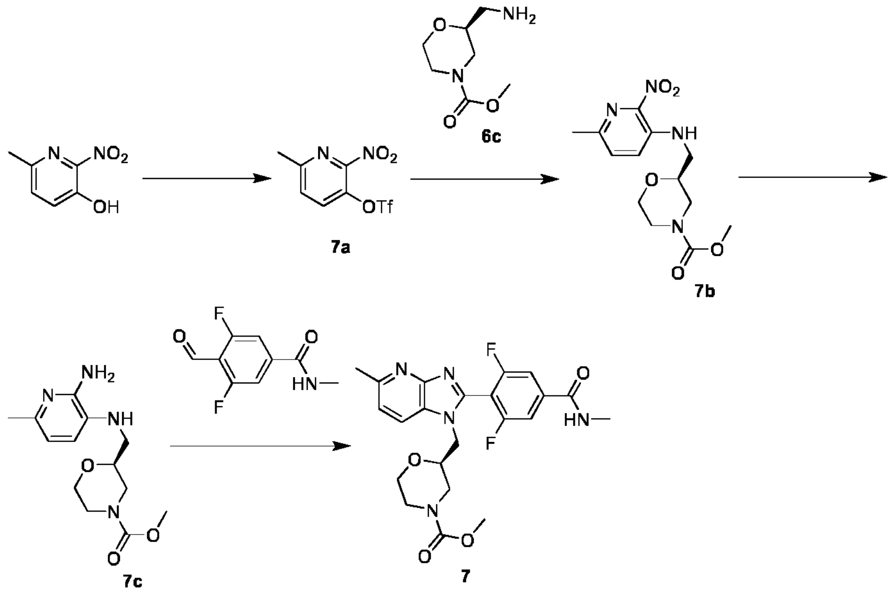
6c 7a 7b 7c 7

Step 1: 6-methyl-2-nitropyridin-3-yl trifluoromethanesulfonate (**7a**)

To a solution of 6-methyl-2-nitropyridin-3-ol (1.00 g, 6.50 mmol) in dichloromethane (10 mL) were added triethylamine (1.4 mL, 9.70 mmol) and trifluoromethanesulfonic anhydride (1.3 mL, 7.80 mmol) at 0 °C under nitrogen atmosphere, and the resulting solution was stirred at 0 °C for 2 h. Water (80 mL) was added to quench the reaction, and the mixture was extracted with ethyl acetate (50 mL × 3). The combined organic phase was washed with brine (80 mL × 3), dried over anhydrous sodium sulfate, filtered and concentrated under reduced pressure to give the title compound **7a** (1.70 g, yield: 92%).
$^1$H NMR (400 MHz, $CDCl_3$) $\delta$ 7.82 (d, *J* = 8.4 Hz, 1H), 7.60 (d, *J* = 8.4 Hz, 1H), 2.70 (s, 3H).

Step 2: methyl (*S*)-2-(((6-methyl-2-nitropyridin-3-yl)amino)methyl)morpholine-4-carboxylate (**7b**)

To a solution of compound **7a** (1.20 g, 4.2 mmol) and compound **6c** (870 mg, 3.5 mmol) in acetonitrile (15 mL) was added dropwise triethylamine (1.10 g, 10.5 mmol) at room temperature, and the resulting solution was stirred at 80 °C for 12 h. After LCMS showed that the starting material was consumed completely, the reaction solution was concentrated under reduced pressure, and the resulting crude product was purified by C18 reverse phase column chromatography (methanol/water solution (0.1% TFA) = 50%) and lyophilized to give the title compound **7b** (275.0 mg, yield: 25%).
MS (ESI) m/z = 310.9 [M+H]$^+$.
$^1$H NMR (400 MHz, $CDCl_3$) $\delta$ 7.92-7.80 (m, 1H), 7.35-7.29 (m, 2H), 3.74 (s, 3H), 3.63-3.52 (m, 3H), 3.51-3.40 (m, 2H), 3.39-3.32 (m, 1H), 3.25 (dd, *J* = 7.6, 13.2 Hz, 1H), 3.11-2.93 (m, 2H), 2.52 (s, 3H).

Step 3: methyl (*S*)-2-(((2-amino-6-methylpyridin-3-yl)amino)methyl)morpholine-4-carboxylate (**7c**)

Compound **7c** was synthesized by referring to step 4 in Example **1,** with compound **1c** replaced by compound **7b.**
MS (ESI) m/z = 280.9 [M+H]$^+$.
$^1$H NMR (400 MHz, $CDCl_3$) $\delta$ 6.75 (d, *J* = 8.0 Hz, 1H), 6.53 (d, *J* = 7.6 Hz, 1H), 3.74 (s, 3H), 3.61-3.53 (m, 3H), 3.46 (dd, *J* = 3.6, 13.6 Hz, 1H), 3.25 (dd, *J* = 7.6, 13.6 Hz, 1H), 3.11-3.02 (m, 2H), 2.91-2.69 (m, 2H), 2.34 (s, 3H).

Step 4: methyl (*S*)-2-((2-(2,6-difluoro-4-(methylcarbamoyl)phenyl)-5-methyl-1*H*-imidazo[4,5-*b*]pyridi n-1-yl)methyl)morpholine-4-carboxylate (**7**)

Compound **7** was synthesized by referring to step 5 in Example **1,** with compound **1d** replaced by compound **7c,** and 4-bromo-2,6-difluorobenzaldehyde replaced by 3,5-difluoro-4-formyl-*N*-methyl benzamide.
MS (ESI) m/z = 460.1 [M+H]$^+$.
$^1$H NMR (400 MHz, $CDCl_3$) $\delta$ 8.91 (br s, 1H), 7.78 (d, *J* = 6.4 Hz, 1H), 7.52 (d, *J* = 8.4 Hz, 2H), 7.22 (d, *J* = 8.4 Hz, 1H), 4.17-3.98 (m, 2H), 3.95-3.59 (m, 6H), 3.59-3.46 (m, 1H), 3.36-3.18 (m, 1H), 3.05 (d, *J* = 4.8 Hz, 3H), 2.86-2.77 (m, 1H), 2.75 (s, 3H), 2.56-2.42 (m, 1H).

Example **8**

Methyl (*S*)-2-((2-(2,6-difluoro-4-(methylcarbamoyl)phenyl)-6-methyl-3*H*-imidazo[4,5-*c*]pyridi n-3-yl)methyl)morpholine-4-carboxylate (**8**)

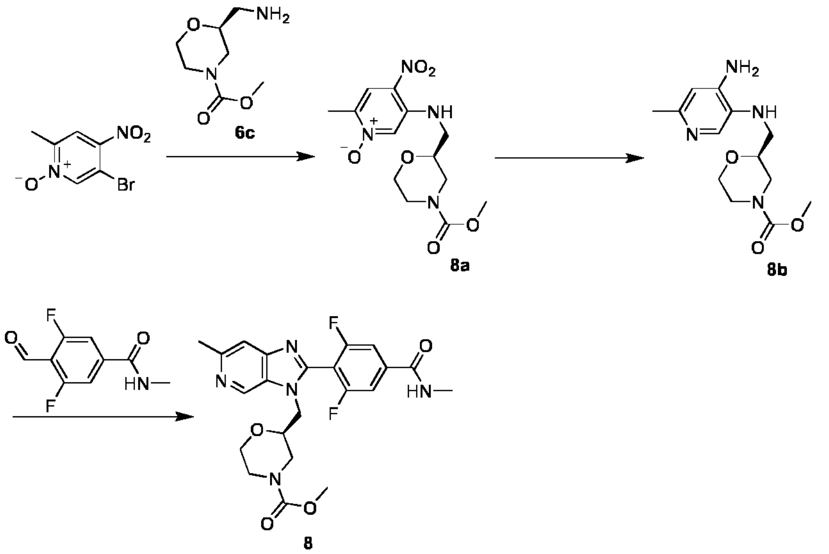

**6c** **8a** **8b** **8**

Step 1: (*S*)-5-(((4-(methoxycarbonyl)morpholin-2-yl)methyl)amino)-2-methyl-4-nitropyridine 1-oxide (**8a**)

5-bromo-2-methyl-4-nitropyridine 1-oxide (200.0 mg, 0.86 mmol) and compound **6c** (329.0 mg, 1.89 mmol) were dissolved in tetrahydrofuran (5 mL) at room temperature, and the resulting solution was stirred at 80 °C for 12 h. After LCMS showed that the starting material was consumed completely, the reaction solution was concentrated under reduced pressure, and methanol (2 mL) was added to the resulting crude product. The mixture was filtered, and the solid was dried to give the title compound **8a** (50.0 mg, yield: 18%).

MS (ESI) m/z = 327.0 $[M+H]^+$.

$^1H$ NMR (400 MHz, $CDCl_3$) $\delta$ 8.06 (s, 1H), 8.01 (s, 1H), 7.90-7.80 (m, 1H), 4.23-3.81 (m, 4H), 3.74 (s, 3H), 3.61-3.55 (m, 1H), 3.42-3.35 (m, 1H), 3.32-3.24 (m, 1H), 3.14-3.00 (m, 2H), 2.41 (s, 3H).

Step 2: methyl (*S*)-2-(((4-amino-6-methylpyridin-3-yl)amino)methyl)morpholine-4-carboxylate (**8b**)

Compound **8b** was synthesized by referring to step 4 in Example 1, with compound **1c** replaced by compound **8a.**

MS (ESI) m/z = 281.2 $[M+H]^+$.

$^1H$ NMR (400 MHz, $CDCl_3$) $\delta$ 7.76 (s, 1H), 6.46 (s, 1H), 4.03-3.81 (m, 4H), 3.73 (s, 3H), 3.69-3.53 (m, 3H), 3.16-3.07 (m, 2H), 2.40 (s, 3H).

Step 3: methyl (*S*)-2-((2-(2,6-difluoro-4-(methylcarbamoyl)phenyl)-6-methyl-3*H*-imidazo[4,5-*c*]pyridi n-3-yl)methyl)morpholine-4-carboxylate (**8**)

Compound **8** was synthesized by referring to step 5 in Example 1, with compound **1d** replaced by compound **8b**, and 4-bromo-2,6-difluorobenzaldehyde replaced by 3,5-difluoro-4-formyl-*N*-methyl benzamide.

MS (ESI) m/z = 460.2 $[M+H]^+$.

$^1H$ NMR (400 MHz, $CDCl_3$) $\delta$ 8.87 (s, 1H), 7.58 (s, 1H), 7.49 (d, *J* = 8.4 Hz, 2H), 6.92-6.75 (m, 1H), 4.25-4.09 (m, 2H), 3.98-3.71 (m, 3H), 3.69 (s, 3H), 3.65-3.55 (m, 1H), 3.36-3.25 (m, 1H), 3.06 (d, *J* = 4.8 Hz, 3H), 2.90-2.77 (m, 1H), 2.72 (s, 3H), 2.60-2.46 (m, 1H).

Example **9**

Methyl (*S*)-2-((2-(2,6-difluoro-4-(2-carbonylpyrrolidin-1-yl)phenyl)-7-methylimidazo[1,2-a]pyr idin-3-yl)methyl)morpholine-4-carboxylate (**9**)

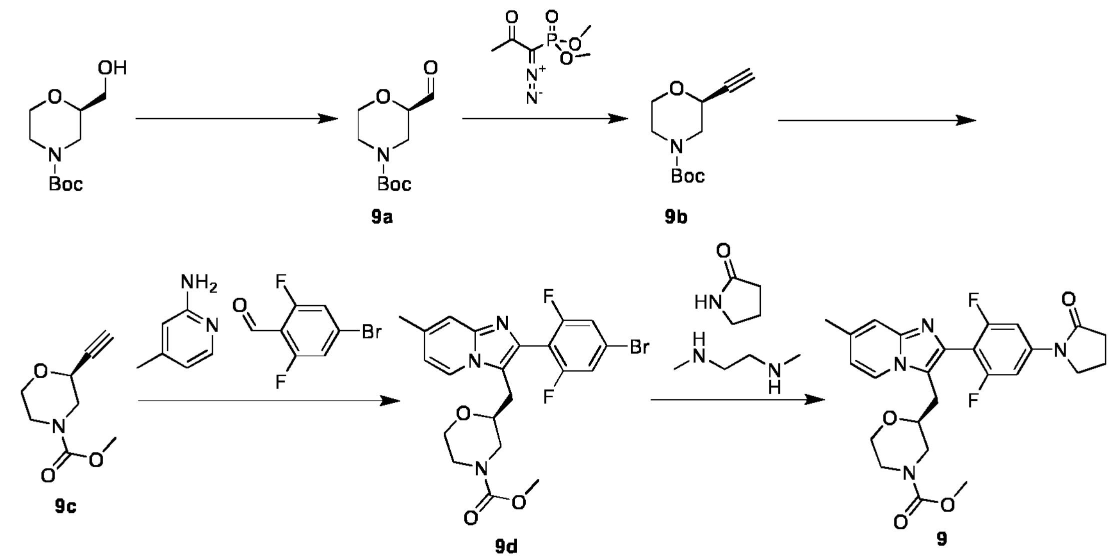

**9a** **9b** **9c** **9d** **9**

Step 1: tert-butyl (2R)-2-formylmorpholine-4-carboxylate (**9a**)

To a solution of oxalyl chloride (9.9 mL, 115.07 mmol) in anhydrous dichloromethane (200 mL) was added dropwise dimethyl sulfoxide (9.8 mL, 138.08 mmol) at -70 °C under nitrogen atmosphere. The mixture was stirred at -70 °C for 30 min. A solution of tert-butyl (2*R*)-2-(hydroxymethyl)morpholine-4-carboxylate (10.00 g, 46.03 mmol) in dichloromethane (30 mL) was added dropwise, and the mixture was stirred at -70 °C for 2 h. Triethylamine (32.0 mL, 230.13 mmol) was added dropwise at -70 °C, and the mixture was stirred at -70 °C for 30 min. After TLC (petroleum ether/ethyl acetate = 1/1) showed that the reaction was completed, the mixture was warmed to room temperature and washed with a saturated $NaHCO_3$ solution (80 mL $\times$ 2) and brine (80 mL). The organic phase was dried over anhydrous sodium sulfate, filtered and concentrated under reduced pressure to give the title compound **9a** (9.90 g, yield: 99.9%).

$^1H$ NMR (400 MHz, $CDCl_3$) $\delta$ 9.67 (s, 1H), 4.15-4.04 (m, 1H), 3.95-3.82 (m, 2H), 3.70-3.53 (m, 2H), 3.13-2.97 (m, 2H), 1.50 (s, 9H).

Step 2: tert-butyl (2R)-2-formylmorpholine-4-carboxylate (**9b**)

To a solution of compound **9a** (9.90 g, 45.99 mmol) in methanol (200 mL) were added potassium carbonate (25.43 g, 183.97 mmol) and dimethyl (1-diazo-2-oxopropyl) phosphonate (17.67 g, 91.99 mmol) successively at 0 °C under nitrogen atmosphere. The mixture was stirred at room temperature for 12 h. After TLC (petroleum ether/ethyl acetate = 3/1) showed that the reaction was completed, water (80 mL) was added to the reaction mixture, and the resulting mixture was concentrated under reduced pressure to remove methanol. The residue was extracted with ethyl acetate (100 mL $\times$ 3). The combined organic phase was dried over anhydrous sodium sulfate, filtered and concentrated under reduced pressure. The residue was purified by flash silica gel chromatography (ethyl acetate/petroleum ether = 0-25%) to give compound **9b** (6.4 g, yield: 65.9%).

$^1H$ NMR (400 MHz, $CDCl_3$) $\delta$ 4.29-4.25 (m, 1H), 3.99-3.96 (m, 1H), 3.93-3.63 (m, 1H), 3.64-3.55 (m, 2H), 3.33-3.27 (m, 2H), 2.50 (d, *J* = 2.4 Hz, 1H), 1.49 (s, 9H).

Step 3: methyl (*S*)-2-ethynylmorpholine-4-carboxylate (**9c**)

To a solution of compound **9b** (6.4 g, 30.29 mmol) in dichloromethane (60 mL) was added dropwise a solution of 4 N HCl in methanol (60 mL, 240.00 mmol) at room temperature, and the mixture was stirred at room temperature for 12 h. After TLC (petroleum ether/ethyl acetate = 3/1) showed that the starting material was reacted completely, the reaction mixture was concentrated under reduced pressure. The resulting residue was dissolved in dichloromethane

(80 mL), and ethyl diisopropylamine (14.8 mL, 89.43 mmol) and methyl chloroformate (2.8 mL, 35.98 mmol) were added dropwise slowly and successively in an ice-water bath. The mixture was stirred at room temperature for 12 h. After TLC (petroleum ether/ethyl acetate = 1/2) showed that the starting material was reacted completely, the reaction mixture was concentrated under reduced pressure. The resulting residue was purified by flash silica gel chromatography (ethyl acetate/petroleum ether = 0-25%) to give the title compound **9c** (5.00 g, yield: 97.6%).

$^1$H NMR (400 MHz, $CDCl_3$) $\delta$ 4.31-4.28 (m, 1H), 4.01-3.97 (m, 1H), 3.90-3.82 (m, 1H), 3.75 (s, 3H), 3.68-3.56 (m, 2H), 3.74-3.34 (m, 2H), 2.52 (d, *J* = 2.0 Hz, 1H).

Step 4: methyl (*S*)-2-((2-(4-bromo-2,6-difluorophenyl)-7-methylimidazo[1,2-*a*]pyridin-3-yl)methyl)mo rpholine-4-carboxylate (**9d**)

Compound **9c** (7.5 g, 44.33 mmol), 4-bromo-2,6-difluorobenzaldehyde (9.80 g, 44.33 mmol) and 4-methylpyridin-2-amine (4.79 g, 44.33 mmol) were dissolved in toluene (75 mL), and cuprous chloride (1.32 g, 13.30 mmol) and copper(II) trifluoromethanesulphonate (4.81 g, 13.30 mmol) were added under nitrogen atmosphere. The mixture was stirred at 85 °C for 5 min, and *N,N*-dimethylacetamide (1.2 mL, 13.30 mmol) was added. The mixture was stirred at 85 °C for 12 h. After TLC showed that the starting material was consumed completely, water (50 mL) and ammonia water (30 mL) were added. The reaction mixture was extracted with dichloromethane (100 mL $\times$ 3). The combined organic phase was dried over anhydrous sodium sulfate and filtered, and the filtrate was concentrated under reduced pressure to give a crude product, which was purified by silica gel column chromatography (ethyl acetate/petroleum ether = 0-85%) to give compound **9d** (6.00 g, yield: 26.8%).

MS (ESI) m/z = 480.1 $[M+H]^+$.

$^1$H NMR (400 MHz, $CDCl_3$) $\delta$ 8.17 (d, *J* = 6.8 Hz, 1H), 7.38 (s, 1H), 7.25-7.18 (m, 2H), 6.67 (br d, *J* = 6.8 Hz, 1H), 4.01-3.74 (m, 3H), 3.68 (s, 3H), 3.56 (br s, 1H), 3.44-3.34 (m, 1H), 3.06-2.85 (m, 3H), 2.60 (dd, *J* = 11.2, 12.8 Hz, 1H), 2.42 (s, 3H).

Step 5: methyl ((*S*)-2-((2-(2,6-difluoro-4-(2-carbonylpyrrolidin-1-yl)phenyl)-7-methylimidazo[1,2-*a*]pyr idin-3 -yl)methyl)morpholine-4-carboxylate (**9**)

To a solution of compound **9d** (48.0 mg, 0.10 mmol) in 1,4-dioxane (2 mL) were added 2-pyrrolidone (34.0 mg, 0.40 mmol), *N,N'*-dimethylethylenediamine (6 μL, 0.05 mmol), cesium carbonate (195.5 mg, 0.60 mmol), and cuprous iodide (9.5 mg, 0.05 mmol) successively in a microwave tube. The mixture was heated to 150 °C under microwave irradiation for 1 h. After LCMS showed that the reaction was completed, the reaction mixture was concentrated under reduced pressure. The residue was dissolved in acetonitrile and filtered, and the filtrate was directly purified by C18 reverse phase chromatography (acetonitrile/water (containing 0.05% $NH_3 \cdot H_2O$) = 5%-95%, flow rate: 60 mL/min) and lyophilized to give the title compound **9** (26.0 mg, yield: 53.7%).

MS (ESI) m/z = 485.3 $[M+H]^+$.

$^1$H NMR (400 MHz, DMSO-$d_6$) $\delta$ 8.40 (d, *J*= 7.3 Hz, 1H), 7.60 (d, *J* = 10.0 Hz, 2H), 7.34 (s, 1H), 6.82 (dd, *J* = 1.4, 7.2 Hz, 1H), 3.88 (t, *J* = 7.2 Hz, 2H), 3.75-3.60 (m, 3H), 3.55 (s, 3H), 3.46 (br d, *J* = 7.3 Hz, 1H), 3.28-3.19 (m, 1H), 3.01 (br d, *J* = 6.0 Hz, 2H), 2.80 (br s, 1H), 2.57 (t, *J* = 8.0 Hz, 3H), 2.37 (s, 3H), 2.08 (quin, *J* = 7.6 Hz, 2H).

Example 10

(*S*)-1-(4-(1-((4-acetylmorpholin-2-yl)methyl)-5-chloro-1*H*-benzo[*d*]imidazol-2-yl)-3,5-difluorophenyl)pyrrolidin-2-one (**10**)

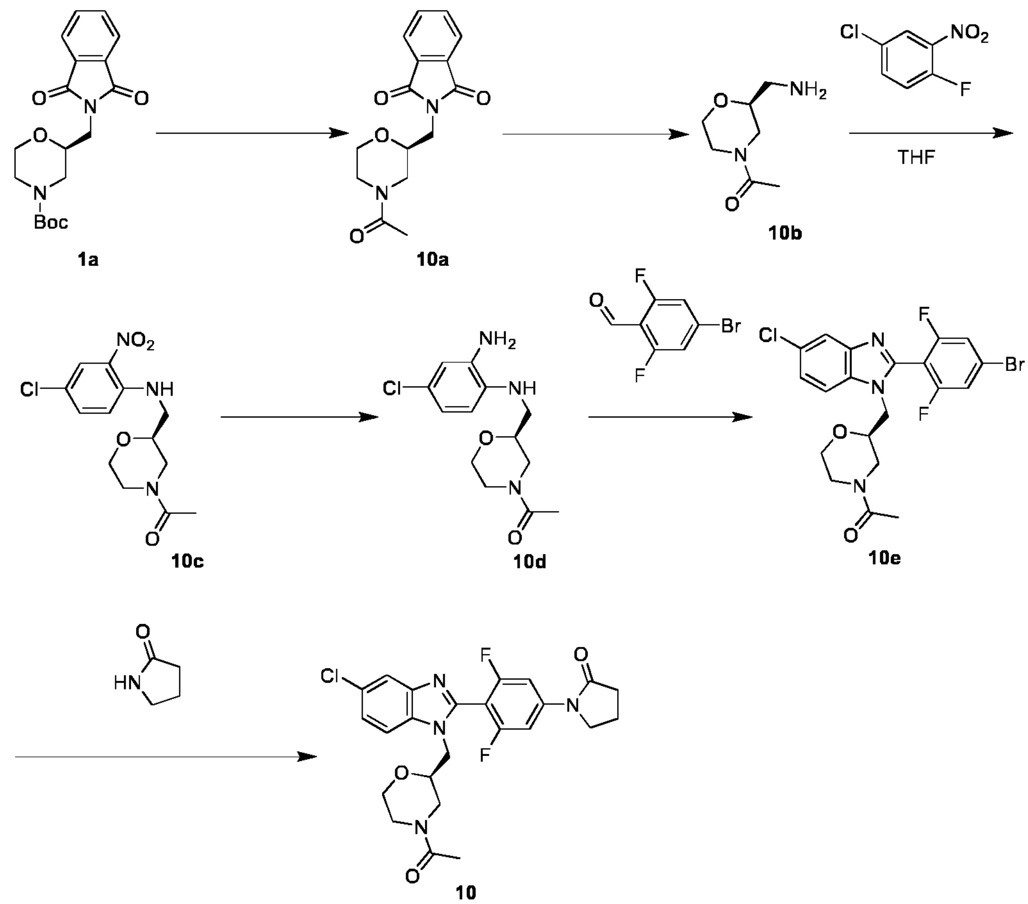


1a 10a 10b 10c 10d 10e 10

Step 1: (*R*)-2-((4-acetylmorpholin-2-yl)methyl)isoindoline-1,3-dione (**10a**)

Compound **1a** (5.00 g, 14.44 mmol) was dissolved in a solution of 4 M HCl in 1,4-dioxane (20 mL), and the mixture was stirred at 25 °C for 2 h. After TLC (petroleum ether/ethyl acetate = 1/1) showed that the reaction was completed, the reaction mixture was concentrated, and the residue was dissolved in tetrahydrofuran (30 mL). Triethylamine (9.8 mL, 70.74 mmol) and acetic anhydride (4.0 mL, 42.44 mmol) were added in an ice-water bath, and the mixture was reacted at room temperature for 1.5 h. After TLC (petroleum ether/ethyl acetate = 1/1) showed that the reaction was completed, water (50 mL) was added to quench the reaction, and the mixture was extracted with ethyl acetate (50 mL $\times$ 3). The combined organic phase was dried over anhydrous sodium sulfate, filtered and concentrated under reduced pressure. The residue was purified by flash silica gel chromatography (ethyl acetate/petroleum ether = 0-100%) to give compound **10a** (2.50 g, yield: 54.0%).

MS (ESI) m/z = 314.1 $[M+H]^+$.

$^1$H NMR (400 MHz, $CDCl_3$) $\delta$ 7.88 (br s, 2H), 7.75 (br d, *J* = 5.4 Hz, 2H), 4.55-4.28 (m, 1H), 3.94 (br d, *J* = 7.2 Hz, 2H), 3.83-3.64 (m, 3H), 3.60-3.42 (m, 1H), 3.38-3.02 (m, 1H), 2.93-2.61 (m, 1H), 2.10 (br d, *J*= 6.0 Hz, 3H).

Step 2: (*S*)-1-(2-(aminomethyl)morpholino)ethan-1-one **(10b)**

To a solution of compound **10a** (1.00 g, 3.47 mmol) in ethanol (6 mL) was added hydrazine hydrate (206 μL, 4.16 mmol) at room temperature, and the mixture was stirred at 80 °C for 1 h. After TLC (petroleum ether/ethyl acetate = 1/1) showed that the reaction was completed, the reaction mixture was cooled to room temperature and filtered, and the filtrate was concentrated under reduced pressure. The residue was dissolved in ethyl acetate (20 mL) and filtered, and the filtrate was concentrated under reduced pressure to give the title compound **10b** (600.0 mg, yield: 98.4%).

$^1$H NMR (400 MHz, $CDCl_3$) $\delta$ 4.42-4.32 (m, 1H), 3.95-3.87 (m, 1H), 3.68-3.43 (m, 3H), 3.42-3.18 (m, 2H), 3.03-2.91 (m, 1H), 2.88-2.57 (m, 3H), 2.46 (dd, *J*= 10.8, 13.2 Hz, 1H), 2.07 (s, 3H).

Step 3: (*S*)-1-(2-(((4-chloro-2-nitrophenyl)amino)methyl)morpholino)ethane- -one (**10c**)

To a solution of compound **10b** (300.0 mg, 1.90 mmol) in tetrahydrofuran (5 mL) were added 1-fluoro-4-chloro-2-nitrobenzene (399.5 mg, 2.28 mmol) and potassium carbonate (523.0 mg, 3.79 mmol) at room temperature, and the mixture was stirred at 25 °C for 2 h. After LCMS showed that the starting material was consumed completely, water (10 mL) was added, and the mixture was extracted with ethyl acetate (15 mL × 3). The combined organic phase was dried over anhydrous sodium sulfate, filtered and concentrated under reduced pressure to give a crude product, which was purified by flash silica gel chromatography (ethyl acetate/petroleum ether = 0-50%, 35 mL/min) to give the title compound **10c** (300.0 mg, yield: 30.2%).

MS (ESI) m/z = 314.1 $[M+H]^+$.

$^1$H NMR (400 MHz, $CDCl_3$) $\delta$ 8.27-8.15 (m, 2H), 7.46-7.37 (m, 1H), 6.89-6.79 (m, 1H), 4.54 (br d, *J* = 13.2 Hz, 1H), 4.02 (dd, *J* = 2.8, 10.8 Hz, 1H), 3.77-3.53 (m, 3H), 3.51-3.28 (m, 3H), 2.67 (dd, *J*= 10.8, 13.2 Hz, 1H), 2.15-2.10 (m, 3H).

Step 4: (*S*)-1-(2-(((2-amino-4-chlorophenyl)amino)methyl)morpholino)ethane-1-one (**10**d)

To a mixed solution of compound **10c** (400.0 mg, 1.28 mmol) in tetrahydrofuran (5 mL), ethanol (5 mL) and water (5 mL) were added ammonium chloride (675.7 mg, 12.75 mmol) and iron powder (356.0 mg, 6.38 mmol) at room temperature, and the mixture was stirred at 60 °C for 2 h. After LCMS showed that the starting material was consumed completely, the mixture was filtered, and the filtrate was extracted with ethyl acetate (30 mL × 3). The combined organic phase was dried over anhydrous sodium sulfate, filtered and concentrated under reduced pressure to give the title compound **10d** (380.0 mg, yield: 94.5%).

$^1$H NMR (400 MHz, $CDCl_3$) $\delta$ 6.78-6.67 (m, 2H), 6.57-6.51 (m, 1H), 4.56-4.38 (m, 1H), 4.03-3.93 (m, 1H), 3.71-3.50 (m, 5H), 3.36-3.25 (m, 1H), 3.23-3.07 (m, 3H), 2.87-2.60 (m, 1H), 2.11 (d, *J* = 3.2 Hz, 3H).

Step 5: (*S*)-1-(2-((2-(4-bromo-2,6-difluorophenyl)-5-chloro-1H-benzo[*d*]imidazol-1-yl)methyl) morpholino)ethan-1-one (**10e**)

To a solution of compound **10d** (190.0 mg, 0.67 mmol) in n-butanol (5 mL) were added 4-bromo-2,6-difluorobenzaldehyde (163.0 mg, 0.74 mmol) and acetic acid (238 μL, 1.34 mmol) at room temperature, and the resulting mixture was stirred at 90 °C for 12 h. After LCMS showed that the starting material was reacted completely, the reaction solution was concentrated in vacuum to give a crude product, which was purified by flash silica gel chromatography (petroleum ether/ethyl acetate = 0-80%) to give the title compound **10e** (300.0 mg, yield: 83.1%).

$^1$H NMR (400 MHz, $CDCl_3$) $\delta$ 7.86-7.79 (m, 1H), 7.46-7.40 (m, 1H), 7.38-7.27 (m, 3H), 4.50-4.31 (m, 1H), 4.16-4.10 (m, 3H), 3.85-3.70 (m, 1H), 3.55-3.47 (m, 1H), 3.35-3.25 (m, 1H), 3.18-3.06 (m, 1H), 2.37-2.26 (m, 1H), 2.05-2.05 (m, 3H).

Step 6: (*S*)-1-(4-(1-((4-acetylmorpholin-2-yl)methyl)-5-chloro-1*H*-benzo[*d*]imidazol-2-yl)-3,5-difluorophenyl)pyrrolidin-2-one (**10**)

To a solution of compound **10e** (50.0 mg, 0.172 mmol) and pyrrolidin-2-one (8.7 mg, 0.10 mmol) in 1,4-dioxane (3 mL) were added $Cs_2CO_3$ (67.2 mg, 0.21 mmol), $Pd(dba)_2$ (5.9 mg, 0.01 mmol) and Xantphos (11.9 mg, 0.02 mmol) at room temperature, and the resulting mixture was purged with nitrogen for 5 min and stirred at 90 °C for 12 h. After LCMS showed that the reaction was completed, the reaction mixture was cooled to room temperature and filtered, and the filtrate was concentrated under reduced pressure. The residue was purified by preparative HPLC (column: YMC-Actus Triart C18 150×30 mm×7 μm, acetonitrile/water (containing 0.05% ammonia water) = 47%-70%, 9 min) and lyophilized to give the title compound **10** (13.5 mg, yield: 26.8%).

MS (ESI) m/z = 489.1 $[M+H]^+$.

$^1$H NMR (400 MHz, DMSO-$d_6$) $\delta$ 7.82-7.73 (m, 2H), 7.66 (d, *J* = 10.8 Hz, 2H), 7.36 (dd, *J* = 2.0, 8.8 Hz, 1H), 4.33 (br dd, *J* = 3.2, 15.2 Hz, 1H), 4.17 (br dd, *J* = 7.6, 15.2 Hz, 1H), 3.92 (t, *J* = 7.2 Hz, 2H), 3.69-3.48 (m, 3H), 3.21 (br d, *J* = 10.0 Hz, 2H), 3.10 (br s, 1H), 3.00 (br s, 1H), 2.60 (t, *J* = 8.0 Hz, 2H), 2.17-2.11 (m, 2H), 1.93 (s, 3H).

Example **11**

(*S*)-1-(4-(1-((4-acetylmorpholin-2-yl)methyl)-6-fluoro-5-methyl-1*H*-benzo[*d*]imidazol-2 -yl)-3-chloro-5-fluorophenyl)pyrrolidin-2-one (**11**)

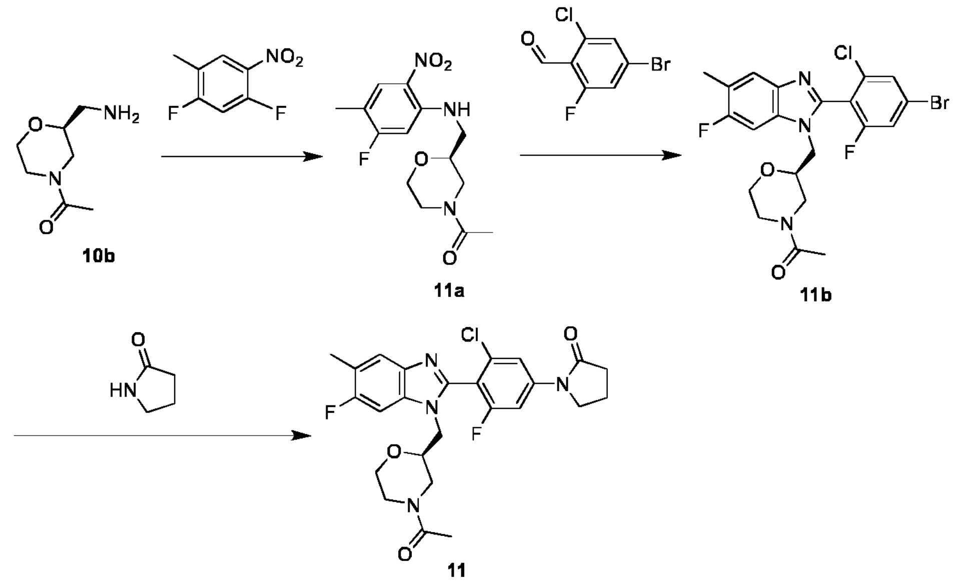

10b 11a 11b 11

Step 1: (*S*)-1-(2-(((5-fluoro-4-methyl-2-nitrophenyl)amino)methyl)morpholino)ethane-1-one (**11a**)

Compound **11a** was synthesized by referring to step 3 in Example **10,** with 1-fluoro-4-chloro-2-nitrobenzene replaced by 1,5-difluoro-2-methyl-4-nitrobenzene. MS (ESI) m/z = 312.1 $[M+H]^+$.

$^1H$ NMR (400 MHz, $CDCl_3$) $\delta$ 8.24 (br s, 1H), 8.11 - 8.04 (m, 1H), 6.53 - 6.45 (m, 1H), 4.54 (br d, *J* = 13.3 Hz, 1H), 4.07 - 4.01 (m, 1H), 3.74 - 3.58 (m, 3H), 3.46 - 3.26 (m, 3H), 2.66 (dd, *J* = 10.8, 13.1 Hz, 1H), 2.20 - 2.12 (m, 6H).

Step 2: (*S*)-1-(2-((2-(4-bromo-2-chloro-6-fluorophenyl)-6-fluoro-5-methyl-1*H*-benzo[*d*]imidazo 1-1-yl)methyl)morpholino)ethan-1-one (**11b**)

To a mixed solution of compound **11a** (100.0 mg, 0.32 mmol) in ethanol (2 mL) and DMSO (2 mL) were added 4-bromo-2-chloro-6-fluorobenzaldehyde (83.9 mg, 0.35 mmol) and $Na_2S_2O_4$ (447.4 mg, 2.57 mmol) at room temperature, and the resulting mixture was stirred at 90 °C for 12 h. After LCMS showed that the starting material was reacted completely, water (15 mL) was added to quench the reaction, and the mixture was extracted with ethyl acetate (20 mL × 3). The combined organic phase was dried over anhydrous sodium sulfate, filtered and concentrated under reduced pressure. The residue was purified by C18 reverse phase chromatography (acetonitrile/water (containing 0.05% $NH_3 \cdot H_2O$) = 10%-75%, flow rate: 40 mL/min) and lyophilized to give the title compound **11b** (100.0 mg, yield: 62.5%).

MS (ESI) m/z = 498.1 $[M+H]^+$.

Step 3: (*S*)-1-(4-(1-((4-acetylmorpholin-2-yl)methyl)-6-fluoro-5-methyl-1*H*-benzo[*d*]imidazol-2 -yl)-3-chloro-5-fluorophenyl)pyrrolidin-2-one (**11**)

Compound **11** was synthesized by referring to step 6 in Example **10**, with compound **10e** replaced by compound **11b.**

MS (ESI) m/z = 503.4 $[M+H]^+$.

Example **12**

(*S*)-1-(4-(1-((4-acetylmorpholin-2-yl)methyl)-5-chloro-6-fluoro-1*H*-benzo[*d*]imidazol-2 -yl)-3-chloro-5-fluorophenyl)pyrrolidin-2-one (**12**)

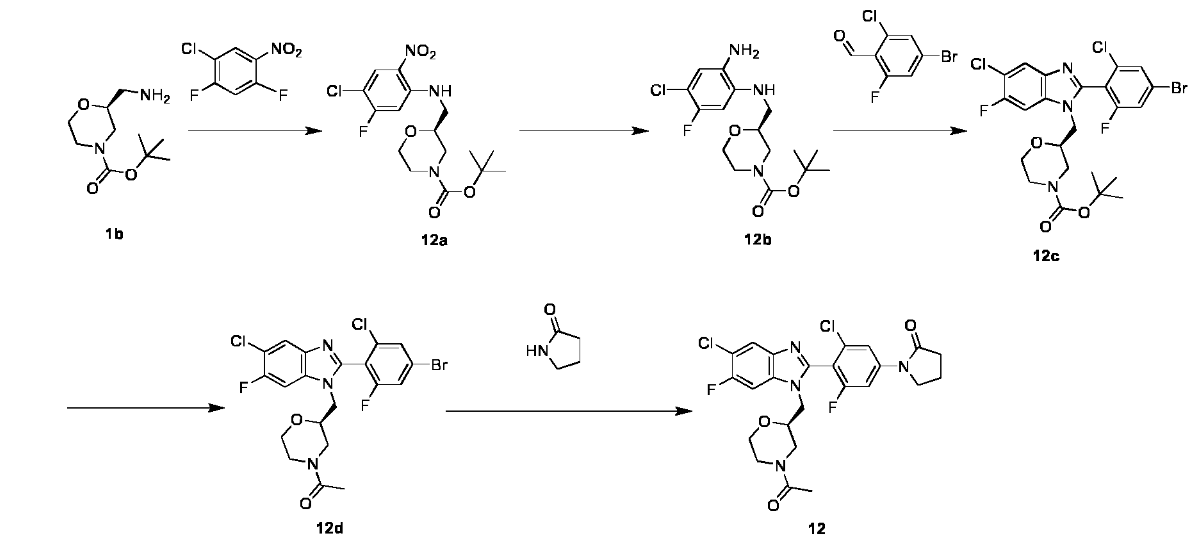

1b 12a 12b 12c 12d 12

Step 1: *tert*-butyl (*S*)-2-(((4-chloro-5-fluoro-2-nitrophenyl)amino)methyl)morpholine-4-carboxylate **(12a)**

Compound **12a** was synthesized by referring to step 3 in Example **10,** with 1-fluoro-4-chloro-2-nitrobenzene replaced by 1-chloro-2,4-difluoro-5-nitrobenzene.

$^1$H NMR (400 MHz, $CDCl_3$) $\delta$ 8.36 - 8.25 (m, 2H), 6.65 (d, *J* = 11.6 Hz, 1H), 4.09 - 3.80 (m, 3H), 3.75 - 3.67 (m, 1H), 3.63 - 3.53 (m, 1H), 3.43 - 3.35 (m, 1H), 3.34 - 3.25 (m, 1H), 2.99 (br s, 1H), 2.79 (br s, 1H), 1.48 (s, 9H).

Step 2: tert-butyl (*S*)-2-(((2-amino-4-chloro-5-fluorophenyl)amino)methyl)morpholine-4-carboxylate **(12b)**

Compound **12b** was synthesized by referring to step 4 in Example **10,** with compound **10c** replaced by compound **12a.**

$^1$H NMR (400 MHz, DMSO-$d_6$) $\delta$ 7.81-7.65 (m, 1H), 6.63-6.37 (m, 1H), 4.68 (br s, 1H), 4.40-4.18 (m, 1H), 3.84 (br d, *J* = 12.0 Hz, 1H), 3.76-3.61 (m, 2H), 3.12-3.00 (m, 1H), 2.85 (br s, 1H), 2.53 (br s, 2H), 1.40 (d, *J*= 1.6 Hz, 9H), 1.27-1.20 (m, 3H).

Step 3: tert-butyl (*S*)-2-((2-(4-bromo-2-chloro-6-fluorophenyl)-5-chloro-6-fluoro-1*H*-benzo[*d*]imidazol-1-yl)methyl)morpholine-4-carboxylate (**12c**)

Compound **12c** was synthesized by referring to step 5 in Example **10,** with compound **10d** replaced by compound **12b,** and 4-bromo-2,6-difluorobenzaldehyde replaced by 4-bromo-2-chloro-6-fluorobenzaldehyde.

MS (ESI) m/z = 578.1 [M+H]$^+$.

$^1$H NMR (400 MHz, $CDCl_3$) $\delta$ 7.87 (d, *J* = 6.8 Hz, 1H), 7.59-7.55 (m, 1H), 7.40-7.33 (m, 2H), 4.08-3.97 (m, 2H), 3.75 (br s, 3H), 3.62-3.50 (m, 1H), 3.33 (br s, 1H), 2.81 (br s, 1H), 2.47 (br t, *J* = 11.6 Hz, 1H), 0.96 (s, 9H).

Step 4: (*S*)-1-(2-((2-(4-bromo-2-chloro-6-fluorophenyl)-5-chloro-6-fluoro-1*H*-benzo[*d*]imidazol -1-yl)methyl)morpholino)ethan-1-one (**12d**)

Compound **12c** was synthesized by referring to step 1 in Example **10,** with compoundla replaced by compound **12c.**

MS (ESI) m/z = 518.0 [M+H]$^+$.

$^1$H NMR (400 MHz, $CDCl_3$) $\delta$ 7.90-7.85 (m, 1H), 7.61-7.56 (m, 1H), 7.41-7.32 (m, 2H), 4.50-4.38 (m, 1H), 4.08-4.01 (m, 2H), 3.86-3.79 (m, 1H), 3.55 (br d, *J*= 11.2 Hz, 2H), 3.41-3.32 (m, 1H), 3.24-3.11 (m, 1H), 2.42-2.27 (m, 1H), 2.09 (s, 3H).

Step 5: (*S*)-1-(4-(1-((4-acetylmorpholin-2-yl)methyl)-5-chloro-6-fluoro-1*H*-benzo[*d*]imidazol-2 -yl)-3-chloro-5-fluorophenyl)pyrrolidin-2-one (**12**)

Compound **12** was synthesized by referring to step 6 in Example **10,** with compound **10e** replaced by compound **12d.**

MS (ESI) m/z = 523.3 $[M+H]^+$.

$^1H$ NMR (400 MHz, DMSO-$d_6$) $\delta$ 7.93-7.87 (m, 3H), 7.78 (dd, $J$ = 2.0, 12.0 Hz, 1H), 4.38-3.99 (m, 3H), 3.93 (t, $J$ = 7.2 Hz, 2H), 3.72-3.44 (m, 3H), 3.33-3.13 (m, 2H), 3.06-2.85 (m, 1H), 2.60 (t, $J$ = 8.4 Hz, 2H), 2.13 (quin, $J$ = 7.6 Hz, 2H), 1.95 (s, 3H).

Example **13**

(*S*)-1-(4-(1-((4-acetylmorpholin-2-yl)methyl)-5-chloro-7-fluoro-1*H*-benzo[*d*]imidazol-2 -yl)-3,5-difluorophenyl)pyrrolidin-2-one (**13**)

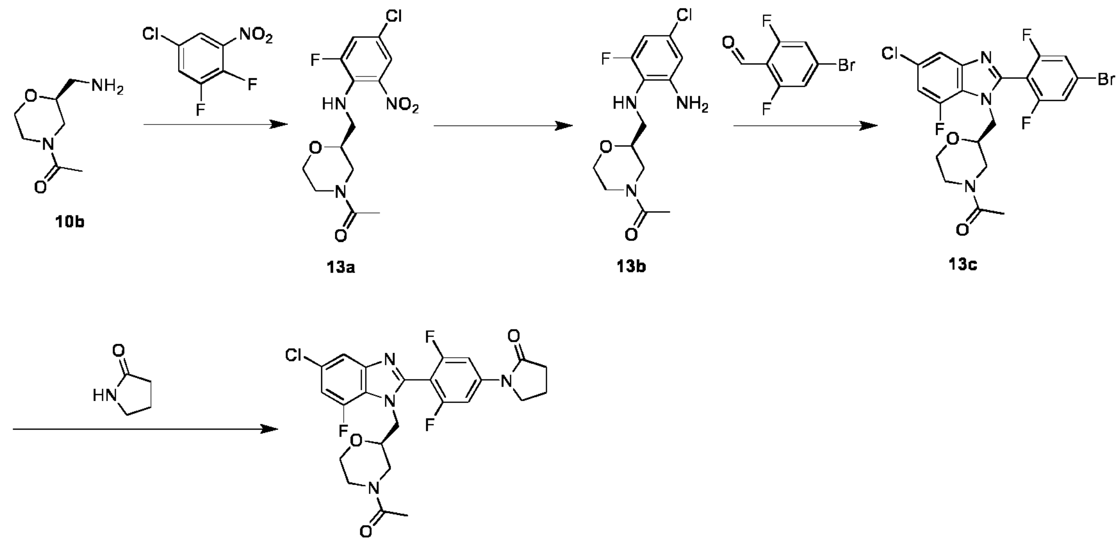

10b 13a 13b 13c

13

Step 1: (*S*)-1-(2-(((4-chloro-2-fluoro-6-nitrophenyl)amino)methyl)morpholino)ethane-1-one (**13a**)

Compound **13a** was synthesized by referring to step 3 in Example **10,** with 1-fluoro-4-chloro-2-nitrobenzene replaced by 5-chloro-1,2-difluoro-3-nitrobenzene. MS (ESI) m/z = 332.2 $[M+H]^+$.

$^1H$ NMR (400 MHz, $CDCl_3$) $\delta$ 8.05-7.92 (m, 2H), 7.27-7.20 (m, 1H), 4.54-4.40 (m, 1H), 4.05-3.97 (m, 1H), 3.83-3.73 (m, 1H), 3.71-3.60 (m, 2H), 3.60-3.51 (m, 2H), 3.36-3.06 (m, 1H), 2.87-2.55 (m, 1H), 2.12 (s, 3H).

Step 2: (*S*)-1-(2-(((2-amino-4-chloro-6-fluorophenyl)amino)methyl)morpholino)ethan-1-one (**13b**)

Compound **13b** was synthesized by referring to step 4 in Example **10,** with compound **10c** replaced by compound **13a.**

$^1H$ NMR (400 MHz, $CDCl_3$) $\delta$ 6.53-6.45 (m, 2H), 4.42 (br d, $J$ = 13.2 Hz, 1H), 4.13 (q, $J$ = 7.2 Hz, 3H), 4.00 (br d, $J$ = 11.6 Hz, 1H), 3.63-3.44 (m, 3H), 3.36-3.25 (m, 1H), 3.09-2.92 (m, 2H), 2.85-2.53 (m, 1H), 2.09 (d, $J$= 9.2 Hz, 3H).

Step 3: (*S*)-1-(2-((2-(4-bromo-2,6-difluorophenyl)-5-chloro-7-fluoro-1*H*-benzo[*d*]imidazol-1-yl )methyl)morpholino)ethan-1-one (**13c**)

Compound **13c** was synthesized by referring to step 5 in Example **10,** with compound **10d** replaced by compound **13b.**

MS (ESI) m/z = 503.8 $[M+H]^+$.

$^1H$ NMR (400 MHz, $CDCl_3$) $\delta$ 7.67-7.60 (m, 1H), 7.33-7.27 (m, 2H), 7.14-7.05 (m, 1H), 4.51 (br d, $J$ = 13.2 Hz, 1H), 4.41-4.27 (m, 2H), 3.73-3.58 (m, 2H), 3.28 (br t, $J$ = 11.6 Hz, 1H), 3.15-3.03 (m, 1H), 2.81 (br t, $J$ = 12.2 Hz, 1H), 2.64-2.53 (m, 1H), 2.34-2.25 (m, 1H), 2.05 (s, 3H).

Step 4: (*S*)-1-(4-(1-((4-acetylmorpholin-2-yl)methyl)-5-chloro-7-fluoro-1*H*-benzo[*d*]imidazol-2 -yl)-3,5-difluorophenyl)pyrrolidin-2-one (**13**)

Compound **13** synthesized by referring to step 6 in Example **10,** with compound **10e** replaced by compound **13c.**
MS (ESI) m/z = 507.2 $[M+H]^+$.
$^1H$ NMR (400 MHz, DMSO-$d_6$) $\delta$ 7.79-7.65 (m, 3H), 7.41 (d, *J* = 11.2 Hz, 1H), 4.51-4.37 (m, 1H), 4.23 (br d, *J* = 13.2 Hz, 1H), 4.11 (br dd, *J* = 8.4, 15.2 Hz, 1H), 4.02 (br d, *J* = 13.2 Hz, 1H), 3.90 (t, *J* = 7.2 Hz, 2H), 3.73 (br d, *J* = 12.8 Hz, 1H), 3.63-3.50 (m, 2H), 3.28-3.07 (m, 1H), 3.02-2.72 (m, 1H), 2.59 (t, *J* = 8.0 Hz, 2H), 2.30-2.20 (m, 1H), 2.57-2.07 (m, 2H), 1.93 (s, 3H).

Example **14**

(*S*)-1-(4-(1-((4-acetylmorpholin-2-yl)methyl)-5-(methyl-$d_3$)-1*H*-benzo[*d*]imidazol-2-yl)-3,5-difluorophenyl)pyrrolidin-2-one (**14**)

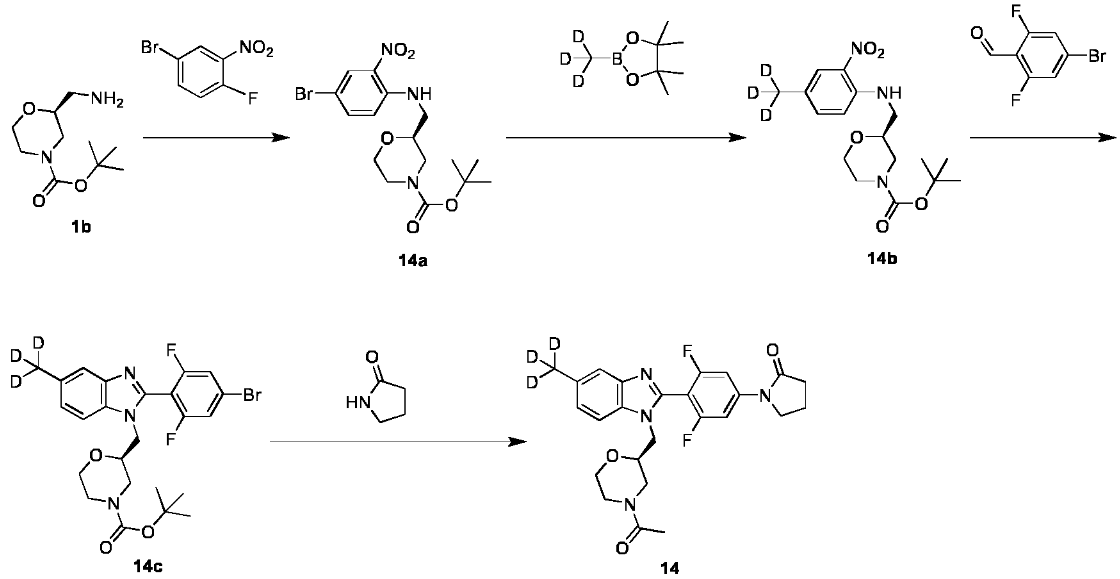

1b 14a 14b 14c 14

Step 1: tert-butyl (*S*)-2-(((4-bromo-2-nitrophenyl)amino)methyl)morpholine-4-carboxylate **(14a)**

To a solution of compound **1b** (2.00 g, 7.40 mmol) in 1,4-dioxane (10 mL) were added 4-bromo-1-fluoro-2-nitrobenzene (1.63 g, 7.40 mmol) and triethylamine (2.0 mL, 14.80 mmol) at room temperature, and the mixture was stirred at 80 °C for 12 h. After LCMS showed that the starting material was consumed completely, the reaction mixture was directly concentrated under reduced pressure to give a crude product, which was purified by flash silica gel chromatography (ethyl acetate/petroleum ether = 0%-15%, 60 mL/min) to give the title compound **14a** (2.00 g, yield: 58.4%).
$^1H$ NMR (400 MHz, $CDCl_3$) $\delta$ 8.25 (d, *J* = 2.4 Hz, 1H), 8.15 (br s, 1H), 7.44 (dd, *J* = 2.4, 9.2 Hz, 1H), 6.71 (d, *J* = 9.2 Hz, 1H), 3.96-3.72 (m, 3H), 3.63 (tdd, *J* = 3.2, 7.2, 10.4 Hz, 1H), 3.55-3.46 (m, 1H), 3.39-3.23 (m, 2H), 2.92 (br s, 1H), 2.71 (br s, 1H), 1.40 (s, 9H).

Step 2: *tert*-butyl (*S*)-2-(((4-(methyl-$d_3$)-2-nitrophenyl)amino)methyl)morpholine-4-carboxylate (**14b**)

To a mixed solution of compound **14a** (1.10 g, 2.64 mmol) and 4,4,5,5-tetramethyl-2-(methyl-$d_3$)-1,3,2-dioxaborolan (1.15 g, 7.93 mmol) in 1,4-dioxane (15 mL) and water (5 mL) were added $Cs_2CO_3$ (2.58 g, 7.93 mmol) and $Pd(dppf)Cl_2CH_2Cl_2$ (58.8 mg, 0.072 mmol) at room temperature, and the resulting mixture was purged with nitrogen for 5 min and stirred at 100 °C for 12 h under nitrogen atmosphere. After LCMS showed that the reaction was completed, the reaction mixture was cooled to room temperature. Water (20 mL) was added, and the mixture was extracted with ethyl acetate (50 mL × 3). The combined organic phase was dried over anhydrous sodium sulfate, filtered and concentrated under reduced pressure to give a crude product, which was purified by flash silica gel chromatography (ethyl acetate/petroleum ether = 0-20%, 35 mL/min) to give the title compound **14b** (470.0 mg, yield: 45.2%).

MS (ESI) m/z = 299.2 [M+H-56]+.

$^1$H NMR (400 MHz, DMSO-*d*$_6$) $\delta$ 8.09 (br t, *J* = 5.6 Hz, 1H), 7.87 (d, *J* = 2.4 Hz, 1H), 7.39 (dd, *J* = 2.0, 8.8 Hz, 1H), 7.04 (d, *J* = 8.8 Hz, 1H), 3.87 (br d, *J* = 11.2 Hz, 2H), 3.70 (br d, *J* = 13.2 Hz, 1H), 3.63-3.50 (m, 2H), 3.47-3.36 (m, 2H), 3.27-3.24 (m, 1H), 2.88 (br s, 1H), 1.39 (s, 9H).

Step 3: *tert*-butyl (*S*)-2-((2-(4-bromo-2,6-difluorophenyl)-5-(methyl-*d*$_3$)-1*H*-benzo[*d*]imidazol-1-yl)methy l)morpholine-4-carboxylate (**14c**)

To a mixed solution of compound **14b** (200.0 mg, 0.56 mmol) in ethanol (6 mL) and water (2 mL) were added 4-bromo-2,6-difluorobenzaldehyde (124.7 mg, 0.56 mmol) and $Na_2S_2O_4$ (294.8 mg, 1.69 mmol) at room temperature, and the resulting mixture was stirred at 80 °C for 4 h under nitrogen atmosphere. After LCMS showed that the starting material was reacted completely, the reaction mixture was filtered, and water (3 mL) was added to the filtrate. The resulting mixture was extracted with ethyl acetate (10 mL × 3). The combined organic phase was dried over anhydrous sodium sulfate, filtered and concentrated under reduced pressure to give the title compound **14c** (340.0 mg, yield: 114.7%).

MS (ESI) m/z = 525.1 [M+H]+.

Step 4: (*S*)-1-(4-(1-((4-acetylmorpholin-2-yl)methyl)-5-(methyl-*d*$_3$)-1*H*-benzo[*d*]imidazol-2-yl)-3,5-difluorophenyl)pyrrolidin-2-one (**14**)

To a solution of compound **14c** (300.0 mg, 0.57 mmol) and pyrrolidin-2-one (48.4 mg, 0.57 mmol) in 1,4-dioxane (8 mL) were added $Cs_2CO_3$ (372.1 mg, 1.14 mmol), Pd(dba)$_2$ (32.8 mg, 0.057 mmol) and Xantphos (66.1 mg, 0.11 mmol) at room temperature, and the resulting mixture was purged with nitrogen for 5 min and stirred at 100 °C for 2 h. After LCMS showed that the reaction was completed, the reaction mixture was cooled to room temperature, followed by the addition of *p*-toluenesulfonic acid (981.5 mg, 5.70 mmol). The resulting mixture was stirred at 100 °C for 1 h. After LCMS showed that the reaction was completed, the reaction mixture was cooled to room temperature, methanol (5 mL) was added, and triethylamine (1.2 mL, 8.55 mmol) was added in an ice-water bath to adjust the pH to 8, followed by the addition of acetic anhydride (267 μL, 2.85 mmol). The resulting mixture was reacted at room temperature for 1 h. After LCMS showed that the reaction was completed, water (10 mL) was added, and the mixture was extracted with ethyl acetate (20 mL × 3). The combined organic phase was dried over anhydrous sodium sulfate, filtered and concentrated under reduced pressure. The residue was purified by preparative HPLC (column: YMC Triart C18 250×50 mm×7 μm, acetonitrile/water (containing 0.05% ammonia water) = 12%-52%, 9 min) and lyophilized to give the title compound **14** (90.2 mg, yield: 33.3%).

MS (ESI) m/z = 472.2 [M+H]+.

$^1$H NMR (400 MHz, DMSO-*d*$_6$) $\delta$ 7.69-7.58 (m, 3H), 7.49 (d, *J* = 1.2 Hz, 1H), 7.16 (dd, *J* = 1.6, 8.4 Hz, 1H), 4.27 (br dd, *J* = 3.6, 15.2 Hz, 1H), 4.12 (br dd, *J* = 7.2, 15.2 Hz, 1H), 3.92 (t, *J* = 7.2 Hz, 2H), 3.64 (br dd, *J* = 2.0, 11.6 Hz, 1H), 3.54 (br s, 2H), 3.23 (br s, 2H), 3.14-3.09 (m, 2H), 2.60 (t, *J* = 8.0 Hz, 2H), 2.18-2.08 (m, 2H), 1.93 (s, 3H).

Example **15**

(*S*)-1-(3-chloro-4-(1-((4-(cyclopropylcarbonyl)morpholin-2-yl)methyl)-5-methyl-1*H*-be nzo[*d*]imidazol-2-yl)-5-fluorophenyl)pyrrolidin-2-one **(15)**

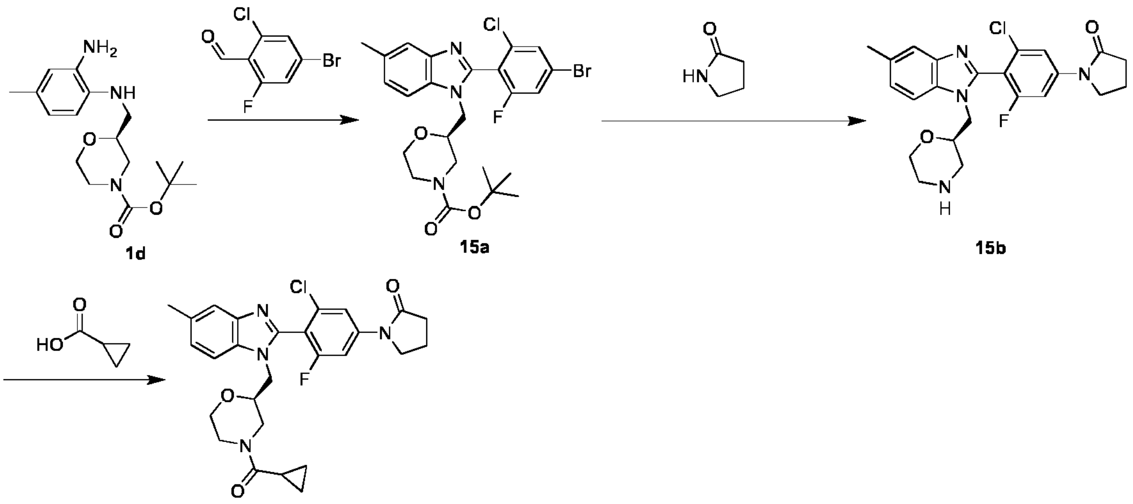

1d 15a 15b 15

Step 1: *tert*-butyl (*S*)-2-((2-(4-bromo-2-chloro-6-fluorophenyl)-5-methyl-1*H*-benzo[*d*]imidazol-1-yl)meth yl)morpholine-4-carboxylate **(15a)**

Compound **15a** was synthesized by referring to step 5 in Example **1,** with 4-bromo-2,6-difluorobenzaldehyde replaced by 4-bromo-2-chloro-6-fluorobenzaldehyde.

MS (ESI) m/z = 540.1 $[M+H+2]^+$.

Step 2: (*R*)-1-(3-chloro-5-fluoro-4-(5-methyl-1-(morpholin-2-ylmethyl)-1*H*-benzo[*d*]imidazol-2 -yl)phenyl)pyrrolidin-2-one **(15b)**

To a solution of compound **15a** (870.0 mg, 1.62 mmol) and pyrrolidin-2-one (549.7 mg, 6.46 mmol) in 1,4-dioxane (10 mL) were added $Cs_2CO_3$ (1.05 g, 3.23 mmol), $Pd(dba)_2$ (92.9 mg, 0.16 mmol) and Xantphos (186.8 mg, 0.32 mmol) at room temperature, and the resulting mixture was purged with nitrogen for 5 min and stirred at 100 °C for 4 h. After LCMS showed that the reaction was completed, the reaction mixture was cooled to room temperature, followed by the addition of *p*-toluenesulfonic acid monohydrate (3.07 g, 16.14 mmol). The resulting mixture was stirred at 100 °C for 1 h. After LCMS showed that the reaction was completed, water (15 mL) was added, and the mixture was extracted with ethyl acetate (20 mL × 3). The aqueous phase was adjusted to pH 8 with 1 N aqueous NaOH solution and extracted with ethyl acetate (20 mL × 3). The combined organic phases were dried over anhydrous sodium sulfate, filtered and concentrated under reduced pressure to give the title compound **15b** (680.0 mg, yield: 95.1%).

MS (ESI) m/z = 443.5 $[M+H]^+$.

Step 3: (*S*)-1-(3-chloro-4-(1-((4-(cyclopropylcarbonyl)morpholin-2-yl)methyl)-5-methyl-1*H*-be nzo[*d*]imidazol-2-yl)-5-fluorophenyl)pyrrolidin-2-one **(15)**

To a solution of compound **15b** (50.0 mg, 0.11 mmol) in DMF (1 mL) were added cyclopropanecarboxylic acid (19.4 mg, 0.23 mmol), HATU (85.8 mg, 0.0.23 mmol) and *N,N*-diisopropylethylamine (75 μL, 0.45 mmol) successively, and the mixture was stirred at room temperature for 1 h. The reaction mixture was directly purified by C18 reverse phase chromatography (acetonitrile/water (containing 0.05% ammonia water) = 10%-75%) and lyophilized to give the title compound **15** (32.0 mg, yield: 55.5%).

MS (ESI) m/z = 469.4 $[M+H]^+$.

$^1H$ NMR (400 MHz, DMSO-$d_6$, t = 75 °C) δ 7.87 (s, 1H), 7.77 (dd, *J* = 12.0, 2.1 Hz, 1H), 7.60 (d, *J* = 8.3 Hz, 1H), 7.49 (s, 1H), 7.16 (dd, *J* = 8.4, 1.6 Hz, 1H), 4.27-4.11 (m, 2H), 4.08 (t, *J* = 5.1 Hz, 2H), 3.99 (d, *J* = 13.5 Hz, 1H), 3.93 (t, *J* = 7.1 Hz, 2H), 3.68 (br s, 1H), 3.56 (br s, 1H), 3.47 (dd, *J* = 7.0, 5.1 Hz, 2H), 3.25 (t, *J* = 11.7 Hz, 1H), 2.59 (dd, *J* = 8.5, 7.6 Hz, 2H), 2.46 (s, 3H), 2.13 (p, *J* = 7.5 Hz, 2H), 1.08 (t, *J* = 7.0 Hz, 4H).

Example **16**

(*S*)-1-(3-chloro-5-fluoro-4-(5-methyl-1-((4-(3,3,3-trifluoropropionyl)morpholin-2-yl)me thyl)-1*H*-benzo[*d*]imidazol-2-yl)phenyl)pyrrolidin-2-one **(16)**

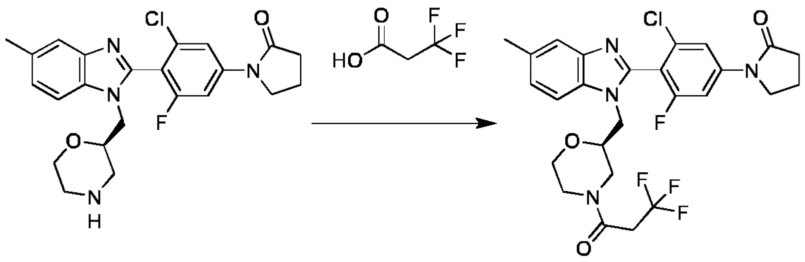

**15b** **16**

Compound **16** was synthesized by referring to step 3 in Example **15,** with cyclopropanecarboxylic acid replaced by 3,3,3-trifluoropropionic acid.

$^1$H NMR (400 MHz, DMSO-$d_6$, t = 75 °C) $\delta$ 7.86 (s, 1H), 7.76 (dd, *J* = 12.0, 2.1 Hz, 1H), 7.60 (d, *J* = 8.3 Hz, 1H), 7.49 (s, 1H), 7.16 (dd, *J* = 8.5, 1.6 Hz, 1H), 4.41-4.13 (m, 2H), 4.08 (t, *J* = 5.1 Hz, 2H), 3.93 (t, *J* = 7.1 Hz, 2H), 3.78-3.64 (m, 1H), 3.59-3.44 (m, 5H), 3.35-3.16 (m, 1H), 2.59 (t, *J* = 8.1 Hz, 2H), 2.46 (s, 3H), 2.13 (p, *J* = 7.6 Hz, 2H).

Example **17**

(*S*)-6-chloro-8-fluoro-7-(5-methyl-1-((4-propionylmorpholin-2-yl)methyl)-1*H*-benzo[*d*]i midazol-2-yl)-2*H*-benzo[*b*][1,4] oxazin-3(4*H*)-one **(17)**

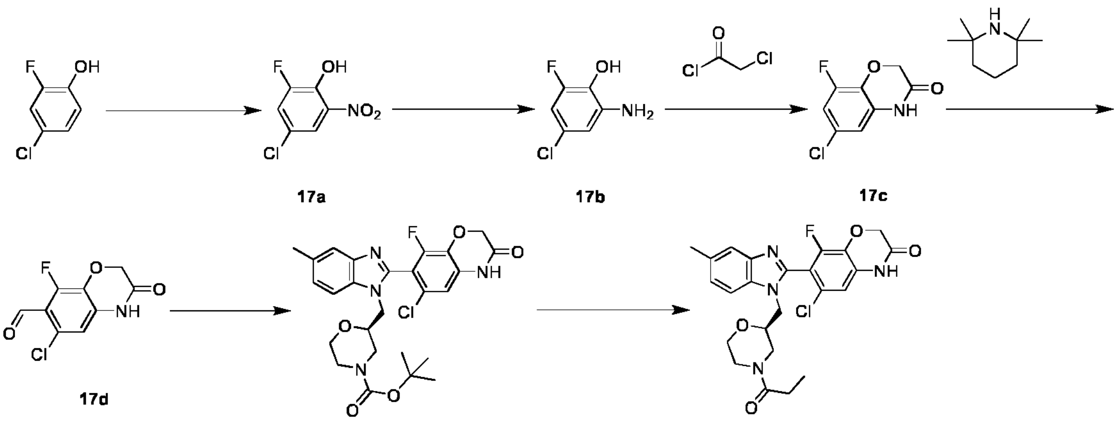

**17a** **17b** **17c** **17d** **17e** **17**

Step 1: 4-chloro-2-fluoro-6-nitrophenol **(17a)**

Fuming nitric acid (4.51 g, 71.65 mmol) was slowly added dropwise to a solution of 4-chloro-2-fluorophenol (10.00 g, 68.24 mmol) in acetic acid (100 mL) in an ice-water bath, and the mixture was stirred in the ice-water bath for 1 h. After LCMS showed that the starting material was consumed completely, the reaction mixture was slowly poured into water (1.5L) while stirring. The resulting mixture was stirred for 1 h and filtered in vacuum to give a solid, which was dried to obtain the title compound **17a** (11.00 g, yield: 84%).

$^1$H NMR (400 MHz, $CDCl_3$) $\delta$ 10.38 (s, 1H), 7.94 (t, *J* = 2.4 Hz, 1H), 7.45 (dd, *J* = 2.4, 9.6 Hz, 1H).

Step 2: 2-amino-4-chloro-6-fluorophenol **(17b)**

To a mixed solution of compound **17a** (5.00 g, 26.10 mmol) in tetrahydrofuran (60 mL), ethanol (60 mL) and water (30 mL) were added ammonium chloride (13.96 g, 261.04 mmol) and iron powder (11.69 g, 208.83 mmol) at room temperature, and the mixture was stirred at 60 °C for 2 h. After LCMS showed that the starting material was consumed completely, the reaction mixture was filtered, and the filtrate was concentrated under reduced pressure. The residue was dispersed in ethyl acetate (500 mL) and filtered, and the filtrate was concentrated under reduced pressure to give the title compound **17b** (4.20 g, yield: 99.6%).
MS (ESI) m/z = 162.0 $[M+H]^+$.
$^1H$ NMR (400 MHz, $CDCl_3$) $\delta$ 6.56-6.49 (m, 2H), 5.03 (br s, 1H), 3.93 (br s, 2H).

Step 3: 6-chloro-8-fluoro-2*H*-benzo[*b*][1,4]oxazin-3(4*H*)-one **(17c)**

To a solution of compound **17b** (4.20 g, 26.00 mmol) in tetrahydrofuran (50 mL) were added potassium carbonate (10.78 g, 77.99 mmol) and chloroacetyl chloride (3.1 mL, 38.99 mmol) in an ice-water bath, and the mixture was stirred at 40 °C for 15 h. After LCMS showed that the starting material was consumed completely, water (100 mL) was added to quench the reaction, and the mixture was extracted with dichloromethane (100 mL $\times$ 3). The organic phase was dried and concentrated, and the crude product was purified by silica gel column chromatography (ethyl acetate/petroleum ether = 30%, flow rate: 30 mL/min) to give the title compound **17c** (4.30 g, 82.0%).
$^1H$ NMR (400 MHz, DMSO-$d_6$) $\delta$ 11.00 (br s, 1H), 7.10 (dd, *J* = 2.4, 10.4 Hz, 1H), 6.74 (t, *J* = 2.0 Hz, 1H), 4.68 (s, 2H).

Step 4: 6-chloro-8-fluoro-3-oxo-3,4-dihydro-2*H*-benzo[*b*][1,4]oxazine-7-carbaldehyde **(17d)**

To a solution of 2,2,6,6-tetramethylpiperidine (3.8 mL, 22.32 mmol) in anhydrous tetrahydrofuran (4 mL) was added a solution of 2.5 M *n*-butyllithium in *n*-hexane (8.9 mL, 22.32 mmol) dropwise at -78 °C. After the addition, the mixture was stirred for 20 min with the temperature maintained at -78 °C, and then a solution of compound **17c** (1.50 g, 7.44 mmol) in anhydrous tetrahydrofuran (15 mL) was slowly added dropwise. The mixture was stirred at -78 °C for 3 h, and then *N,N*-dimethylformamide (1.09 g, 14.88 mmol) was slowly added dropwise. The resulting mixture was slowly warmed to room temperature and reacted for 30 min. After TLC (petroleum ether/ethyl acetate = 1/1) showed that the reaction was completed, a saturated aqueous ammonium chloride solution was added to quench the reaction, and the mixture was extracted with ethyl acetate (50 mL $\times$ 3). The combined organic phase was dried over anhydrous sodium sulfate, filtered and concentrated to give the title compound **17d** (600.0 mg, 35.0%).
$^1H$ NMR (400 MHz, DMSO-$d_6$) $\delta$ 11.38 (br s, 1H), 10.16 (d, *J* = 0.8 Hz, 1H), 6.85 (d, *J* = 1.6 Hz, 1H), 4.76 (s, 2H).

Step 5: *tert*-butyl (*S*)-2-((2-(6-chloro-8-fluoro-3-carbonyl-3,4-dihydro-2*H*-benzo[*b*][1,4]oxazin-7-yl)-5-m ethyl-1*H*-benzo[*d*]imidazol-1-yl)methyl)morpholine-4-carboxylate **(17e)**

Compound **17e** was synthesized by referring to step 3 in Example **14,** with compound **14b** replaced by compound **1c,** and 4-bromo-2,6-difluorobenzaldehyde replaced by compound **17d.**
MS (ESI) m/z = 531.2 $[M+H]^+$.
$^1H$ NMR (400 MHz, $CDCl_3$) $\delta$ 9.81 (br s, 1H), 7.69 (s, 1H), 7.41 (dd, *J* = 2.4, 8.4 Hz, 1H), 7.21 (d, *J* = 8.4 Hz, 1H), 6.79 (dd, *J* = 1.2, 5.6 Hz, 1H), 4.75-4.71 (m, 2H), 4.12-4.02 (m, 2H), 3.99-3.69 (m, 3H), 3.66-3.56 (m, 1H), 3.41-3.26 (m, 1H), 2.90-2.76 (m, 1H), 2.52 (s, 3H), 2.52-2.44 (m, 1H), 1.43 (s, 9H).

Step 6: (*S*)-6-chloro-8-fluoro-7-(5-methyl-1-((4-propionylmorpholin-2-yl)methyl)-1*H*-benzo[*d*]i midazol-2-yl)-277-benzo[*b*][1,4]oxazin-3(4*H*)-one **(17)**

To a solution of compound **17e** (182.0 mg, 0.34 mmol) in dichloromethane (5 mL) was slowly added dropwise a solution of 4 M HCl in 1,4-dioxane (0.9 mL) in an ice-water bath, and the mixture was stirred at room temperature for 4 h. After TLC (petroleum ether/ethyl acetate = 1/1) showed that the reaction was completed, the reaction mixture was concentrated, and the residue was dissolved in dichloromethane (3 mL). Triethylamine (240 $\mu$L, 1.72 mmol) and propionyl chloride (33 $\mu$L, 0.38 mmol) were added in an ice-water bath, and the mixture was reacted at room temperature for 2 h. After TLC (petroleum ether/ethyl acetate = 1/1) showed that the reaction was completed, water (20 mL) was added to quench the reaction, and the mixture was extracted with dichloromethane (20 mL $\times$ 3). The combined organic phase was dried over anhydrous sodium sulfate, filtered and concentrated under reduced pressure. The residue was purified by flash silica gel chromatography (ethyl acetate/petroleum ether = 0-100%) to give compound **17** (74.0 mg, yield: 44.7%).
MS (ESI) m/z = 487.1 $[M+H]^+$.

**Biological Assay**

Test Example 1. Evaluation of *In Vitro* Biological Activity

The compounds were screened for antagonistic activity against hP2X3 and hP2X2/3 receptors (changes in calcium flux signals represent the effect of the compounds on ion channels) by an FLIPR assay.

1. Experimental instruments and materials

| Instrument | Equipment manufacturer | Model |
|---|---|---|
| FLIPR | Molecular Devices | TETRA |
| $CO_2$ incubator | Thermo Fisher | 3111 |
| Name of reagents and consumables | Source | Cat. No. |
| FLIPR® calcium 4 assay kit | Molecular Devices | R8141 |
| FLIPR pipettor tip | Molecular Devices | 9000-0764 |
| Matrix D.A.R.T.s Tips-30ul | Thermo | 5416 |
| FBS | Gibco | 10099-141 |
| DMEM | Gibco | 11965 |
| Hygromycin B | Invitrogen | 10687010 |
| G418 disulfate salt | SIGMA | G5013 |
| 384-well assay plate | Corning | CC3712 |
| 384-well compound plate | Corning | CC3657 |
| Echo qualified 384-well polypropylene microplate | LABCYTE | P-05525 |
| Probenecid | Sigma | P8761-25G |
| 1x HBSS | Invitrogen | 14025 |
| ATP hydrolase | Sigma | A7646 |
| HEPES | Invitrogen | 15630-080 |
| Versene | Gibco | 15040066 |
| $\alpha\beta$-meATP | Sigma | M6517 |
| Stable cell lines | Chempartner | 1321N1/hP2X3, 1321N1/hP2X2/3 |

2. Experimental procedures

1321N1 cells (adherent cells) stably transfected with hP2X3 and hP2X2/3 receptors were digested, centrifuged, resuspended in a plating medium (DMEM + 10% DFBS) and counted, adjusted to $2\times10^5$ cells/mL. The cells were seeded in a 384-well assay plate at 50 $\mu$L/well, and incubated in an incubator at 37 °C with 5% $CO_2$ for 16-24 h. Test compounds (20 mM DMSO stock solution) that were 180 times the desired concentration were prepared with DMSO, and added to a 384-well compound plate at 500 nL/well. Then, each well was supplemented with 30 $\mu$L of FLIPR buffer (1$\times$ HBSS containing 1.26 mM $Ca^{2+}$ + 2 mM $CaCl_2$ + 20 mM HEPES), and the mixture was shaken for 20-40 min to be mixed well. The agonist $\alpha,\beta$-meATP (final concentration of 500 nM for hP2X3 cells and final concentration of 1000 nM for hP2X2/3 cells) that was 3 times the desired concentration was prepared with an FLIPR buffer, and added to another 384-well compound plate at 35 $\mu$L/well. The cell plate in which cells had been plated and cultured for 16-24 h was taken out, and the cell supernatant was removed by pipetting. 30 $\mu$L of dye (FLIPR® calcium 4 assay kit, diluted with an FLIPR buffer) was added to each well, and the plate was incubated for 1 h. 15 $\mu$L of the compound was added to each well containing cells (dosing using an FLIPR instrument), and after 15 min, 22.5 $\mu$L of agonist was added to each well and the fluorescent signal was detected (excitation wavelength: 470-495 nm, emission wavelength: 515-575 nm). With the difference between the peak and the trough of the signal taken as the basic data, the data of the highest concentration of the positive drug taken as the 100% inhibition rate, and the data of DMSO taken as the 0% inhibition rate, the inhibition effect curves of the compounds were fitted on the software Graphpad Prism 6 and $IC_{50}$ values were calculated.

Table 1. The half maximal inhibitory concentrations ($IC_{50}$) of the compounds of the present disclosure against hP2X3 and hP2X2/3 receptors

| Compound No. | hP2X3 ($IC_{50}$, nM) | hP2X2/3 ($IC_{50}$, nM) |
|---|---|---|
| **MK-7264** | 35.4 | 116.2 |
| **1** | 36.8 | 5566 |
| **2** | 105.0 | 18990 |
| **3** | 41.2 | 1805 |
| **4** | 31.6 | 1927 |
| **5** | 100.4 | NT |
| **6** | >10000 | NT |
| **7** | 5843 | NT |
| **8** | >10000 | NT |
| **9** | 75.8 | 30270 |
| **10** | 54.1 | 7407 |
| **11** | 79.4 | NT |
| **12** | 87.7 | NT |
| **13** | 765.9 | NT |
| **14** | 33.0 | NT |
| **15** | 85.1 | NT |
| **16** | 70.5 | NT |
| **17** | 45.3 | 3501 |
| NT: not tested. | | |

Test Example 2. CYP Inhibition Assay

The metabolic reactions of representative substrates of 5 major human CYP subtypes (CYP1A2, CYP2C9, CYP2C19, CYP2D6, CYP3A4/5) were evaluated using 150-donor pooled human liver microsomes (purchased from Coming, Cat. No. 452117). The effects of different concentrations of the test compounds on the metabolic reactions of phenacetin (CYP1A2), diclofenac sodium (CYP2C9), S-mephenytoin (CYP2C19), bufuralol hydrochloride (CYP2D6) and midazolam (CYP3A4/5) were determined by liquid chromatography-tandem mass spectrometry (LC/MS/MS).

30 μM phenacetin, 10 μM diclofenac sodium, 35 μM S-mephenytoin, 5 μM bufuralol hydrochloride, 3 μM midazolam, 1 mM NADPH, and test compounds (at concentrations of 0.1 μmol/L, 0.3 μmol/L, 1 μmol/L, 3 μmol/L, 10 μmol/L, and 30 μmol/L, respectively) or positive compound or blank were incubated with 200 μL of a reaction system of pooled human liver microsomes (0.2 mg/mL) (100 mmol/L phosphate buffer, pH 7.4, containing 0.3% by volume of DMSO, 0.6% by volume of acetonitrile, and 0.1% by volume of methanol) at 37 °C for 5 min. Then, 200 μL of acetonitrile containing 3% formic acid and 40 nM internal standard verapamil was added, and the mixture was centrifuged at 4000 rpm for 50 min. The mixture was cooled on ice for 20 min and centrifuged at 4000 rpm for 20 min to precipitate the protein. 200 μL of the supernatant was analyzed by LC/MS/MS.

The peak area was calculated from the chromatogram. The residual activity rate (%) was calculated by the following formula:

$$\text{peak area rate} = \text{peak area of metabolite/peak area of internal standard}$$

$$\text{residual activity rate (\%)} = \text{peak area proportion of the test compound group/peak area proportion of the blank group}$$

The half maximal inhibitory concentrations ($IC_{50}$) against CYP were calculated by Excel XLfit 5.3.1.3.
The determined half maximal inhibitory concentrations ($IC_{50}$) values against CYP were shown in the table below.

Table 2. Half maximal inhibitory concentrations ($IC_{50}$) of part of the compounds of the present disclosure against CYP

| Examples No. | CYP 1A2 (μM) | CYP 2C9 (μM) | CYP 2C19 (μM) | CYP 2D6 (μM) | CYP 3A4/5 (μM) |
|---|---|---|---|---|---|
| **1** | >30 | 21.7 | 13.0 | >30 | >30 |
| **2** | >30 | 3.06 | 14.6 | >30 | >30 |
| **3** | >30 | >30 | >30 | >30 | >30 |
| **4** | >30 | >30 | >30 | >30 | >30 |
| **9** | >30 | 1.48 | 12.9 | >30 | 12.9 |
| **10** | >30 | >30 | >30 | >30 | >30 |
| **14** | >30 | 21.9 | >30 | >30 | >30 |

Test Example 3. Assay on *In Vitro* Metabolic Stability in Human Hepatocytes

The concentrations of the test compounds in the reaction system were determined by LC/MS/MS, so that the intrinsic clearance of the test compounds was calculated, and the *in vitro* metabolic stability in human hepatocytes was evaluated.
247.5 μL of a mixed solution of human hepatocytes at $1\times10^6$ cells/mL (purchased from BioreclamationIVT, Cat. No. S01205) and 2.5 μL of 100 μM test compound or positive control was added to a plate to initiate the reaction. The plate was incubated at 37 °C and 600 rpm. 20 μL of incubation system was transferred to a stop plate at 0.5 min, 5 min, 15 min, 30 min, 45 min, 60 min, 80 min, 100 min and 120 min. Then, the mixture was vortexed for 2 min to be mixed well. The stop plate was centrifuged at 4000 rpm for 20 min. 40 μL of supernatant of each compound was transferred to a 96-well sample template, after which 160 μL of pure water was added to dilute the samples.
The resulting samples were quantified from ion chromatogram. The residual rate was calculated from the peak area of the test compound or the positive control. Slope k was determined by linear regression of the natural pair values of residual rates versus incubation time using Microsoft Excel.
Intrinsic clearance (*in vitro* $CL_{int}$, μL/min/$10^6$ cells) was calculated from the slope value according to the following equation:

$$\textit{in vitro}\ CL_{int} = kV/N$$

V = incubation volume (0.25 mL);
N = number of cells per well ($0.25\times10^6$ cells)

The determined values of intrinsic clearance in human hepatocytes are shown in Table 3.

Table 3. Intrinsic clearance of part of the compounds of the present disclosure in human hepatocytes

| Compound No. | Intrinsic clearance (μL/min/$10^6$ cells) |
|---|---|
| **1** | <1 |
| **2** | 2.98 |
| **3** | 4.08 |
| **4** | <1 |
| **9** | 3.86 |
| **10** | <1 |
| **14** | <1 |
| **17** | 4.1 |

Test Example 4. Caco-2 Permeability Assay

The apparent permeability coefficient ($P_{app}$) of the analytical drug was determined by liquid chromatography-tandem mass spectrometry (LC/MS/MS) through a Caco-2 cell model.

The transfer rate of drug from the apical end to the basal end was determined. 108 μL of HBSS (25 mM HEPES, pH 7.4) containing 10 μM test compound was added to the apical end of the upper chamber of Transwell (purchased from Coming) containing Caco-2 cells (purchased from ATCC) at a density of $6.86\times10^5$ cells/$cm^2$, and meanwhile, 8 μL of the sample was added, as an initial dosing-end sample (A-B), to a new 96-well plate to which 72 μL of HBSS (25 mM HEPES, pH 7.4) and 240 μL of acetonitrile (containing 100 nM alprazolam, 200 nM caffeine and 100 nM tolbutamide) had been added, and the plate was vortexed at 1000 rpm for 10 min. 300 μL of HBSS (25 mM HEPES, pH 7.4) was added to the basal end.

The transfer rate of drug from the basal end to the apical end was determined. 308 μL of HBSS (25 mM HEPES, pH 7.4) containing 10 μM test compound was added to wells of the basal end of the plate, and meanwhile, 8 μL of the sample was added, as an initial dosing-end sample (B-A), to a new 96-well plate to which 72 μL of HBSS (25 mM HEPES, pH 7.4) and 240 μL of acetonitrile (containing 100 nM alprazolam, 200 nM caffeine and 100 nM tolbutamide) had been added. The plate was vortexed at 1000 rpm for 10 min, and 100 μL of HBSS (25 mM HEPES, pH 7.4) was added to the apical end. The transfer rate from the apical end to the basal end and the transfer rate from the basal end to the apical end need to be determined simultaneously.

At the end of the transfer cycle, 8 μL of the sample was taken from the dosing end (apical end of A-B flow and basal end of B-A flow) and added to 72 μL of HBSS (25 mM HEPES, pH 7.4) and 240 μL of acetonitrile (containing 100 nM alprazolam, 200 nM caffeine and 100 nM tolbutamide), and the mixture was added to a new 96-well plate. 80 μL of the liquid was taken directly from each of the basal end of the A-B flow and the apical end of the B-A flow, and added together with 240 μL of acetonitrile (containing 100 nM alprazolam, 200 nM caffeine and 100 nM tolbutamide) to a new 96-well plate. The plates were vortexed at 1000 rpm for 10 min. The samples were centrifuged at 4000 rpm for 30 min. 100 μL of the supernatant was transferred to a new 96-well plate. All samples were analyzed by LC-MS/MS with 100 μL of pure water. Data were calculated using Microsoft Excel and peak areas were calculated from the chromatograms. The apparent permeability coefficient (Papp) is in cm/s and is calculated using the following formula:

$$P_{app} = \frac{dQ/Dt}{A \times D_0}$$

Papp is apparent permeability (cm/s$\times10^{-6}$);
dQ/dt is the drug delivery rate (pmol/s);
A is the surface area of the film ($cm^2$);
$D_0$ is the initial supply-end drug concentration (nM; pmol/$cm^3$).

The outflow ratio can be determined by the following equation:

$$\text{Outflow ratio} = \frac{P_{app(B-A)}}{P_{app(A-B)}}$$

Papp (B-A) is the apparent permeability coefficient in the direction from the basal end to the apical end;
Papp (A-B) is the apparent permeability coefficient in the direction from the apical end to the basal end.

The apparent permeability coefficient values of the Caco-2 cells are shown in Table 4.

Table 4

| Compound No. | $P_{app}$ (A-B) ($10^{-6}$, cm/s) | Outflow ratio |
|---|---|---|
| **4** | 8.1 | 3.8 |
| **BLU-5937** | 2.4 | 1.2 |

Note:

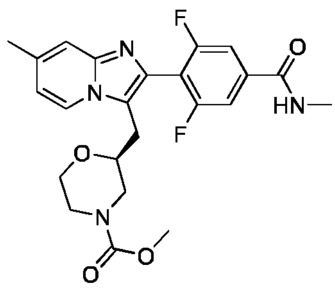

**BLU-5937.**

Test Example 5. *In Vivo* Pharmacokinetic Experiment in Rats

The drug concentrations in the plasma of the test animals (rats) at different time points after intragastric administration of the compound of the present disclosure were determined by an LC/MS/MS method. The pharmacokinetic behavior in rats of the compound of the present disclosure was studied and its pharmacokinetic profile was evaluated.

1. Test protocol

1.1 Test compounds
Compound **4** and **BLU-5937.**
1.2 Test animals
Male, healthy SD rats of SPF grade, 6-8 weeks old, 3 rats in each group.
1.3 Drug preparation
Intragastric administration: a certain amount of compound was weighed and prepared into a 1 mg/mL white suspension by adding 0.5% by volume of hydroxypropyl methylcellulose, 0.1% by volume of tween 80 and 99.4% by volume of water.
1.4 Administration
SD rats were administered intragastrically with compound **4** or **BLU-5937** at a dose of 5 mg/kg after fasting overnight.

2. Operation

Rats were administered intragastrically with the compound of the present disclosure. 0.2 mL of blood was collected from the jugular vein at 0.25 h, 0.5 h, 1 h, 2 h, 4 h, 8 h and 24 h after the administration, placed in a tube containing EDTA-K2, separated by centrifugation at 4 °C at 4000 rpm for 5 min to obtain plasma, which was stored at -75 °C.

Determination of the content of the test compounds at different concentrations in the plasma of rats after intragastric administration: 50 μL of rat plasma at each time point after the administration was taken, 200 μL of a solution of internal standard dexamethasone (50 ng/mL) in acetonitrile was added thereto, and the mixture was vortexed for 30 s to be mixed well, centrifuged at 4 °C at 4700 rpm for 15 min. The supernatant of the plasma sample was subjected to 3-fold dilution with water, and 2.0 μL of the dilution was taken for LC/MS/MS analysis.

3. Results of pharmacokinetic parameters

The pharmacokinetic parameters for part of the compounds of the disclosure in rats are as follows:

Table 5

| | Compound 4 | **BLU-5937** |
|---|---|---|
| V (L/kg) | 2.05 | 1.09 |
| $AUC_{0-t}$ (h*ng/ml) | 408 | 361 |
| $C_{max}$ (ng/ml) | 356 | 151 |
| $T_{1/2}$ (h) | 0.96 | 2.90 |

(continued)

| | Compound 4 | **BLU-5937** |
|---|---|---|
| $T_{max}$ (h) | 0.42 | 0.25 |

## Claims

**1.** A compound of formula (I) or a pharmaceutically acceptable salt or isomer thereof,

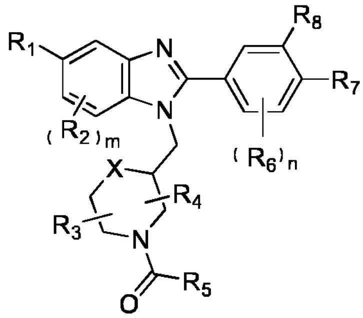

(I),

wherein $R_1$ is selected from the group consisting of hydrogen, deuterium, halogen, $C_1$-$C_6$ hydroxyalkyl, $C_1$-$C_6$ alkyl optionally substituted with halogen or deuterium, and $C_1$-$C_6$ alkoxy optionally substituted with halogen or deuterium;

$R_2$ are each independently selected from the group consisting of hydrogen, deuterium, halogen, $C_1$-$C_6$ hydroxyalkyl, $C_1$-$C_6$ alkyl optionally substituted with halogen or deuterium, and $C_1$-$C_6$ alkoxy optionally substituted with halogen or deuterium;
$R_3$ and $R_4$ are each independently selected from the group consisting of hydrogen, halogen and $C_1$-$C_4$ alkyl optionally substituted with halogen, or $R_3$ and $R_4$ form, together with the carbon atom to which they are attached, $C_3$-$C_6$ cyclohydrocarbylene optionally substituted with halogen, or $R_3$ and $R_4$ on adjacent carbon atoms together form $C_3$-$C_8$ cyclohydrocarbyl optionally substituted with halogen;
$R_5$ is selected from the group consisting of $C_1$-$C_6$ alkyl optionally substituted with halogen or cyano, $C_3$-$C_6$ cyclohydrocarbyl optionally substituted with halogen or cyano, heterocyclyl optionally substituted with halogen or cyano, $C_1$-$C_6$ alkoxy optionally substituted with halogen or cyano, and amino optionally substituted with alkyl;
$R_6$ are each independently selected from the group consisting of hydrogen, deuterium, halogen, cyano, cyclopropyl, and $C_1$-$C_6$ alkyl optionally substituted with halogen or deuterium;
$R_7$ and $R_8$ are each independently selected from the group consisting of:

a) hydrogen, deuterium, halogen, cyano, amino, hydroxy, $C_1$-$C_6$ alkyl optionally substituted with halogen, sulfone, sulfoxide, sulfonamide, sulfenamide, $C_{1-3}$ carboxyl, and $C_1$-$C_6$ alkoxy optionally substituted with halogen;
b)

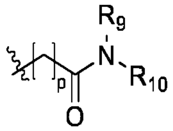,
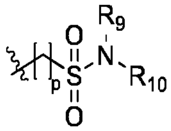
and
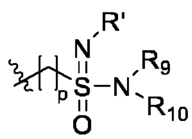,

wherein p is selected from the group consisting of 0, 1 and 2; $R_9$ and $R_{10}$ are each independently selected from the group consisting of hydrogen, $C_1$-$C_6$ alkyl, $C_1$-$C_6$ alkoxy, and $C_3$-$C_8$ cyclohydrocarbyl, or $R_9$ and $R_{10}$ form, together with the nitrogen atom to which they are attached, 4- to 6-membered heterocyclyl, wherein the heterocyclyl is optionally substituted with one or more substituents selected from the group consisting of halogen, hydroxy, cyano, $C_1$-$C_6$ haloalkyl, and $C_1$-$C_6$ alkyl; R' is selected from the group consisting of hydrogen, $C_1$-$C_6$ alkyl, $C_3$-$C_8$ cycloalkyl, aryl and heteroaryl; and in

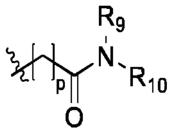

,

when p is 0 and $R_9$ is hydrogen, $R_{10}$ is not methyl; and when p is 0 and $R_9$ is methyl, $R_{10}$ is not hydrogen;
c)

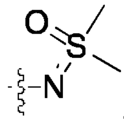

;

d) heterocyclyl and heteroaryl, wherein the heterocyclyl and heteroaryl are each optionally substituted with one or more substituents selected from the group consisting of oxo, halogen, hydroxy, carbonyl, $C_1$-$C_6$ alkyl and cyano, wherein the $C_1$-$C_6$ alkyl is optionally substituted with one or more halogens; and
e)

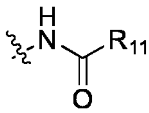 , 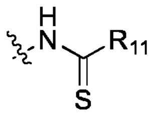 , 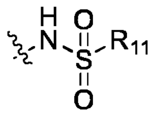 and 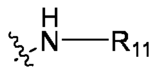 ,

wherein $R_{11}$ is selected from the group consisting of $C_1$-$C_6$ alkyl, $C_1$-$C_6$ alkoxy, aryl, heteroaryl, $C_3$-$C_8$ cyclohydrocarbyl, heterocyclyl, $C_1$-$C_6$ cyanoalkyl, $C_3$-$C_8$ cyclohydrocarbyloxy, and amino optionally substituted with $C_1$-$C_6$ alkyl; or

$R_7$ and $R_8$ form, together with the atom to which they are attached, an optionally substituted aromatic or non-aromatic heterocyclic ring;
X is selected from the group consisting of an oxygen atom, -NH- and methylene, wherein the methylene is optionally substituted with one or more substituents selected from the group consisting of halogen, $C_3$-$C_8$ cyclohydrocarbyl, $C_3$-$C_6$ cyclohydrocarbylene, and $C_1$-$C_6$ alkyl;
m is an integer of 1-3; and
n is an integer of 1-4.

**2.** The compound of formula (I) or the pharmaceutically acceptable salt or isomer thereof according to claim 1, wherein $R_7$ and $R_8$ are each independently selected from the group consisting of:

a) hydrogen, deuterium, halogen, cyano, amino, sulfone, sulfonamide, sulfenamide, and $C_1$-$C_3$ alkyl substituted with one or more halogen;
b)

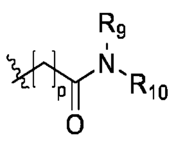 , 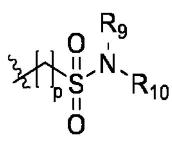 and 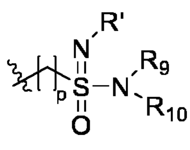 ,

wherein p is selected from the group consisting of 0, 1 and 2; $R_9$ and $R_{10}$ are each independently selected from the group consisting of hydrogen, $C_1$-$C_3$ alkyl, $C_1$-$C_3$ alkoxy, and $C_3$-$C_6$ cyclohydrocarbyl, or $R_9$ and $R_{10}$ form, together with the nitrogen atom to which they are attached, 4- to 6-membered heterocyclyl, wherein the heterocyclyl is optionally substituted with one or more substituents selected from the group consisting of halogen, hydroxy and $C_1$-$C_3$ alkyl; R' is selected from the group consisting of hydrogen, $C_1$-$C_3$ alkyl, $C_3$-$C_6$ cycloalkyl, aryl and heteroaryl; and in

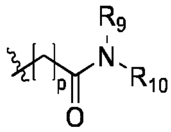,

when p is 0, the combination of $R_9$ and $R_{10}$ is not hydrogen and methyl;
c)

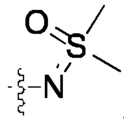;

d) 4- to 6-membered heterocyclyl and heteroaryl, wherein the heterocyclyl and heteroaryl are each optionally substituted with one or more substituents selected from the group consisting of oxo, halogen, hydroxy, carbonyl, $C_1$-$C_3$ alkyl and cyano, wherein the $C_1$-$C_3$ alkyl is optionally substituted with one or more halogens; and
e)

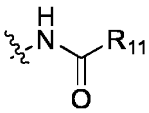, 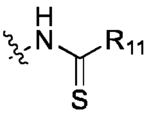, 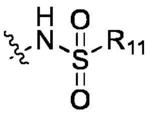 and 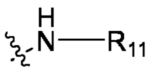,

wherein $R_{11}$ is selected from the group consisting of $C_1$-$C_3$ alkyl, $C_1$-$C_3$ alkoxy, 5- to 6-membered aryl or heteroaryl, 3- to 8-membered cyclohydrocarbyl, 3- to 8-membered heterocyclyl, $C_1$-$C_3$ cyanoalkyl, $C_3$-$C_6$ cyclohydrocarbyloxy, and amino optionally substituted with $C_1$-$C_3$ alkyl.

**3.** The compound of formula (I) or the pharmaceutically acceptable salt or isomer thereof according to claim 1 or 2, wherein

$R_7$ is 4- to 6-membered heterocyclyl or heteroaryl, wherein the heterocyclyl or heteroaryl is optionally substituted with one or more substituents selected from the group consisting of oxo, halogen, hydroxy, carbonyl, $C_1$-$C_3$ alkyl and cyano, wherein the $C_1$-$C_3$ alkyl is optionally substituted with one or more halogens; and
$R_8$ is selected from the group consisting of hydrogen, deuterium, halogen, cyano, and $C_1$-$C_3$ alkyl substituted with one or more halogens.

**4.** The compound of formula (I) or the pharmaceutically acceptable salt or isomer thereof according to any one of claims 1 to 3, wherein
$R_7$ is 4- to 6-membered heterocyclyl, wherein the heterocyclyl comprises -NH-C(=O)- or -NH-S(=O)$_2$-, and the heterocyclyl is optionally substituted with one or more substituents selected from the group consisting of oxo, halogen, hydroxy, carbonyl, $C_1$-$C_3$ alkyl and cyano, wherein the $C_1$-$C_3$ alkyl is optionally substituted with one or more halogens; further, $R_7$ is preferably 5-membered heterocyclyl, wherein the heterocyclyl comprises -NH-C(=O)-, and the heterocyclyl is optionally substituted with one or more substituents selected from the group consisting of oxo, halogen, hydroxy, carbonyl, $C_1$-$C_3$ alkyl and cyano, wherein the $C_1$-$C_3$ alkyl is optionally substituted with one or more halogens.

**5.** The compound of formula (I) or the pharmaceutically acceptable salt or isomer thereof according to claim 1, wherein $R_7$ and $R_8$ form, together with the atom to which they are attached, a 3- to 12-membered aromatic or non-aromatic heterocyclic ring, wherein the heterocyclic ring is monocyclic or bicyclic, and the heterocyclic ring is optionally substituted with one or more substituents selected from the group consisting of $C_1$-$C_6$ alkylamide, halogen, oxo, $C_1$-$C_6$ alkyl optionally substituted with halogen, and $C_1$-$C_6$ alkoxy; further, $R_7$ and $R_8$ preferably form, together with the atom to which they are attached, a 3- to 12-membered non-aromatic heterocyclic ring, wherein the heterocyclic ring is monocyclic or bicyclic, the heterocyclic ring comprises -NH-C(=O)- or -NH-S(=O)$_2$-, and the heterocyclic ring is optionally substituted with one or more substituents selected from the group consisting of $C_1$-$C_6$ alkylamide, halogen, oxo, $C_1$-$C_6$ alkyl optionally substituted with halogen and $C_1$-$C_6$ alkoxy; $R_7$ and $R_8$ more preferably form, together with the atom to which they are attached, a 4- to 8-membered non-aromatic heterocyclic ring, wherein the

heterocyclic ring is monocyclic or bicyclic, the heterocyclic ring comprises -NH-C(=O)- or -NH-S(=O)$_2$-, and the heterocyclic ring is optionally substituted with one or more substituents selected from the group consisting of $C_1$-$C_3$ alkylamide, halogen, oxo, $C_1$-$C_3$ alkyl optionally substituted with halogen and $C_1$-$C_3$ alkoxy.

**6.** The compound of formula (I) or the pharmaceutically acceptable salt or isomer thereof according to claim 1, wherein

$R_5$ is $C_1$-$C_6$ alkyl optionally substituted with halogen or cyano or $C_1$-$C_6$ alkoxy optionally substituted with halogen or cyano;
$R_6$ are each independently selected from the group consisting of hydrogen, deuterium, halogen and cyano; and
n is an integer of 1-4.

**7.** The compound of formula (I) or the pharmaceutically acceptable salt or isomer thereof according to claim 1 or 6, wherein

$R_1$ is selected from the group consisting of hydrogen, deuterium, $C_1$-$C_3$ alkyl optionally substituted with halogen or deuterium, and halogen;
$R_2$ are each independently selected from the group consisting of hydrogen, deuterium, $C_1$-$C_3$ alkyl optionally substituted with halogen or deuterium, and halogen;
$R_3$ and $R_4$ are each independently hydrogen or halogen, or $R_3$ and $R_4$ form, together with the carbon atom to which they are attached, $C_3$-$C_6$ cyclohydrocarbylene optionally substituted with halogen;
$R_5$ is $C_1$-$C_6$ alkyl or $C_1$-$C_6$ alkoxy;
$R_6$ are each independently selected from the group consisting of hydrogen, deuterium, halogen and cyano;
m is an integer of 1-3; and
n is an integer of 1-4.

**8.** The compound of formula (I) or the pharmaceutically acceptable salt or isomer thereof according to claim 1, wherein

$R_7$ is the following group optionally substituted with one or more substituents selected from the group consisting of methyl, a fluorine atom, a chlorine atom, halomethyl and cyano:

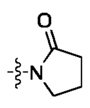 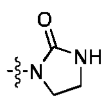 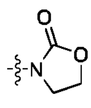 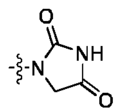 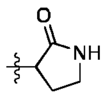 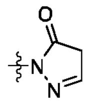 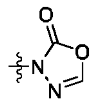 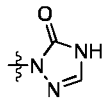 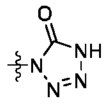 ,

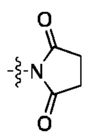 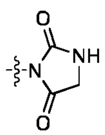 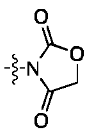 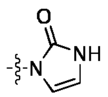 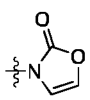 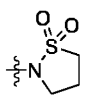 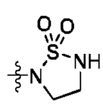 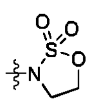 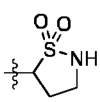 ,

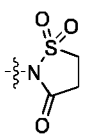 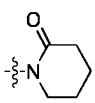 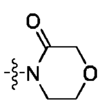 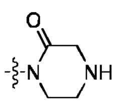 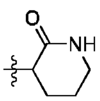 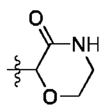 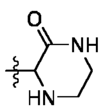 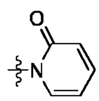 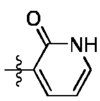

and

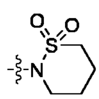 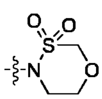 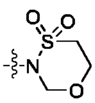 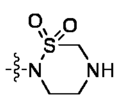 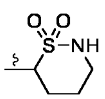 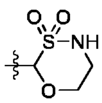 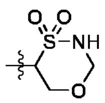 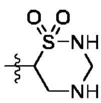 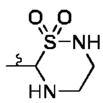 ;

and
$R_8$ is selected from the group consisting of hydrogen, deuterium, halogen, cyano, and $C_1$-$C_3$ alkyl substituted with one or more halogens.

**9.** The compound of formula (I) or the pharmaceutically acceptable salt or isomer thereof according to claim 1, wherein

$R_7$ and $R_8$ form, together with the atom to which they are attached, a heterocyclic ring A

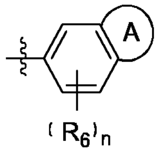,

wherein the heterocyclic ring A is selected from the group consisting of the following structures:

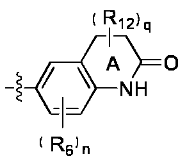 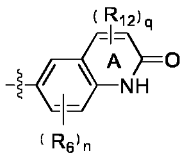 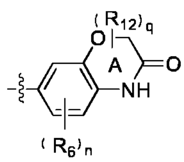 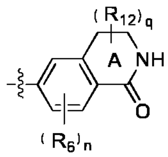 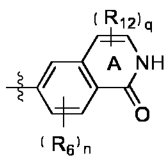 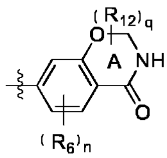,

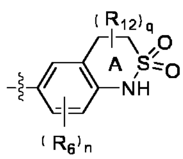 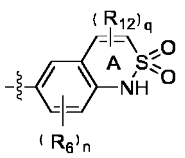 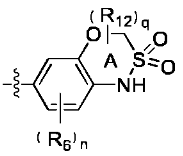 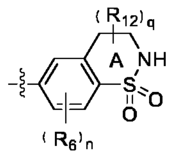 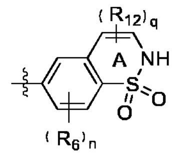 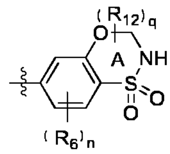

,

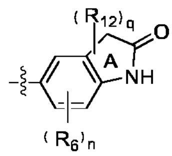 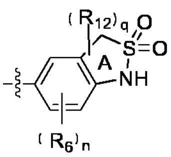 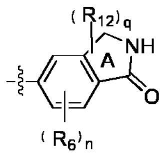 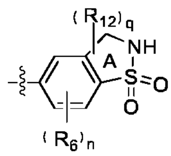 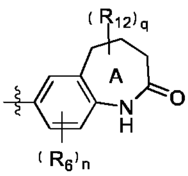 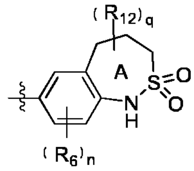

and

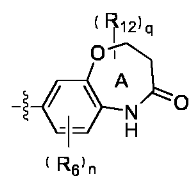 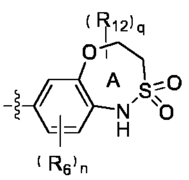 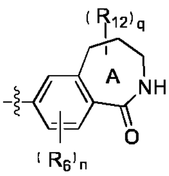 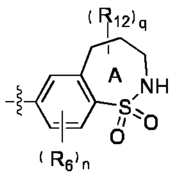 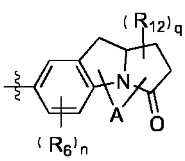 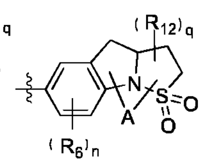;

$R_6$ are each independently selected from the group consisting of hydrogen, deuterium, halogen and cyano;
$R_{12}$ is independently selected from the group consisting of halogen, $C_1$-$C_3$ alkyl, and $C_3$-$C_6$ cyclohydrocarbylene, or adjacent $R_{12}$ together form a ring, wherein the ring is optionally substituted with one or more halogens or $C_1$-$C_3$ alkyl;
n is an integer selected from the group consisting of 1-3; and
q is an integer of 0-6.

**10.** The compound of formula (I) or the pharmaceutically acceptable salt or isomer thereof according to claim 1, wherein

$R_1$ is selected from the group consisting of hydrogen, deuterium, $C_1$-$C_3$ alkyl optionally substituted with halogen or deuterium, and halogen;
$R_2$ are each independently selected from the group consisting of hydrogen, deuterium, $C_1$-$C_3$ alkyl optionally substituted with halogen or deuterium, and halogen;

$R_3$ and $R_4$ are each independently hydrogen or halogen, or $R_3$ and $R_4$ form, together with the carbon atom to which they are attached, $C_3$-$C_6$ cyclohydrocarbylene optionally substituted with halogen;
$R_5$ is $C_1$-$C_6$ alkyl or $C_1$-$C_6$ alkoxy;
$R_7$ and $R_8$ form, together with the atom to which they are attached, a heterocyclic ring A

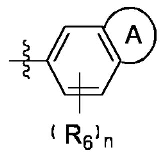

,

wherein the heterocyclic ring A is selected from the group consisting of the following structures:

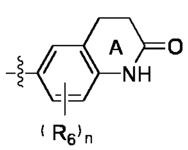
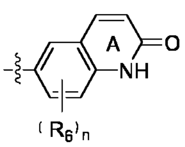
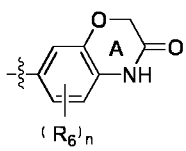
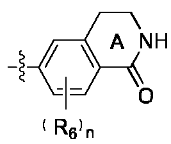
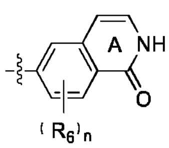
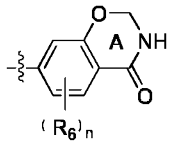
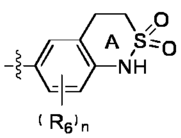
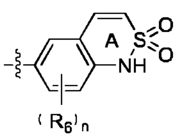
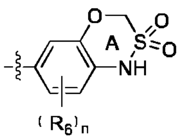
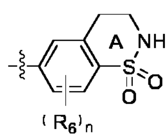
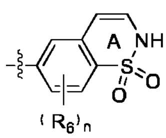
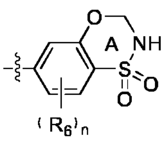
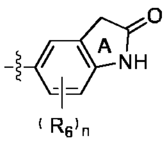
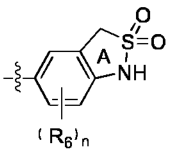
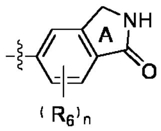
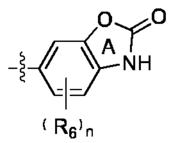
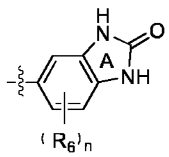
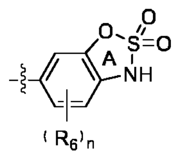

;

$R_6$ are each independently selected from the group consisting of hydrogen, deuterium, halogen and cyano;
m is an integer of 1-3; and
n is an integer of 1-3.

**11.** The compound of formula (I) or the pharmaceutically acceptable salt or isomer thereof according to claim 1, being

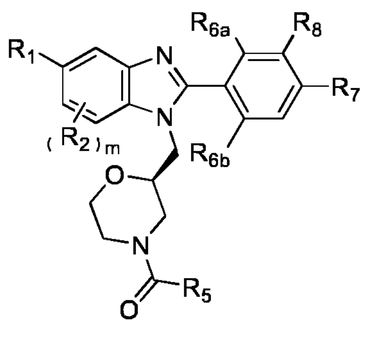

(I-1)

wherein $R_1$ is selected from the group consisting of hydrogen, deuterium, $C_1$-$C_3$ alkyl optionally substituted with halogen or deuterium, and halogen;

$R_2$ are each independently selected from the group consisting of hydrogen, deuterium, $C_1$-$C_3$ alkyl optionally substituted with halogen or deuterium, and halogen;

$R_5$ is $C_1$-$C_3$ alkyl or $C_1$-$C_3$ alkoxy;

$R_{6a}$ and $R_{6b}$ are each independently selected from the group consisting of hydrogen, deuterium, a chlorine atom, a fluorine atom and cyano;

$R_7$ is a 4- to 6-membered heterocyclyl, wherein the heterocyclyl comprises -NH-C(=O)- or -NH-S(=O)$_2$-, and the heterocyclyl is optionally substituted with one or more substituents selected from the group consisting of oxo, halogen, hydroxy, carbonyl, $C_1$-$C_3$ alkyl and cyano, wherein the $C_1$-$C_3$ alkyl is optionally substituted with one or more halogens;

$R_8$ is selected from the group consisting of hydrogen, deuterium, halogen, cyano and $C_1$-$C_3$ alkyl substituted with one or more halogens; and

m is an integer of 1-3.

**12.** The compound of formula (I-1) or the pharmaceutically acceptable salt or isomer thereof according to claim 11, wherein

$R_7$ is the following group optionally substituted with one or more substituents selected from the group consisting of methyl, a fluorine atom, a chlorine atom, halomethyl and cyano:

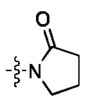 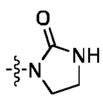 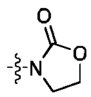 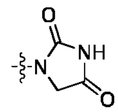 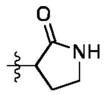 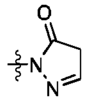 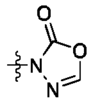 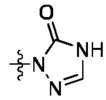 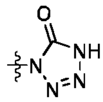 ,

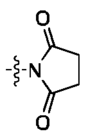 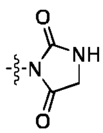 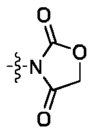 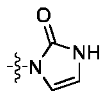 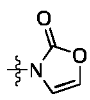 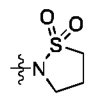 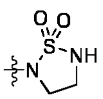 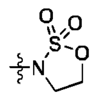 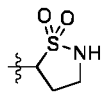 ,

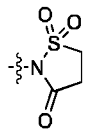 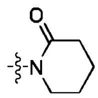 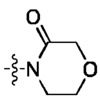 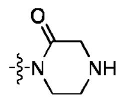 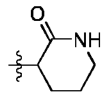 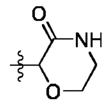 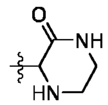 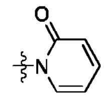 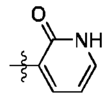

and

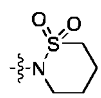 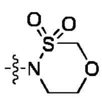 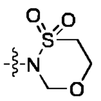 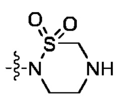 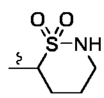 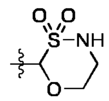 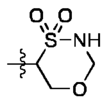 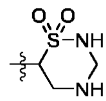 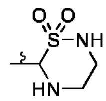 ;

and

$R_8$ is selected from the group consisting of hydrogen, deuterium, halogen and cyano.

**13.** The compound of formula (I) or the pharmaceutically acceptable salt or isomer thereof according to claim 1, being

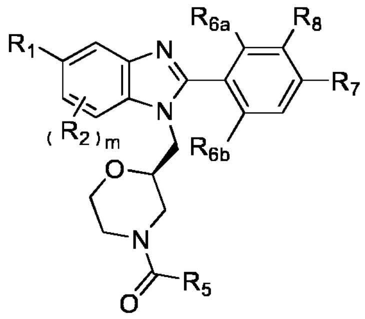

(I-1)

wherein $R_1$ is selected from the group consisting of hydrogen, deuterium, $C_1$-$C_3$ alkyl optionally substituted with halogen or deuterium, and halogen;

$R_2$ are each independently selected from the group consisting of hydrogen, deuterium, $C_1$-$C_3$ alkyl optionally substituted with halogen or deuterium, and halogen;
$R_5$ is $C_1$-$C_3$ alkyl or $C_1$-$C_3$ alkoxy;
$R_{6a}$ and $R_{6b}$ are each independently selected from the group consisting of hydrogen, deuterium, a chlorine atom, a fluorine atom and cyano;
$R_7$ and Rg form, together with the atom to which they are attached, a 4- to 8-membered non-aromatic heterocyclic ring, wherein the heterocyclic ring is monocyclic or bicyclic, the heterocyclic ring comprises -NH-C(=O)- or -NH-$S(=O)_2$-, and the heterocyclic ring is optionally substituted with one or more substituents selected from the group consisting of $C_1$-$C_3$ alkylamide, halogen, oxo, $C_1$-$C_3$ alkyl optionally substituted with halogen, and $C_1$-$C_3$ alkoxy; and
m is an integer of 1-3.

**14.** The compound of formula (I-1) or the pharmaceutically acceptable salt or isomer thereof according to claim 13, wherein

$R_7$ and $R_8$ form, together with the atom to which they are attached, a heterocyclic ring A

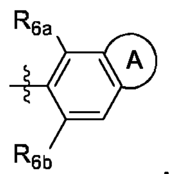
,

wherein the heterocyclic ring A is selected from the group consisting of the following structures:

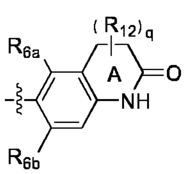 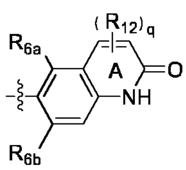 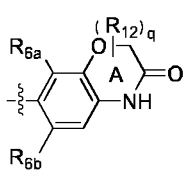 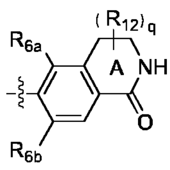 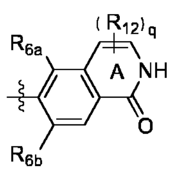 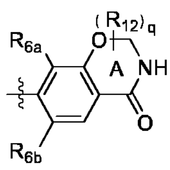

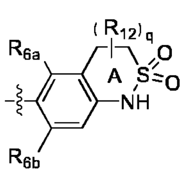 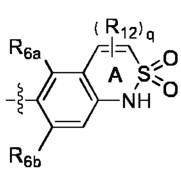 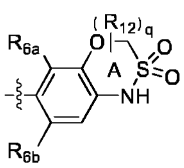 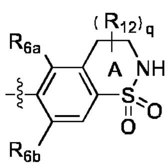 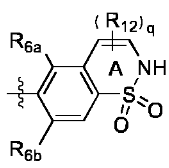 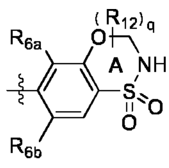

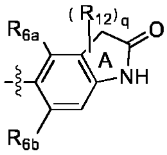 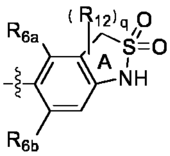 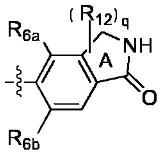 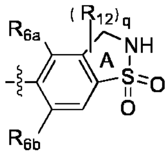 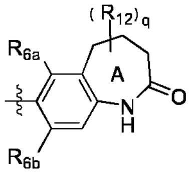 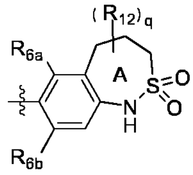

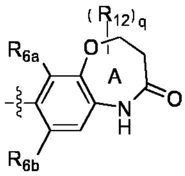 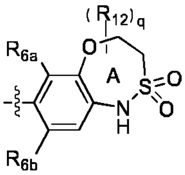 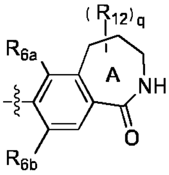 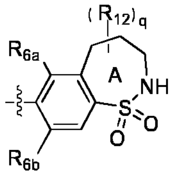 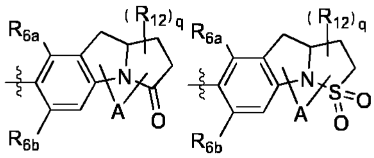 ;

$R_{6a}$ and $R_{6b}$ are each independently selected from the group consisting of hydrogen, deuterium, a chlorine atom and a fluorine atom;

$R_{12}$ are each independently selected from the group consisting of halogen, $C_1$-$C_3$ alkyl, and $C_3$-$C_6$ cyclohydrocarbylene, or adjacent $R_{12}$ together form a ring, wherein the ring is optionally substituted with one or more halogens or $C_1$-$C_3$ alkyl; and

q is an integer of 0-6.

**15.** The compound of formula (1-1) or the pharmaceutically acceptable salt or isomer thereof according to claim 14, wherein

$R_7$ and $R_8$ form, together with the atom to which they are attached, a heterocyclic ring A

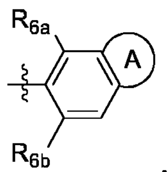 ,

wherein the heterocyclic ring A is selected from the group consisting of the following structures:

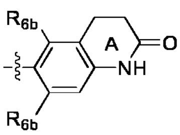 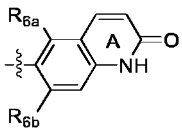 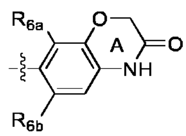 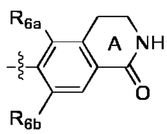 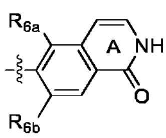 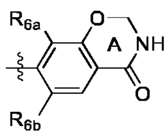 ,

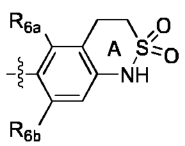 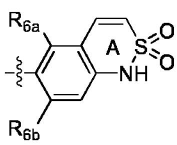 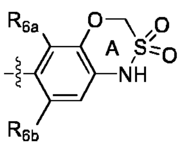 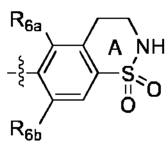 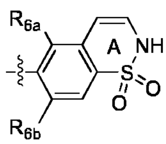 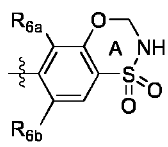

and

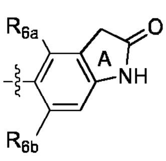 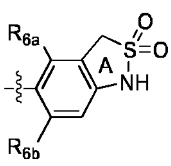 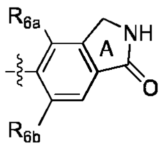 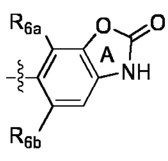 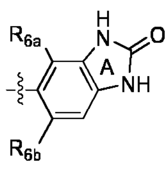 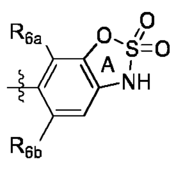 ;

and

$R_{6a}$ and $R_{6b}$ are each independently selected from the group consisting of hydrogen, deuterium, a chlorine atom and a fluorine atom.

**16.** The compound of formula (I) or the pharmaceutically acceptable salt or isomer thereof according to claim 1, being

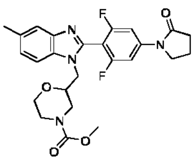,
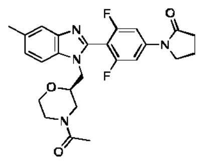,
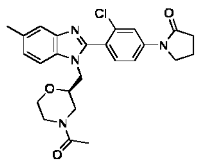,
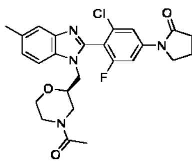,
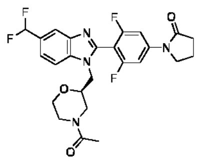,
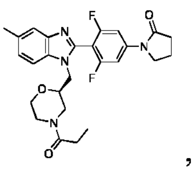,
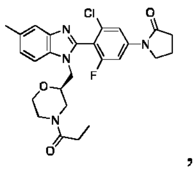,
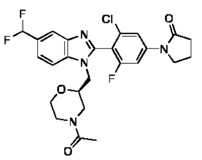,
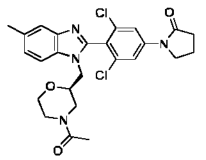,
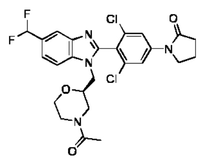,
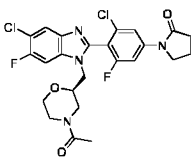,
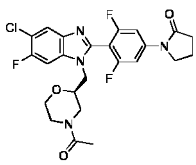,
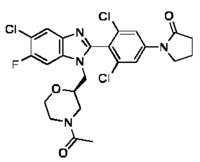,
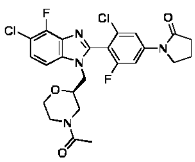,
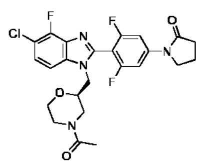,
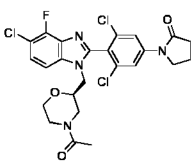,
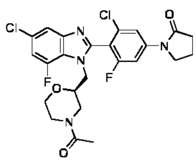,
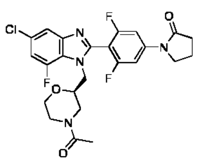,
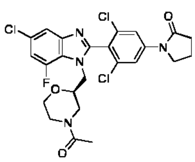,
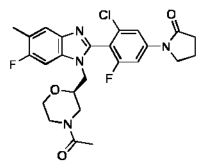,
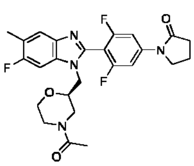,
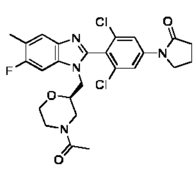,
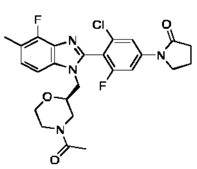,
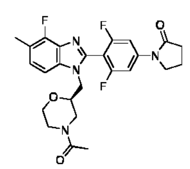,
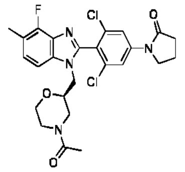,
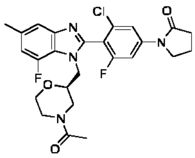,
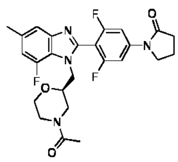,
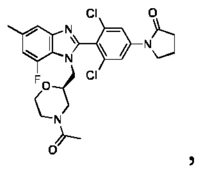,
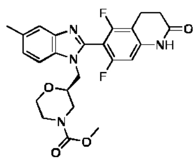,
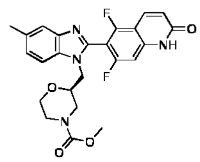,
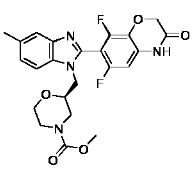,
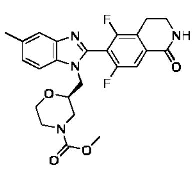,
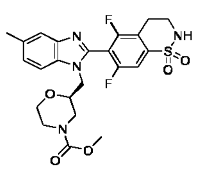,
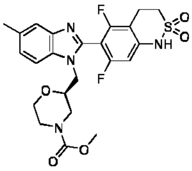,
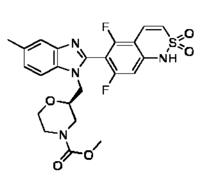,

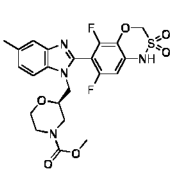 , 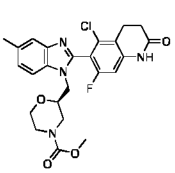 , 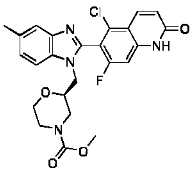 , 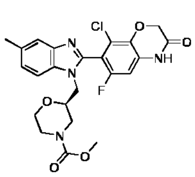 , 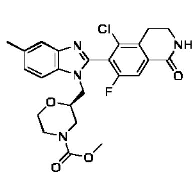 ,

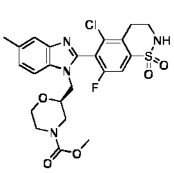 , 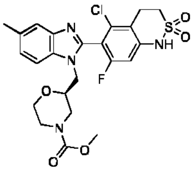 , 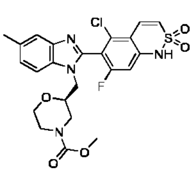 , 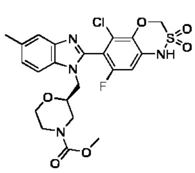 , 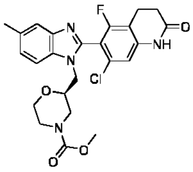 ,

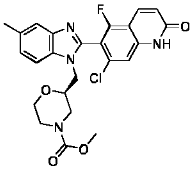 , 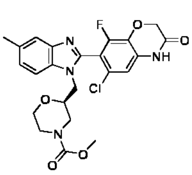 , 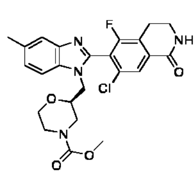 , 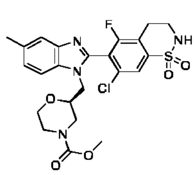 , 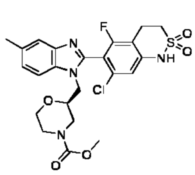 ,

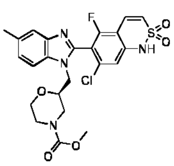 , 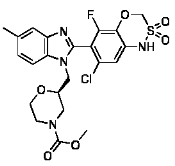 , 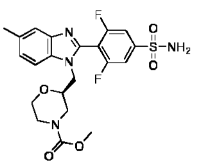 , 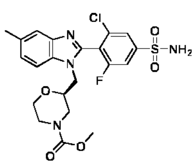 , 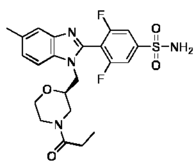 ,

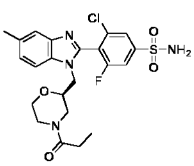 , 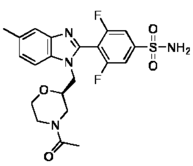 , 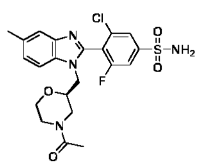 , 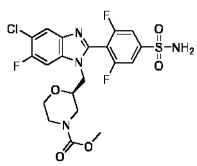 , 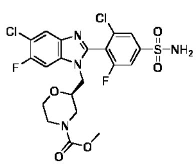 ,

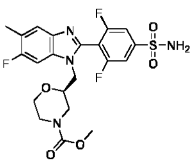 , 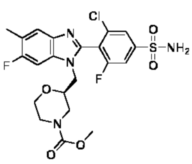 , 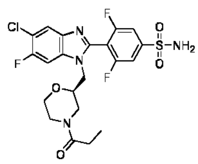 , 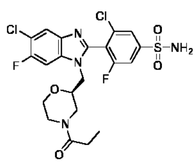 , 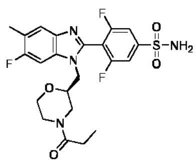 ,

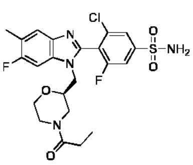 , 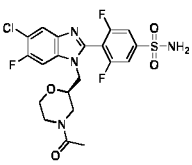 , 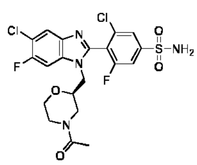 , 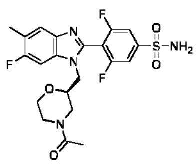 , 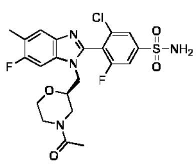 ,

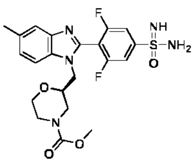 , 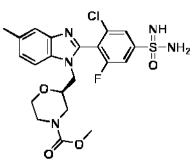 , 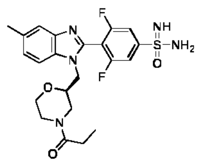 , 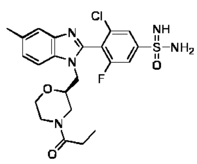 , 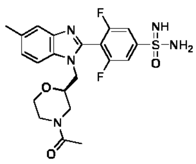 ,

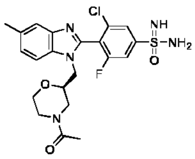 , 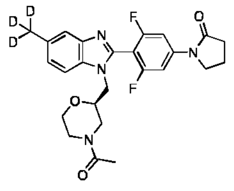 , 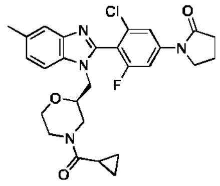 , 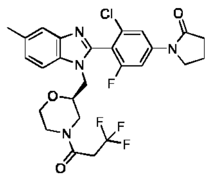 ,

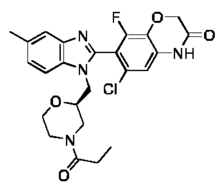 and 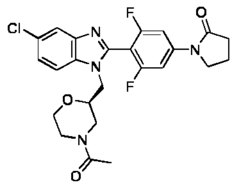 .

**17.** A method for preparing the compound of formula (I) or the pharmaceutically acceptable salt or isomer thereof according to claim 1, comprising the following steps:

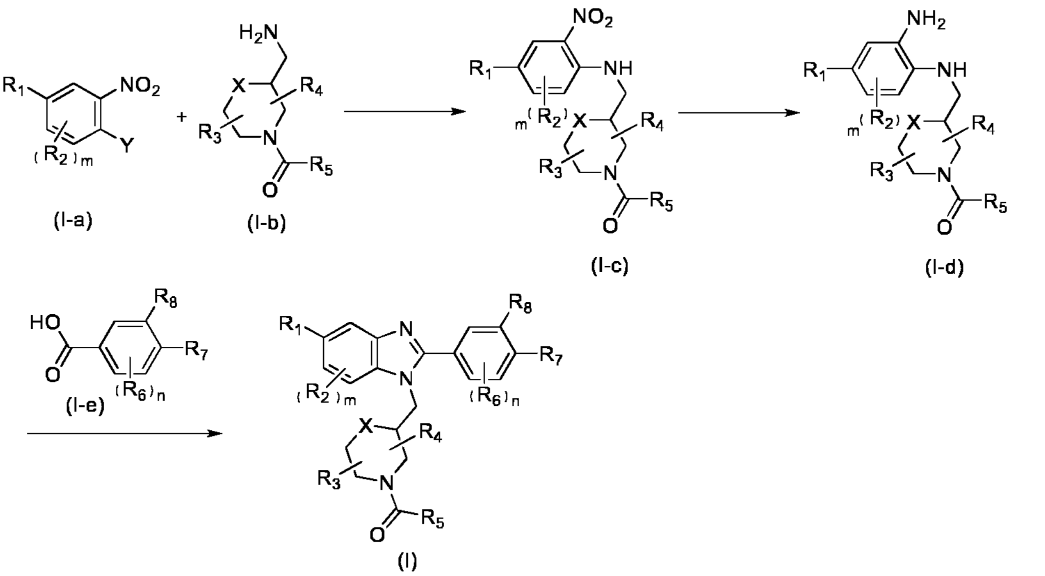

(I-a) (I-b) (I-c) (I-d) (I-e) (I)

subjecting a compound of formula (I-a) to a reaction with a compound of formula (I-b) under an alkaline condition to give a compound of formula (I-c); subjecting the compound of formula (I-c) to a reduction reaction to give a compound of formula (I-d);
subjecting the compound of formula (I-d) to a ring closure reaction with a compound of formula (I-e) under an acidic condition to give the compound of formula (I);
wherein $R_1$, $R_2$, $R_3$, $R_4$, $R_5$, $R_6$, $R_7$, $R_8$, X, m and n are as defined in claim 1; and
Y is selected from the group consisting of halogen, sulfonyl and sulfinyl.

**18.** A method for preparing the compound of formula (I) or the pharmaceutically acceptable salt or isomer thereof according to claim 1, comprising the following steps:

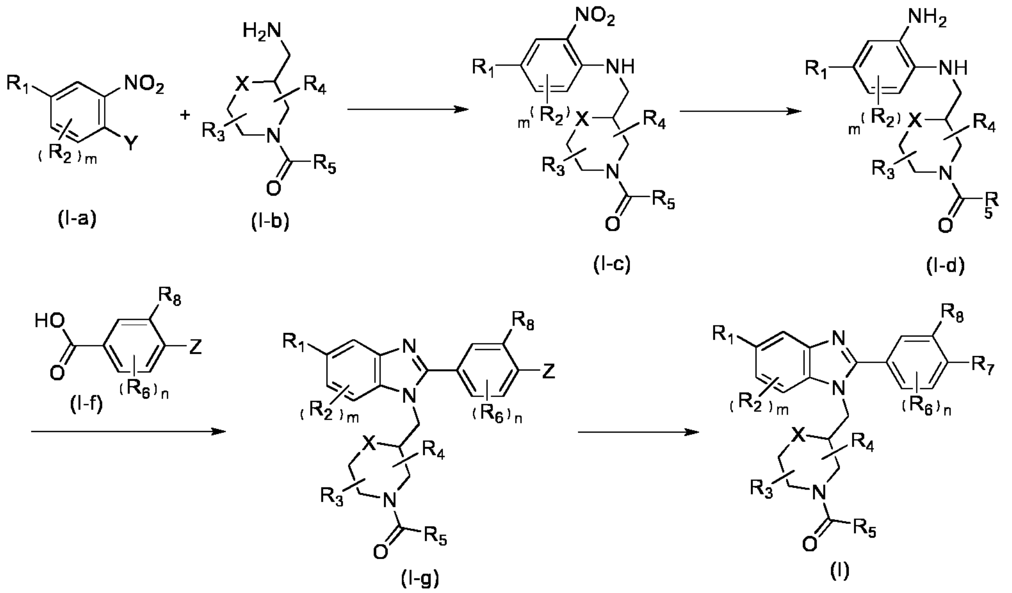

(I-a) (I-b) (I-c) (I-d) (I-f) (I-g) (I)

subjecting a compound of formula (I-a) to a reaction with a compound of formula (I-b) under an alkaline condition to give a compound of formula (I-c); subjecting the compound of formula (I-c) to a reduction reaction to give a compound of formula (I-d); subjecting the compound of formula (I-d) to a ring closure reaction with a compound of formula (I-e) under an acidic condition to give a compound of formula (I-g); and subjecting the compound of formula (I-g) under the action of a catalyst to give the compound of formula (I);

wherein the catalyst is selected from the group consisting of palladium/carbon, Raney Ni, tetrakis(triphenylphosphine)palladium(0), palladium dichloride, palladium acetate, [1,1'-bis(diphenylphosphino)ferrocene]palladium(II) dichloride, 1,1'-bis(dibenzylphosphino)ferrocene-palladium(II)dichloride, tris(dibenzylideneacetone)dipalladium(0), 2-dicyclohexylphosphino-2',6'-dimethoxybiphenyl, [1,1'-bis(di-*tert*-butylphosphino)ferrocene]palladium (II) dichlorine, cuprous iodide, cuprous bromide, cuprous chloride and copper(II) trifluoromethanesulphonate;

$R_1$, $R_2$, $R_3$, $R_4$, $R_5$, $R_6$, $R_7$, $R_8$, X, m, and n are as defined in claim 1; and

Y and Z are each independently selected from the group consisting of halogen, sulfonyl and sulfinyl.

**19.** A pharmaceutical composition comprising the compound or the pharmaceutically acceptable salt or isomer thereof according to any one of claims 1 to 16, and at least one pharmaceutically acceptable carrier, diluent or excipient.

**20.** Use of the compound or the pharmaceutically acceptable salt or isomer thereof according to any one of claims 1 to 16, or the pharmaceutical composition according to claim 19 in the preparation of a medicament for treating a disease related to P2X3 activity.

**21.** Use of the compound or the pharmaceutically acceptable salt or isomer thereof according to any one of claims 1 to 16, or the pharmaceutical composition according to claim 19 in the preparation of a medicament for treating a disease selected from the group consisting of pain, urinary tract diseases and cough.

## INTERNATIONAL SEARCH REPORT

International application No.
**PCT/CN2021/112386**

**A. CLASSIFICATION OF SUBJECT MATTER**

C07D 413/06(2006.01)i; A61K 31/5377(2006.01)i; A61P 13/00(2006.01)i; A61P 25/04(2006.01)i; A61P 11/14(2006.01)i

According to International Patent Classification (IPC) or to both national classification and IPC

**B. FIELDS SEARCHED**

Minimum documentation searched (classification system followed by classification symbols)

C07D;A61K;A61P

Documentation searched other than minimum documentation to the extent that such documents are included in the fields searched

Electronic data base consulted during the international search (name of data base and, where practicable, search terms used)

WPI, STN, EPODOC, CNPAT, CNKI, 万方, 百度学术, 上海拓界生物医药, 李文明, 刘宁, 刘彪, 刘浩淼, 余健, 邹昊, 祝伟, 李正涛, 李云飞, 苯并咪唑, 疼痛, 泌尿, 咳嗽, benzimidazole, P2X3, pain, urinary, disease, cough, structural formula search in STN

**C. DOCUMENTS CONSIDERED TO BE RELEVANT**

| Category* | Citation of document, with indication, where appropriate, of the relevant passages | Relevant to claim No. |
|---|---|---|
| X | CN 102741245 A (ASTRAZENECA AB) 17 October 2012 (2012-10-17)<br>abstract, and description, paragraphs [0013]-[0022], [0160] and [0167] | 1, 6, 7, 17 |
| X | WO 2020135771 A1 (WUHAN LANGLAI TECHNOLOGY DEVELOPMENT CO., LTD. et al.) 02 July 2020 (2020-07-02)<br>abstract and description pages 2-6, 65-78 and 114 | 1-21 |
| A | WO 2019064079 A2 (BELLUS HEALTH COUGH INC. et al.) 04 April 2019 (2019-04-04)<br>abstract and description, paragraphs [0005]-[0013] | 1-21 |
| A | CN 108904507 A (LAI, Yingjie) 30 November 2018 (2018-11-30)<br>abstract and description, paragraphs [0013]-[0023] | 1-21 |
| A | WO 2012158117 A1 (ASTRAZENECA AB et al.) 22 November 2012 (2012-11-22)<br>abstract and description paragraphs [11]-[13] and paragraphs [85]-[89] | 1-21 |

☐ Further documents are listed in the continuation of Box C.  ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **22 October 2021** | **11 November 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2021/112386**

| Patent document cited in search report | Publication date (day/month/year) | Patent family member(s) | Publication date (day/month/year) |
|---|---|---|---|
| CN 102741245 A | 17 October 2012 | AU 2010322478 B2 | 14 November 2013 |
| | | CN 102741245 B | 05 November 2014 |
| | | UY 33037 A | 30 June 2011 |
| | | US 2012289504 A1 | 15 November 2012 |
| | | EP 2501697 A1 | 26 September 2012 |
| | | MX 2012005329 A | 29 May 2012 |
| | | RU 2012124268 A | 27 December 2013 |
| | | RU 2542582 C2 | 20 February 2015 |
| | | KR 20120113709 A | 15 October 2012 |
| | | JP 5657018 B2 | 21 January 2015 |
| | | EP 2501697 A4 | 24 April 2013 |
| | | BR 112012011518 A2 | 28 June 2016 |
| | | US 8530467 B2 | 10 September 2013 |
| | | CA 2777746 A1 | 26 May 2011 |
| | | ES 2442797 T3 | 13 February 2014 |
| | | JP 2013511517 A | 04 April 2013 |
| | | EP 2501697 B1 | 23 October 2013 |
| | | AU 2010322478 A1 | 31 May 2012 |
| | | WO 2011062550 A1 | 26 May 2011 |
| | | BR 112012011518 B1 | 10 March 2020 |
| | | TW 201121957 A | 01 July 2011 |
| | | AR 079050 A1 | 21 December 2011 |
| | | IN 3391DEN2012 A | 23 October 2015 |
| | | CA 2777746 C | 14 May 2019 |
| WO 2020135771 A1 | 02 July 2020 | CN 111377917 A | 07 July 2020 |
| WO 2019064079 A2 | 04 April 2019 | WO 2019064079 A3 | 16 May 2019 |
| | | US 2020230148 A1 | 23 July 2020 |
| | | IL 273348 D0 | 31 May 2020 |
| | | AU 2018342751 A1 | 23 April 2020 |
| | | US 10111883 B1 | 30 October 2018 |
| | | KR 20200092938 A | 04 August 2020 |
| | | SG 11202002460 Q A | 29 April 2020 |
| | | CN 111601601 A | 28 August 2020 |
| | | EP 3684368 A4 | 26 May 2021 |
| | | JP 2020534355 A | 26 November 2020 |
| | | EP 3684368 A2 | 29 July 2020 |
| | | US 2021069201 A1 | 11 March 2021 |
| | | CA 3076150 A1 | 04 April 2019 |
| CN 108904507 A | 30 November 2018 | None | |
| WO 2012158117 A1 | 22 November 2012 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2005095359 A